(19) Europäisches Patentamt
European Patent Office
Office européen des brevets

(11) EP 3 230 403 B1

(12) # EUROPÄISCHE PATENTSCHRIFT

(45) Veröffentlichungstag und Bekanntmachung des Hinweises auf die Patenterteilung:
**09.10.2019 Patentblatt 2019/41**

(21) Anmeldenummer: **15805389.2**

(22) Anmeldetag: **18.11.2015**

(51) Int Cl.:
***C09K 11/06*** *(2006.01)* ***C07D 401/14*** *(2006.01)*
***C07C 255/58*** *(2006.01)* ***C07C 255/59*** *(2006.01)*
***C07D 221/20*** *(2006.01)* ***C07D 401/04*** *(2006.01)*
***C07D 401/10*** *(2006.01)* ***C07D 403/10*** *(2006.01)*
***C07D 405/04*** *(2006.01)* ***C07D 405/10*** *(2006.01)*
***C07D 455/04*** *(2006.01)* ***C07D 251/24*** *(2006.01)*
***C07D 491/107*** *(2006.01)* ***C07D 495/10*** *(2006.01)*
***C07D 209/86*** *(2006.01)* ***C07D 209/88*** *(2006.01)*
***C09B 57/00*** *(2006.01)* ***H01L 51/00*** *(2006.01)*
***H01L 51/50*** *(2006.01)*

(86) Internationale Anmeldenummer:
**PCT/EP2015/002311**

(87) Internationale Veröffentlichungsnummer:
**WO 2016/091353 (16.06.2016 Gazette 2016/24)**

(54) **ORGANISCHE VERBINDUNGEN MIT LÖSLICHEN GRUPPEN**

ORGANIC COMPOUNDS WITH SOLUBLE GROUPS

COMPOSÉS ORGANIQUES COMPRENANT DES GROUPES SOLUBLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.12.2014 EP 14004201**

(43) Veröffentlichungstag der Anmeldung:
**18.10.2017 Patentblatt 2017/42**

(73) Patentinhaber: **Merck Patent GmbH**
**64293 Darmstadt (DE)**

(72) Erfinder:
- **PARHAM, Amir Hossain**
  **60486 Frankfurt am Main (DE)**
- **STOESSEL, Philipp**
  **60389 Frankfurt am Main (DE)**
- **PFLUMM, Christof**
  **64291 Darmstadt (DE)**
- **JATSCH, Anja**
  **60489 Frankfurt am Main (DE)**
- **KAISER, Joachim**
  **64289 Darmstadt (DE)**
- **AYDT, Ewald**
  **64380 Rossdorf (DE)**
- **JOOSTEN, Dominik**
  **60487 Frankfurt am Main (DE)**

(56) Entgegenhaltungen:
**EP-A1- 2 599 773** **EP-A2- 2 182 040**
**WO-A1-2011/088877** **DE-A1-102009 023 155**

- **YE TAO ET AL: "Thermally Activated Delayed Fluorescence Materials Towards the Breakthrough of Organoelectronics", ADVANCED MATERIALS, vol. 26, no. 47, 17 September 2014 (2014-09-17), pages 7931-7958, XP055484893, DE ISSN: 0935-9648, DOI: 10.1002/adma.201402532**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 230 403 B1

**Beschreibung**

Die vorliegende Erfindung betrifft organische Verbindungen mit einem geringen Singulett-Triplett Abstand, Zusammensetzungen und Formulierungen enthaltend diese Verbindungen, elektronische Vorrichtungen enthaltend die Verbindungen oder Zusammensetzungen sowie Verfahren zur Herstellung der Vorrichtungen.

Der Aufbau einer speziellen elektronischen Vorrichtung, in denen organische Halbleiter als funktionelle Materialien eingesetzt werden, ist beispielsweise in US 4539507, US 5151629, EP 0676461 und WO 98/27136 beschrieben. Es handelt sich dabei um organische lichtemittierende Dioden (OLED), eine spezielle Gruppe der organischen Elektrolumineszenzvorrichtungen. Als emittierende Materialien werden hierbei insbesondere auch metallorganische Iridium- und Platinkomplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich.

Trotz der guten Ergebnisse, die mit metallorganischen Iridum- und Platinkomplexen erzielt werden, weisen diese jedoch auch eine Reihe von Nachteilen auf: So handelt es sich bei Iridium und Platin um seltene und teure Metalle. Es wäre daher zur Ressourcenschonung wünschenswert, die Verwendung dieser seltenen Metalle vermeiden zu können. Weiterhin weisen derartige Metallkomplexe teilweise eine geringere thermische Stabilität auf als rein organische Verbindungen, so dass auch aus diesem Grund die Verwendung rein organischer Verbindungen vorteilhaft wäre, sofern diese zu vergleichbar guten Effizienzen führen. Weiterhin sind blau, insbesondere tiefblau phosphoreszierende Iridium- bzw. Platinemitter mit hoher Effizienz und Lebensdauer technisch nur schwer zu verwirklichen, so dass es auch hier Verbesserungsbedarf gibt. Weiterhin gibt es insbesondere bei der Lebensdauer phosphoreszierender OLEDs, welche Ir- oder Pt-Emitter enthalten, Verbesserungsbedarf, wenn die OLED bei höherer Temperatur betrieben wird, wie dies für einige Anwendungen erforderlich ist. Weiterhin werden viele der bekannten organischen Halbleitermaterialien zur Herstellung elektronischer Vorrichtungen mittels Sublimation im Vakuum aufgedampft, was teilweise sehr aufwendig und kostenintensiv ist. Zudem können nicht alle Materialien aufgedampft werden, so dass bestimme organische Materialien trotz guter opto-elektronischer Eigenschaften aufgrund ihrer schlechten Prozessierbarkeit nicht in elektronischen Vorrichtungen verwendet werden können. Insbesondere für die Massenproduktion elektronischer Erzeugnisse ist eine kostengünstige Prozessierbarkeit organischer Materialien von sehr großem kommerziellen Interesse. Wünschenswert sind daher hoch effiziente organische Moleküle, die aus Lösung prozessiert werden können.

Eine Alternative zur Entwicklung phosphoreszierender Metallkomplexe ist die Verwendung von Emittern, die thermisch aktivierte verzögerte Fluoreszenz (thermally activated delayed fluorescence, TADF) zeigen (z. B. H. Uoyama et al., Nature 2012, Vol. 492, 234). Hier handelt es sich um organische Materialien, bei denen der energetische Abstand zwischen dem niedrigsten Triplettzustand $T_1$ und dem ersten angeregten Singulettzustand $S_1$ so klein ist, dass dieser Energieabstand kleiner oder im Bereich der thermischen Energie liegt. Aus quantenstatistischen Gründen entstehen bei elektronischer Anregung in der OLED die angeregten Zustände zu 75 % im Triplettzustand und zu 25 % im Singulettzustand. Da rein organische Moleküle üblicherweise nicht aus dem Triplettzustand emittieren können, können 75 % der angeregten Zustände nicht für die Emission genutzt werden, wodurch prinzipiell nur 25 % der Anregungsenergie in Licht umgewandelt werden können. Ist jedoch der energetische Abstand zwischen dem niedrigsten Triplettzustand und dem niedrigsten angeregten Singulettzustand nicht oder nicht wesentlich größer als die thermische Energie, die durch $k_BT$ beschrieben wird, wobei $k_B$ für die Boltzmannkonstante und T für die Temperatur steht, so ist aus dem Triplettzustand durch thermische Anregung der erste angeregte Singulettzustand des Moleküls zugänglich und kann thermisch besetzt werden. Da dieser Singulettzustand ein emissiver Zustand ist, aus dem Fluoreszenz möglich ist, kann dieser Zustand zur Erzeugung von Licht verwendet werden. Somit ist prinzipiell die Umwandlung von bis zu 100 % der elektrischen Energie in Licht möglich bei der Verwendung rein organischer Materialien als Emitter. So wird im Stand der Technik eine externe Quanteneffizienz von mehr als 19 % beschrieben, was in derselben Größenordnung wie für phosphoreszierende OLEDs liegt. Somit ist es mit derartigen rein organischen Materialien möglich, sehr gute Effizienzen zu erreichen und gleichzeitig die Verwendung seltener Metalle wie Iridium oder Platin zu vermeiden. Weiterhin ist es mit solchen Materialien auch möglich, hocheffiziente blau emittierende OLEDs zu erzielen.

Bei TADF-Verbindungen handelt es sich oft um aromatische Verbindungen, die sowohl Donor- wie auch Akzeptorsubstituenten aufweisen (wie zum Beispiel in EP2599773A1 beschrieben).

Die im Stand der Technik beschriebenen Verbindungen, die TADF zeigen, werden durchweg mittels Sublimation im Vakuum aufgedampft, um elektronische Vorrichtungen enthaltend diese Verbindungen herzustellen.

Generell gibt es bei den Verbindungen aus dem Stand der Technik noch erheblichen Verbesserungsbedarf, insbesondere im Hinblick auf Prozessierbarkeit. Weiterhin zeigen die weiter unten beschriebenen erfindungsgemäßen Verbindungen eine erhöhte Luftstabilität gegenüber den organischen Metallkomplexen. Schließlich führen die aus Lösung prozessierten erfindungsgemäßen Verbindungen zu elektronischen Vorrichtungen, insbesondere organischen Elektrolumineszenzvorrichtungen, die weniger Kurzschlüsse zeigen.

Die der vorliegenden Erfindung zugrunde liegende technische Aufgabe besteht somit in der Bereitstellung Verbindungen, die gute Leistungsdaten in elektronischen Vorrichtungen aufweisen, welche die Verwendung von Metallen

in Metallkomplexen vermeiden und aus Lösung prozessiert werden können.

Überraschend wurde gefunden, dass die weiter unten näher beschriebenen Verbindungen den Nachteil aus dem Stand der Technik beseitigen und zu effizienten elektronischen Vorrichtungen führen, die leicht aus Lösung prozessiert werden können und sich somit für eine kostengünstige Massenproduktion elektronischer Vorrichtungen eignen.

Derartige organische Verbindungen, Zusammensetzungen und Formulierungen enthaltend diese Verbindungen, sowie elektronische Vorrichtungen, insbesondere organische Elektrolumineszenzvorrichtungen enthaltend diese Verbindungen und Zusammensetzungen sowie Verfahren zur Herstellung der Vorrichtungen sind daher der Gegenstand der vorliegenden Erfindung.

Gegenstand der vorliegenden Erfindung ist eine organische Verbindung der Formel (1) wie unten beschrieben, die einen Abstand zwischen dem ersten angeregten Triplett-Zustand ($T_1$) und dem ersten angeregten Singulett-Zustand ($S_1$) von kleiner oder gleich 0.15 eV aufweist. Triplett- und Singulett-Zustände sowie die Energien von HOMOs (highest occupied molecular orbitals) und LUMOs (lowest unoccupied molecular orbitals) werden vorliegend mit Hilfe eines quantenchemischen Verfahrens ermittelt, das im Beispielteil ausführlich offenbart ist.

Im Folgenden wird die lumineszente organische Verbindung, die einen Abstand zwischen dem niedrigsten Triplettzustand $T_1$ und dem ersten angeregten Singulettzustand $S_1$ von $\leq$ 0.15 eV aufweist, näher beschrieben. Hierbei handelt es sich um eine Verbindung, die TADF (thermally activated delayed fluorescence) zeigt. Diese Verbindung wird in der folgenden Beschreibung mit "TADF-Verbindung" abgekürzt.

Die erfindungsgemäßen organischen TADF-Verbindung ist eine kohlenstoffhaltige Verbindung, die keine Metalle enthält. Insbesondere ist die erfindungsgemäße TADF-Verbindung eine organische Verbindung, die aus den Elementen C, H, D, B, Si, N, P, O, S, F, Cl, Br und I aufgebaut ist.

Bevorzugt weisen die erfindungsgemäßen organischen TADF-Verbindung einen Abstand zwischen $S_1$ und $T_1$ von kleiner oder gleich 0.12 eV, besonders bevorzugt von kleiner oder gleich 0.10 eV, ganz besonders bevorzugt von kleiner oder gleich 0.08 eV und insbesondere bevorzugt von kleiner oder gleich 0.05 eV auf.

In einer bevorzugten Ausführungsform hat das $S_1$-Niveau der organischen TADF-Verbindung eine höhere Energie als das $T_1$-Niveau. Der Abstand der Energienieveaus $S_1$ und $T_1$ ist dann wie folgt definiert: $\Delta E = E(S_1)-E(T_1)$, wobei $\Delta E$ kleiner oder gleich 0.15 eV ist, bevorzugt ist er kleiner oder gleich 0.12 eV, besonders bevorzugt ist er kleiner oder gleich 0.10 eV, ganz besonders bevorzugt ist er kleiner oder gleich 0.08 eV und insbesondere bevorzugt ist er kleiner oder gleich 0.05 eV.

Singulett und Triplett-Energien werden vorliegend mit Hilfe quantenchemischer Verfahren ermittelt, die im Beispielteil ausführlich allgemein offenbart werden.

Die organische TADF-Verbindung ist eine organische Verbindung der allgemeinen Formel (1)

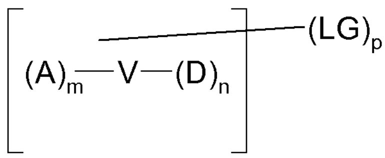

Formel (1)

wobei für die verwendeten Symbole und Indizes gilt:

- m ist eine ganze Zahl ausgewählt aus 1, 2, 3, 4 oder 5;
- n ist eine ganze Zahl ausgewählt aus 1, 2, 3, 4 oder 5;
- p ist eine ganze Zahl, die gleich 1 oder größer als 1 ist; bevorzugt ist p eine ganze Zahl von 1 bis 12, ganz bevorzugt eine ganze Zahl von 1 bis 10, ganz besonders bevorzugt eine ganze Zahl von 1 bis 8, insbesondere bevorzugt eine ganze Zahl von 1 bis 6 und weiterhin bevorzugt eine ganze Zahl von 1 bis 4;
- A ist eine Akzeptorgruppe, die eine Elektronenakzeptorgruppe darstellt, wobei die Elektronenakzeptorgruppe eine Cyanogruppe, Nitrilgruppe, Oxogruppe, $CF_3$ oder elektronenarme Heteroarylgruppe wie Pyrimidine, Pyrazine, Triazine enthält, die mit einem oder mehreren Resten $R^1$, die bei jedem Auftreten gleich oder verscheiden sein können, substituiert sein kann;
- D ist eine Donorgruppe, die eine Elektronendonorgruppe darstellt, wobei die Elektronendonorgruppe eine Diarylaminogruppe, Diarylheteroarylaminogruppe, Carbazolgruppe, Indenocarbazolgruppe oder Indolocarbazolgruppe

enthält, die mit einem oder mehreren Resten $R^1$, die bei jedem Auftreten gleich oder verscheiden sein können, substituiert sein kann;

LG ist eine löslichkeitsvermittelnde Gruppe, die an A, D und/oder V gebunden sein kann, bevorzugt ist sie an die Gruppen A und/oder D gebunden und ganz bevorzugt ist sie an die Gruppe D gebunden; wobei die Gruppe LG ausgewählt ist aus einer geradkettigen Alkyl- oder Alkoxygruppe mit 3 bis 15 C-Atomen;
einer verzweigten oder cyclischen Alkyl- oder Alkoxygruppe mit 3 bis 40 C-Atomen, bevorzugt mit 3 bis 30 C-Atomen, ganz bevorzugt mit 3 bis 20 C-Atomen, ganz besonders bevorzugt mit 3 bis 15 C-Atomen und insbesondere bevorzugt mit 3 bis 10 C-Atomen;
einem aromatischen oder heteroaromatischen Ringsystem mit 3 oder mehr aromatischen oder heteroaromatischen Ringen, die mir einem oder mehreren, gleichen oder verschiedenen Resten $R^1$ substituiert sein können;
einem aromatischen oder heteroaromatischen Ringsystem mit 1 oder 2 aromatischen oder heteroaromatischen Ringen, die mit einem oder mehreren, gleichen oder verschiedenen Resten $R^1$, die ungleich H sind, in ortho Position substituiert sind, wobei die Ringe mit weiteren, gleichen oder verschiedenen Resten $R^1$ substituiert sein können;
einer Gruppe der Formel (LG-1)

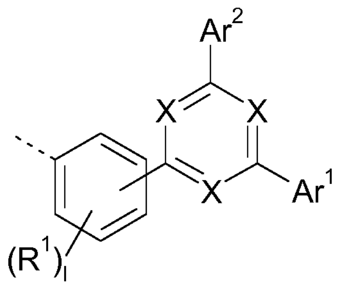

Formel (LG-1)

wobei für die verwendeten Symbole gilt:

$Ar^1$, $Ar^2$ sind jeweils unabhängig voneinander eine Aryl- oder Heteroarylgruppe, welche mit einem oder mehreren Resten $R^1$ substituiert sein kann,

X ist jeweils unabhängig voneinander N oder $CR^2$, wobei maximal eine der drei Gruppen X ein N sein darf; bevorzugt sind alle drei X gleich $CR^2$ und ganz bevorzugt ist X gleich CH,

$R^2$ ist jeweils unabhängig voneinander Wasserstoff, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Silylgruppe oder eine substituierte Ketogruppe mit 1 bis 40 C-Atomen, eine Alkoxy-carbonylgruppe mit 2 bis 40 C-Atomen, eine Aryloxycarbonyl-gruppe mit 7 bis 40 C-Atomen, eine vernetzbare Gruppe oder ein substituiertes oder unsubstituiertes aromatisches oder heteroaromatisches Ringsystem mit 5 bis 60 Ringatomen, oder eine Aryloxy- oder Heteroaryloxygruppe mit 5 bis 60 Ringatomen, oder eine Kombination dieser Systeme, wobei zwei oder mehrere der Gruppen $R^2$ ein mono- oder polycyclisches, aliphatisches oder aromatisches Ringsystem miteinander bilden können;

I ist 0, 1, 2, 3 oder 4;

wobei die gestrichelte Bindung die Bindung von LG an A, D oder V angibt.

V ist eine beliebige organische Brücke zwischen den Gruppen A und D oder eine Einfachbindung, wobei, wenn V eine Einfachbindung ist, entweder m oder n gleich 1 ist, und, wenn V eine organische Brücke darstellt, dann ist die Brücke V aus den Formeln (V-1) bis (V-7) ausgewählt,

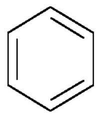

Formel (V-1)

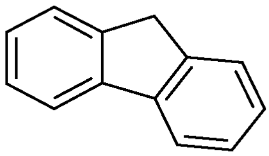

Formel (V-2)

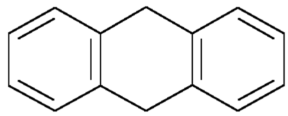

Formel (V-3)

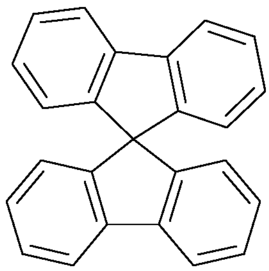

Formel (V-4)

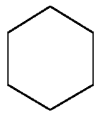

Formel (V-5)

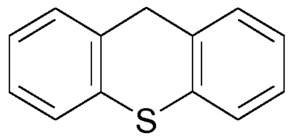

Formel (V-6)

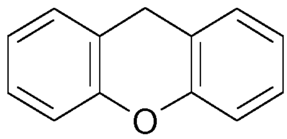

Formel (V-7)

wobei V mit einem oder mehreren Resten $R^1$, die bei jedem Auftreten gleich oder verscheiden sein können, substituiert sein kann;

$R^1$ ist gleich oder verscheiden bei jedem Auftreten Wasserstoff, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen ist oder eine Silylgruppe oder eine substituierte Ketogruppe mit 1 bis 40 C-Atomen, eine Alkoxycarbonylgruppe mit 2 bis 40 C-Atomen, eine Aryloxycarbonyl-gruppe mit 7 bis 40 C-Atomen, eine vernetzbare Gruppe oder ein substituiertes oder unsubstituiertes aromatisches oder heteroaromatisches Ringsystem mit 5 bis 60 Ringatomen, oder eine Aryloxy- oder Heteroaryloxygruppe mit 5 bis 60 Ringatomen, oder eine Kombination dieser Systeme ist, wobei zwei oder mehrere der Gruppen $R^1$ ein mono- oder polycyclisches, aliphatisches oder aromatisches Ringsystem miteinander bilden können, wobei bevorzugt ist wenn zwei oder mehrere der Gruppen $R^1$ kein mono- oder polycyclisches, aliphatisches oder aromatisches Ringsystem miteinander bilden können.

Ferner bevorzugt ist, dass die Verbindung der allgemeinen Formel (1) wenigstens 2, bevorzugt wenigstens 3, ganz bevorzugt wenigstens 4, ganz besonders bevorzugt wenigstens 5 und insbesondere wenigstens 6 aromatische Ringe enthält.

In einer weiteren bevorzugten Ausführungsform handelt es sich bei der organischen TADF-Verbindung um eine organische Verbindung mit einem Molekulargewicht von kleiner oder gleich 5000 g/mol, ganz bevorzugt von kleiner oder gleich 4000 g/mol, besonders bevorzugt von kleiner oder gleich 3000 g/mol, ganz besonders bevorzugt von kleiner oder gleich 2000 g/mol, insbesondere bevorzugt von kleiner oder gleich 1500 g/mol und noch bevorzugter von kleiner oder gleich 1000 g/mol.

Unter der Akzeptorgruppe A (auch Akzeptorsubstituent genannt) wird vorliegend eine Gruppe verstanden, die eine Elektronenakzeptorgruppe darstellt. Was unter einer Akzeptorgruppe verstanden wird, ist dem Fachmann gut bekannt. Es ist bevorzugt, wenn die Akzeptorgruppe einen negativen induktiven Effekt (-I) und/oder einen negativen mesomeren Effekt (-M) aufweist. Die Bestimmung dieser Eigenschaften mit Hilfe der Hammett-Gleichung ist dem Fachmann gut bekannt. Geeignete Akzeptorsubstituenten sind insbesondere Cyanogruppen, Nitrile, Pyrimidine, Pyrazine, =O (Oxogruppe, bspw. Ketone), aber auch $CF_3$ und beispielsweise elektronenarme Heteroarylgruppen, die auch weiter substituiert sein können. Bevorzugte Akkzeptorgruppen A sind dabei Cyanogruppen und Oxogruppen und ganz bevorzugt sind Cyanogruppen. Beispiele bevorzugter elektronenarmer Heteroarylgruppen sind ausgewählt aus der Gruppe

bestehend aus Triazinen, Pyrimidinen, Phosphinoxiden und Ketonen.

Unter der Donorgruppe D (auch Donorsubstituent genannt) wird vorliegend eine Gruppe verstanden, die eine Elektronendonorgruppe darstellt. Was unter einer Donorgruppe verstanden wird, ist dem Fachmann gut bekannt. Es ist bevorzugt, wenn die Donorgruppe einen positiven induktiven Effekt (+I) und/oder einen positiven mesomeren Effekt (+M) aufweist. Die Bestimmung dieser Eigenschaftenh mit Hilfe der Hammett-Gleichung ist dem Fachmann gut bekannt. Geeignete Donorsubstituenten sind insbesondere Diaryl- bzw. -heteroarylaminogruppen sowie Carbazolgruppen bzw. Carbazolderivate, wie Indenocarbazole oder Indolocarbazole, die jeweils bevorzugt über N an die Brücke V bzw. an die Gruppe A gebunden sind. Dabei können diese Gruppen auch weiter substituiert sein.

Unter löslichkeitsvermittelnder Gruppe LG wird vorliegend eine Gruppe verstanden, welche die Löslichkeit organischer Halbleitermaterialien in organischen Lösungsmitteln erhöhen, so dass sich Schichten aus Lösung mit einer Schichtdicke zwischen 10 und 400 nm, bevorzugt zwischen 10 und 200 nm herstellen lassen.

Löslichkeiten der erfindungsgemäßen Verbindungen werden im Rahmen der vorliegenden Erfindung mit dem im Beispielteil beschriebenen Verfahren bestimmt.

Sofern es sich bei den Gruppen $Ar^1$ und $Ar^2$ um Heteroarylgruppen handelt ist bevorzugt, dass die jeweilige Heteroarylgruppe maximal ein Heteroatom aufweist.

Bevorzugt ist, wenn die Gruppen $R^1$ und/oder $R^2$ in der Formel (LG-1) kein mono- oder polycyclisches, aliphatisches oder aromatisches Ringsystem miteinander und/oder mit dem Ring, an dem die Gruppe $R^1$ gebunden ist, bilden können.

Sofern nicht an andere Stelle andere Definition angegeben wurde enthält eine Arylgruppe im Sinne dieser Erfindung 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Thiophen, etc., oder eine kondensierte (anellierte) Aryl- oder Heteroarylgruppe, beispielsweise Naphthalin, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 80 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder $CH_2$-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte $CH_2$-Gruppen durch die oben genannten Gruppen ersetzt sein können; weiterhin können auch ein oder mehrere H-Atome durch D, F, Cl, Br, I, CN oder $NO_2$, bevorzugt F, Cl oder CN, weiter bevorzugt F oder CN, besonders bevorzugt CN ersetzt sein.

Unter einem aromatischen oder heteroaromatischen Ringsystem mit 5 - 30 bzw. 5 - 60 aromatischen Ringatomen, welches noch jeweils mit den oben genannten Resten R, $R^1$ oder $R^2$ substituiert sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Es ist bevorzugt, wenn die Phenylengruppe der Formel (LG-1) ortho oder meta verknüpft ist, ganz besonders bevorzugt ist eine meta Verknüpfung gemäß der folgenden Formel (LG-2)

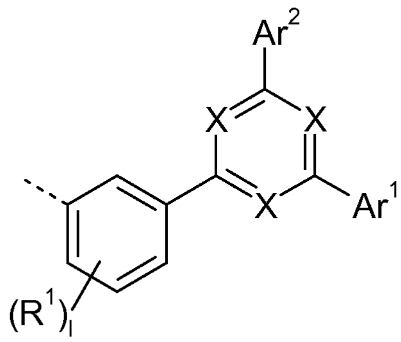

Formel (LG-2)

In einer besonders bevorzugten Ausführungsform der vorliegenden Erfindung ist LG abzugrenzen von den Akzeptorgruppen, d.h. die Gruppen LG enthalten keine elektronenarmen Gruppen, d.h. insbesondere auch keine elektronenarmen Heteroaromaten.

Beispiele besonders bevorzugter Gruppen LG sind in der folgenden Tabelle aufgeführt, wobei die Gruppen weiter mit einem oder mehreren gleichen oder verschiedenen Resten $R^1$ substituiert sein können wobei optional zwei oder mehr benachbarte Substituenten $R^1$ ein monocyclisches oder polycyclisches, aliphatisches, aromatisches oder heteroaromatisches Ringsystem bilden können; bevorzugt bilden zwei oder mehr benachbarte Substituenten $R^1$ kein monocyclisches oder polycyclisches, aliphatisches, aromatisches oder heteroaromatisches Ringsystem.

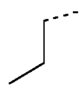

Formel (LG-5)

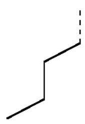

Formel (LG-6)

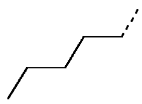

Formel (LG-7)

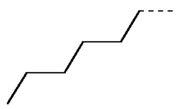

Formel (LG-8)

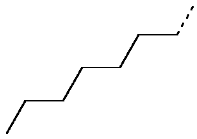
Formel (LG-9)

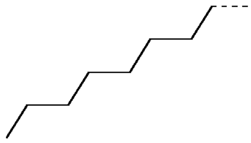
Formel (LG-10)

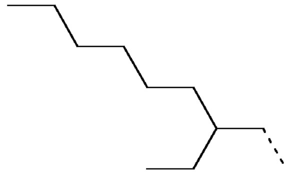
Formel (LG-11)

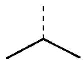
Formel (LG-12)

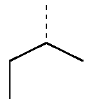
Formel (LG-13)

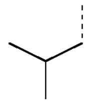
Formel (LG-14)

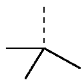
Formel (LG-15)

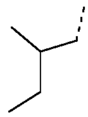
Formel (LG-16)

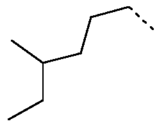
Formel (LG-17)

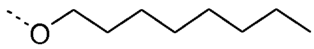
Formel (LG-18)

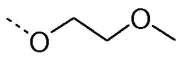
Formel (LG-19)

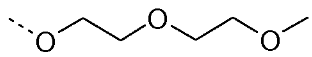
Formel (LG-20)

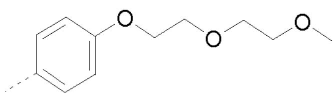
Formel (LG-21)

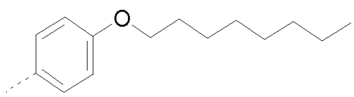
Formel (LG-22)

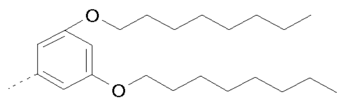
Formel (LG-23)

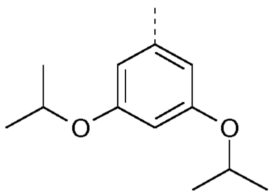
Formel (LG-24)

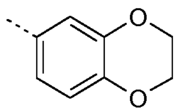
Formel (LG-25)

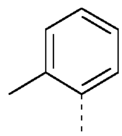
Formel (LG-26)

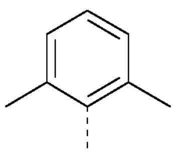
Formel (LG-27)

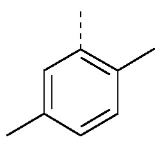
Formel (LG-28)

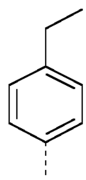
Formel (LG-29)

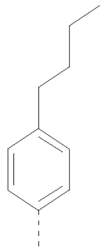

Formel (LG-30)

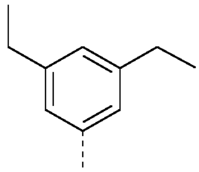

Formel (LG-31)

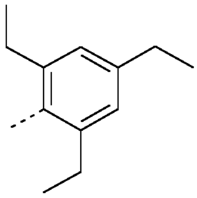

Formel (LG-32)

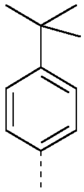

Formel (LG-33)

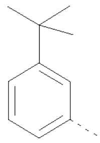

Formel (LG-34)

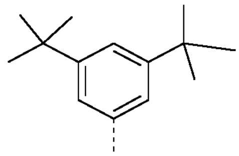

Formel (LG-35)

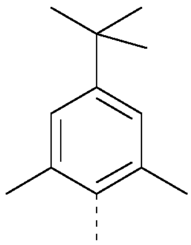

Formel (LG-36)

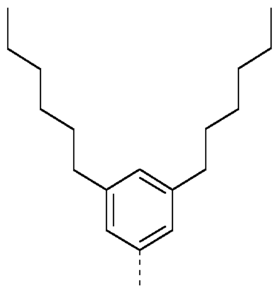

Formel (LG-37)

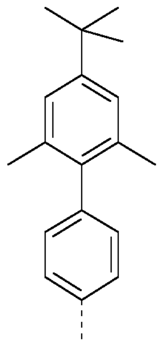

Formel (LG-38)

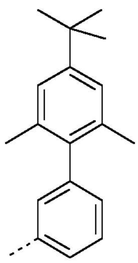

Formel (LG-39)

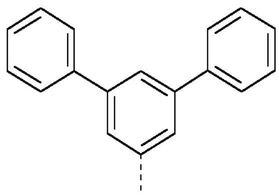

Formel (LG-40)

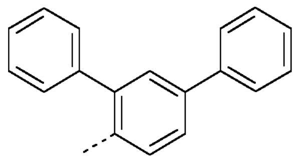

Formel (LG-41)

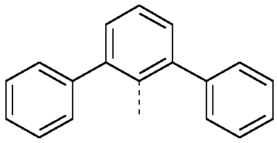

Formel (LG-42)

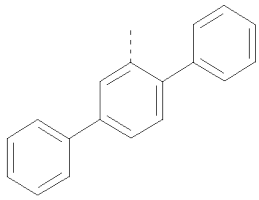

Formel (LG-43)

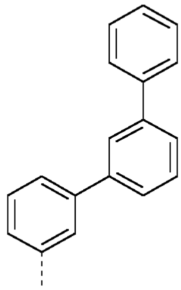

Formel (LG-44)

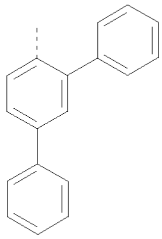

Formel (LG-45)

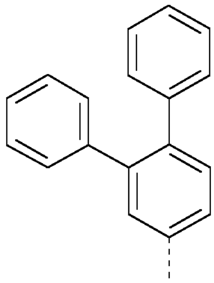

Formel (LG-46)

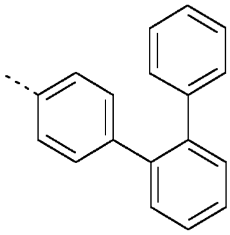

Formel (LG-47)

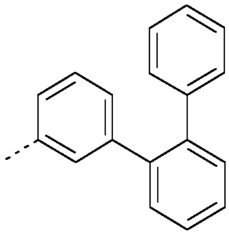

Formel (LG-48)

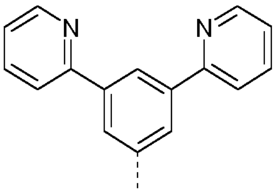

Formel (LG-49)

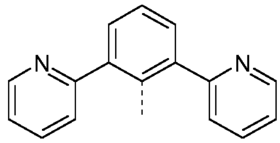

Formel (LG-50)

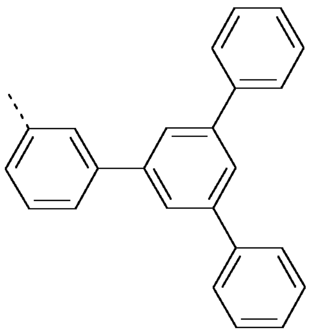

Formel (LG-51)

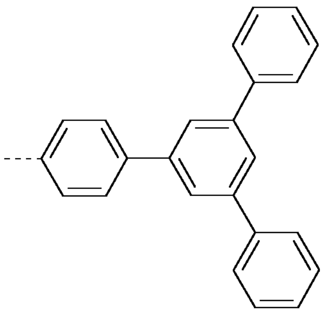

Formel (LG-52)

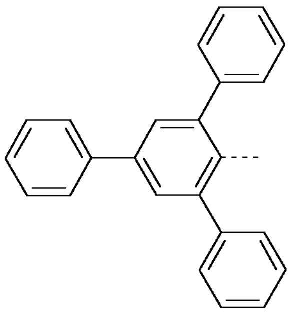

Formel (LG-53)

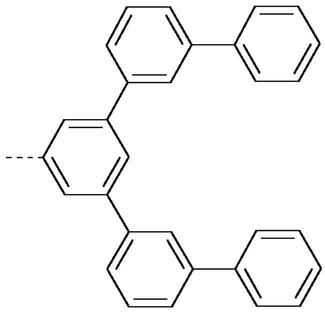

Formel (LG-54)

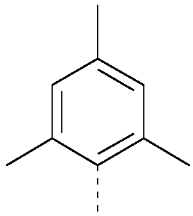

Formel (LG-27a)

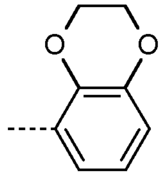

Formel (LG-55)

Eine weitere bevorzugte lösliche Gruppe LG weist die Formel -N($X^3$)($X^4$) auf, wobei wenigstens eine der beiden Gruppen $X^3$ und $X^4$ eine der oben genannten löslichen Gruppen (LG-1) bis (LG-55) ist, wobei ganz bevorzugt ist, wenn beide Gruppen $X^3$ und $X^4$ aus den oben genannten löslichen Gruppen (LG-1) bis (LG-55) ausgewählt sind. Besonders bevorzugt ist, wenn beide Gruppen $X^3$ und $X^4$ aus den oben genannten löslichen Gruppen (LG-1) bis (LG-55) ausgewählt sind und zudem identisch sind.

Sofern nur eine der beiden Gruppen $X^3$ und $X^4$ eine der Gruppen (LG-1) bis (LG-55) darstellt, ist die andere Gruppe bevorzugt eine Aryl- oder Heteroarylgruppe, welche mit einem oder mehreren Resten $R^1$ substituiert sein kann, wobei ganz bevorzugt ist wenn es sich um eine Phenyl-, Biphenyl-, Terphenyl- oder Quarterphenylgruppe handelt, die mit einem oder mehreren Resten $R^1$ substituiert sein kann, wobei besonders bevorzugt ist, wenn es sich um eine unsubstituierte Phenyl-, Biphenyl-, Terphenyl- oder Quarterphenylgruppe handelt.

Weitere bevorzugte Gruppen LG sind solche, die dadurch erhalten werden, dass eine oder mehrere der oben genannten Gruppen (LG-1) bis (LG-55) an eine Phenylengruppe gebunden sind. Beispielsweise können mit Hilfe des Gruppe (LG-8) auch folgende löslichkeitsvermittelnde Gruppen aufgebaut werden:

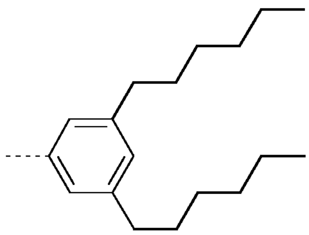

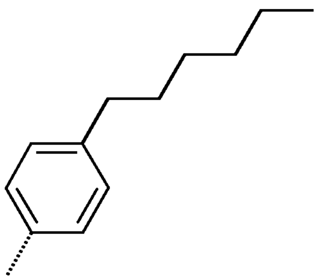

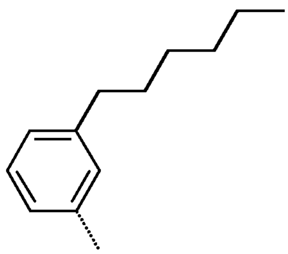

Bevorzugte Gruppen LG sind also auch solche der folgenden Formel (LGX)

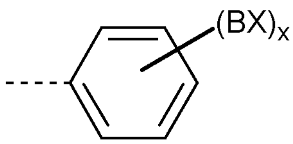

Formel (LGX)

wobei der Index x für eine ganze Zahl von 1 bis 5 steht, bevorzugt steht Index x für 1 oder 2;

BX ist gleich oder verschieden voneinander eine Gruppe der Formeln (LG-1) bis (LG-55).

Weiterhin bevorzugt sind solche Gruppen LG, die eine der Gruppen der Formeln (LG-1) bis (LG-55) oder (LGX) enthalten.

In einer bevorzugten Ausführungsform enthält die Verbindung der allgemeinen Formel (1) in den Gruppen A, D und den Gruppen der Formeln (LG-1) bis (LG-55), bevorzugt 3 bis 15, ganz bevorzugt 3 bis 12, ganz besonders bevorzugt 3 bis 10 und insbesondere 4 bis 10 aromatische Ringe.

Weiterhin bevorzugt ist, wenn die Verbindung der Formel (1) wenigstens einen heterocyclischen Ring enthält.

Ganz bevorzugt enthält die Verbindung der allgemeinen Formel (1) wenigstens einen heteroaromatischen Ring

oder ein heteroaromatisches Ringsystem.

Bei der TADF-Verbindung handelt es sich bevorzugt um eine aromatische Verbindung, die sowohl Donor- wie auch Akzeptorsubstituenten aufweist, wobei das LUMO und das HOMO der Verbindung räumlich nur schwach überlappen. Was unter Donor- bzw. Akzeptorsubstituenten verstanden wird, ist dem Fachmann prinzipiell bekannt und oben bereits erläutert worden. Geeignete Donorsubstituenten sind insbesondere Diaryl- bzw. -heteroarylaminogruppen sowie Carbazolgruppen bzw. Carbazolderivate, die jeweils bevorzugt über N an die aromatische Verbindung gebunden sind. Dabei können diese Gruppen auch weiter substituiert sein. Geeignete Akzeptorsubstituenten sind insbesondere Cyanogruppen, aber auch beispielsweise elektronenarme Heteroarylgruppen, die auch weiter substituiert sein können.

In einer bevorzugten Ausführungsform betrifft die vorliegende Erfindung eine organische TADF-Verbindung der allgemeinen Formel (1) mit einer geringen räumlichen Überlappung der Molekülorbitale A, die bei bestimmten elektronischen Übergängen beteiligt sind (Charge-Transfer-Zustände).

Die Überlappung der Molekülorbitale A wird vorliegend mit Hilfe eines quantenchemischen Verfahrens ermittelt, das im Beispielteil ausführlich offenbart ist.

Vorliegend bedeutet eine geringe Überlappung der Molekülorbitale, dass der Wert des Parameters A 0.3 oder kleiner ist, bevorzugt ist er 0.2 oder kleiner, ganz bevorzugt ist er 0.15 oder kleiner, ganz besonders bevorzugt ist er 0.1 oder kleiner und insbesondere bevorzugt ist er 0.05 oder kleiner.

Orbitalenergien sowie die Überlappung der Molekülorbitale werden vorliegend mit Hilfe quantenchemischer Verfahren ermittelt, die im Beispielteil ausführlich allgemein offenbart werden.

Weiterhin ist es bevorzugt, wenn die organische TADF-Verbindung eine kurze Abklingzeit aufweist. Dabei ist die Abklingzeit bevorzugt kleiner oder gleich 50 µs. Wie die Bestimmung der Abklingzeit im Sinne der vorliegenden Erfindung durchgeführt wird, ist im Beispielteil ausführlich allgemein offenbart.

Besonders bevorzugte TADF-Verbindungen im Sinne der vorliegenden Erfindung sind solche der allgemeinen Formel (2),

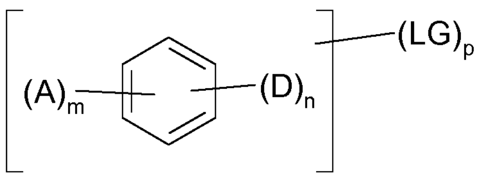

Formel (2)

wobei den verwendeten Indizes und Symbolen die oben genannten Bedeutungen zukommen. Weiterhin stellen die an anderer Stelle der vorliegenden Erfindung für die Indizes und Symbole offenbarten bevorzugten Ausführungsformen auch bevorzugte Ausführungsformen für die Verbindung der Formel (2) dar.

Die ein oder mehrere Gruppen LG binden hierbei bevorzugt an die eine oder die mehreren Gruppen D.

Ganz besonders bevorzugte TADF-Verbindungen sind dabei solche der Formel (3), wobei pp gleich 1, 2 oder 3 ist, bevorzugt ist pp gleich 1 oder 2 und ganz bevorzugt ist pp gleich 2.

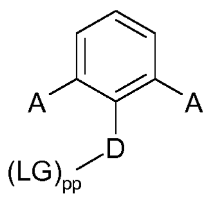

Formel (3)

Insbesondere bevorzugte TADF-Verbindungen sind dabei solche der Formel (4).

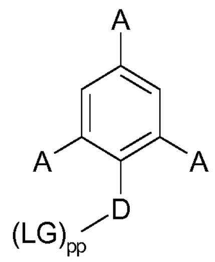

Formel (4)

Weiterhin insbesondere bevorzugte TADF-Verbindungen sind dabei solche der Formel (5), wobei pp gleich oder verschieden sein kann bei jedem Auftreten; q', q" und q'" sind unabhängig voneinander 0 oder 1 wobei die Summe q'+q"+q'" 1, 2 oder 3 beträgt.

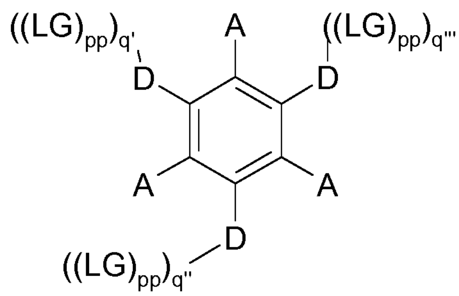

Formel (5)

Weiterhin insbesondere bevorzugte TADF-Verbindungen sind dabei solche der Formel (6), wobei pp gleich oder verschieden sein kann bei jedem Auftreten; q', q", q'" und q"" sind unabhängig voneinander 0 oder 1 wobei die Summe q'+q"+q'"+q"" 1, 2, 3 oder 4 beträgt.

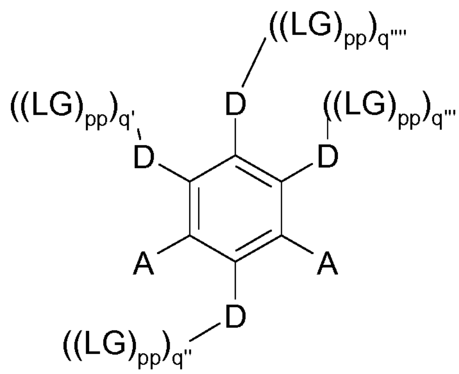

Formel (6)

Weiterhin insbesondere bevorzugte TADF-Verbindungen sind dabei solche der Formel (7), wobei pp gleich oder verschieden sein kann bei jedem Auftreten; q' und q" sind unabhängig voneinander 0 oder 1 wobei die Summe q'+q" 1 oder 2 beträgt.

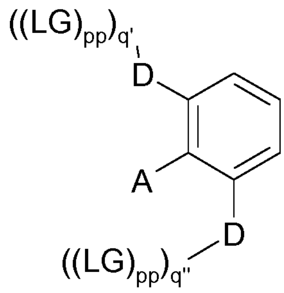

Formel (7)

Weiterhin insbesondere bevorzugte TADF-Verbindungen sind dabei solche der Formel (8), wobei pp gleich oder verschieden sein kann bei jedem Auftreten; q', q" und q'" sind unabhängig voneinander 0 oder 1 wobei die Summe q'+q"+q''' 1, 2 oder 3 beträgt

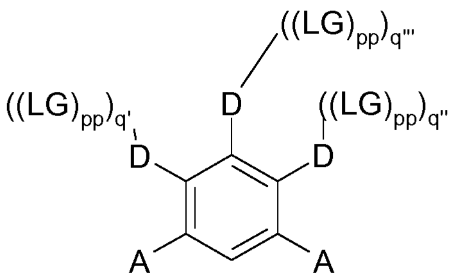

Formel (8)

Es ist ferner besonders bevorzugt, wenn A in den Verbindungen der Formeln (2) bis (8) gleich CN ist. Es ist noch bevorzugter, wenn A in den Verbindungen der Formeln (2) bis (8) gleich CN ist und D ein Carbazol darstellt, wobei das Carbazol mit einem oder mehreren Resten $R^1$ weiter substituiert sein kann.

Besonders bevorzugte TADF-Verbindungen im Sinne der vorliegenden Erfindung sind solche der allgemeinen Formel (9), wobei das Spirobifluoren mit einem oder mehreren Resten $R^1$, die gleich oder verschieden sein können bei jedem Auftreten, substituiert sein kann.

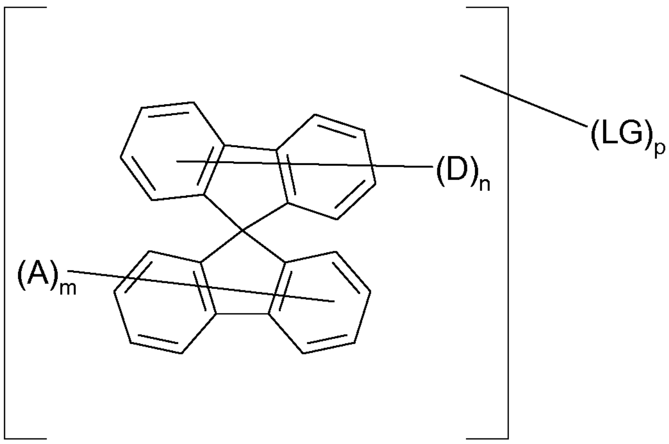

Formel (9)

wobei den verwendeten Indizes und Symbolen die oben genannten Bedeutungen zukommen. Weiterhin stellen die an anderer Stelle der vorliegenden Erfindung für die Indizes und Symbole offenbarten bevorzugten Ausführungsformen auch bevorzugte Ausführungsformen für die Verbindung der Formel (9) dar. Formel (9) macht deutlich, dass A und D auf unterschiedlichen Seiten der Spiroverbindung gebunden sind.

Ganz besonders bevorzugte TADF-Verbindungen sind dabei auch solche der Formel (10).

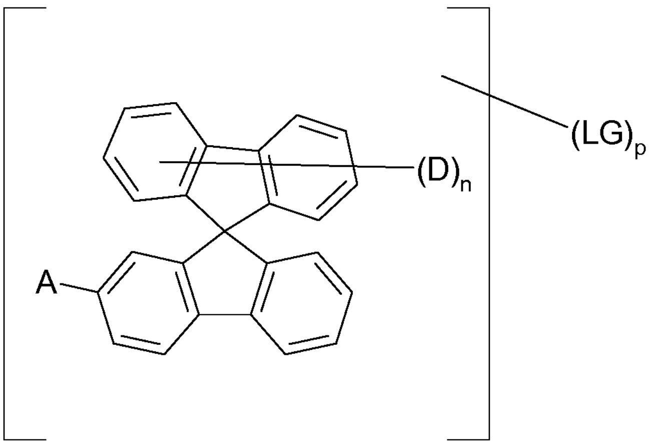

Formel (10)

Insbesondere bevorzugte TADF-Verbindungen sind dabei solche der Formel (11), wobei pp gleich 1, 2 oder 3 ist, bevorzugt ist pp gleich 1 oder 2 und ganz bevorzugt ist pp gleich 2.

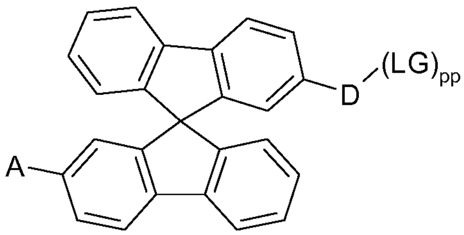

Formel (11)

Ferner ganz besonders bevorzugte TADF-Verbindungen sind dabei auch solche der Formel (12).

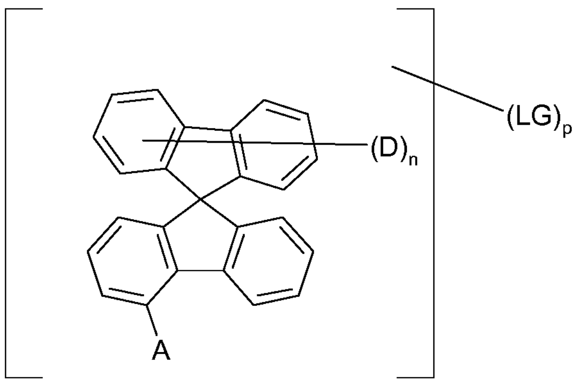

Formel (12)

Insbesondere bevorzugte TADF-Verbindungen sind dabei solche der Formel (13), wobei pp gleich 1, 2 oder 3 ist, bevorzugt ist pp gleich 1 oder 2 und ganz bevorzugt ist pp gleich 2.

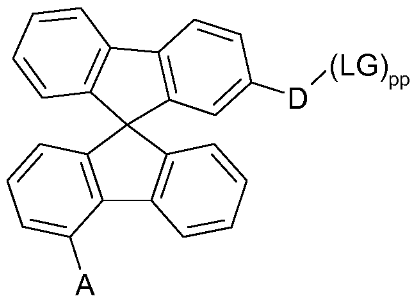

Formel (13)

Ferner ganz besonders bevorzugte TADF-Verbindungen sind solche der Formel (14).

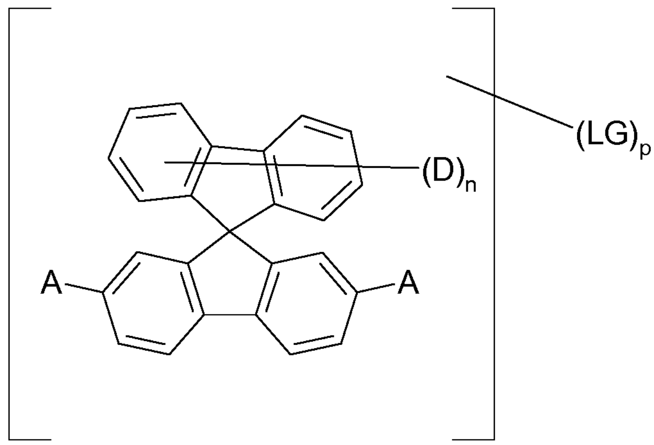

Formel (14)

Insbesondere bevorzugte TADF-Verbindungen sind dabei solche der Formel (15), wobei pp gleich oder verschieden sein kann bei jedem Auftreten; q' und q" sind unabhängig voneinander 0 oder 1 wobei die Summe q'+q" 1 oder 2 beträgt.

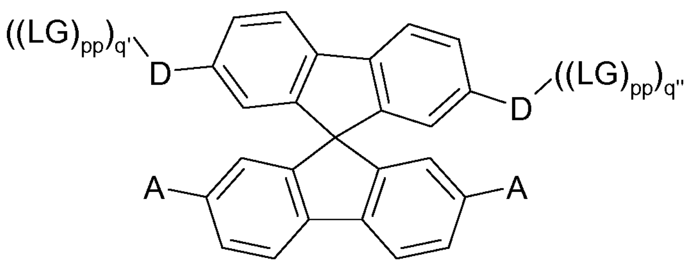

Formel (15)

Weiterhin insbesondere bevorzugte TADF-Verbindungen sind dabei solche der Formel (16), wobei pp gleich 1, 2 oder 3 ist, bevorzugt ist pp gleich 1 oder 2 und ganz bevorzugt ist pp gleich 2.

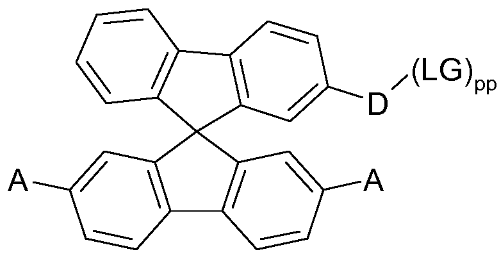

Formel (16)

Weiterhin ganz besonders bevorzugte TADF-Verbindungen sind solche der Formel (17)

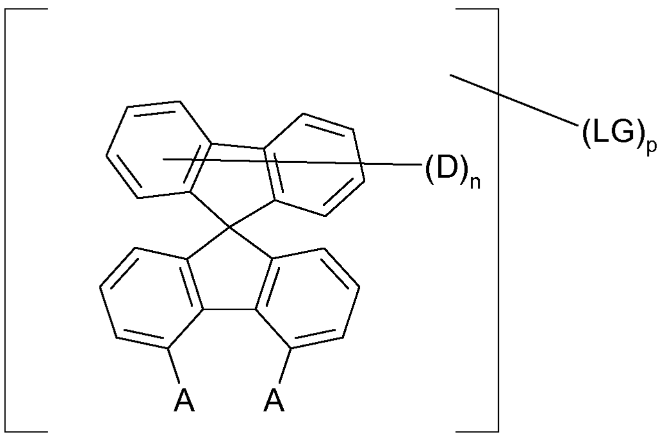

Formel (17)

Insbesondere bevorzugte TADF-Verbindungen sind dabei solche der Formel (18), wobei pp gleich oder verschieden sein kann bei jedem Auftreten; q' und q" sind unabhängig voneinander 0 oder 1 wobei die Summe q'+q" 1 oder 2 beträgt.

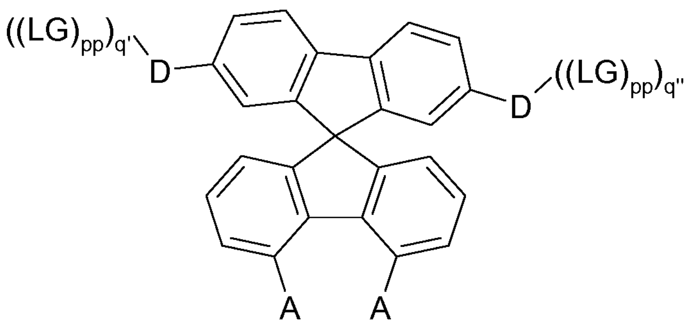

Formel (18)

Weiterhin insbesonders bevorzugte TADF-Verbindungen sind dabei solche der Formel (20), wobei pp gleich 1, 2 oder 3 ist, bevorzugt ist pp gleich 1 oder 2 und ganz bevorzugt ist pp gleich 2.

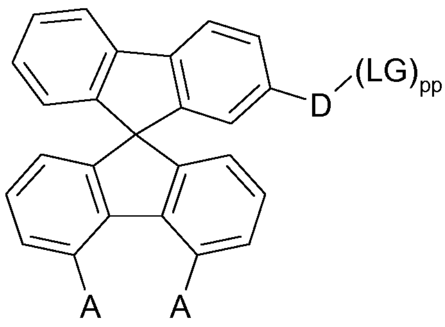

Formel (20)

Weiterhin ganz besonders bevorzugte TADF-Verbindungen sind solche der Formel (21)

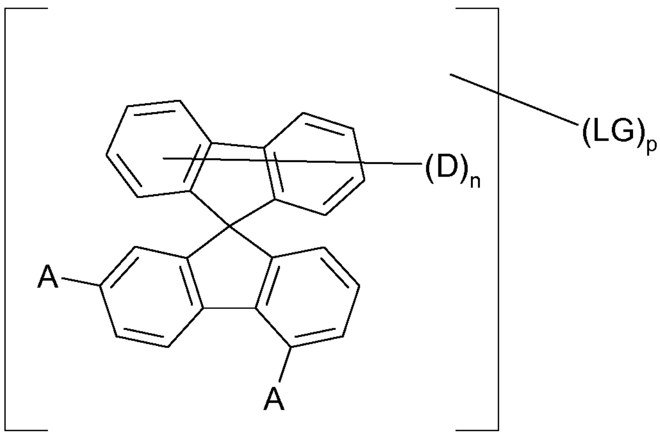

Formel (21)

Insbesonders bevorzugte TADF-Verbindungen sind dabei solche der Formel (22), wobei pp gleich oder verschieden sein kann bei jedem Auftreten; q' und q" sind unabhängig voneinander 0 oder 1 wobei die Summe q'+q" 1 oder 2 beträgt.

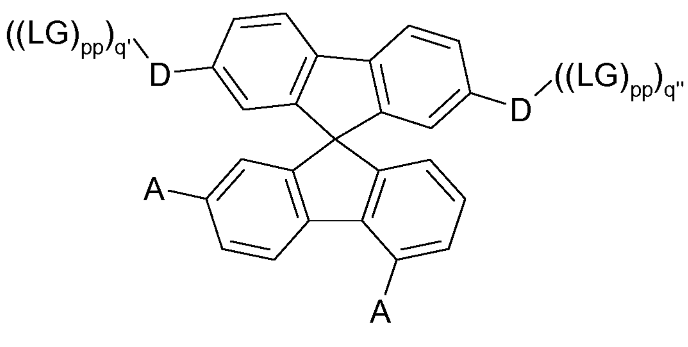

Formel (22)

Weiterhin insbesonders bevorzugte TADF-Verbindungen sind dabei solche der Formel (23), wobei pp gleich 1, 2 oder 3 ist, bevorzugt ist pp gleich 1 oder 2 und ganz bevorzugt ist pp gleich 2.

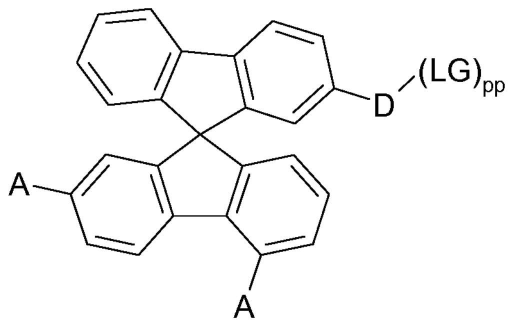

Formel (23)

Weiterhin insbesondere bevorzugte TADF-Verbindungen sind dabei solche der Formel (24), wobei pp gleich 1, 2 oder 3 ist, bevorzugt ist pp gleich 1 oder 2 und ganz bevorzugt ist pp gleich 2.

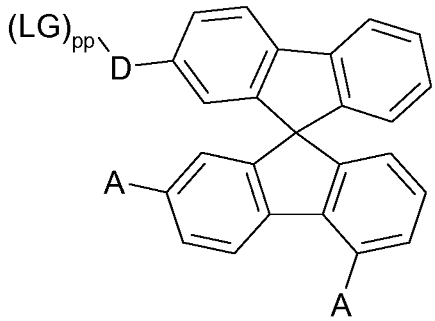

Formel (24)

Die ein oder mehreren Gruppen LG in den Verbindungen der Formeln (6) bis (24) binden hierbei bevorzugt an Gruppe(n) D.

Ganz besonders bevorzugte TADF-Verbindungen sind dabei auch solche der Formel (25).

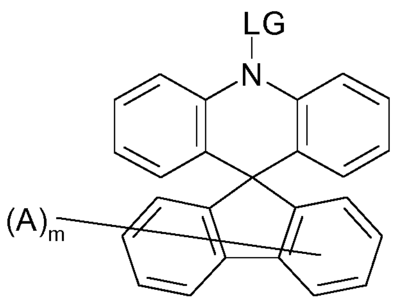

Formel (25)

Ferner ganz besonders bevorzugte TADF-Verbindungen sind solche der Formel (26)

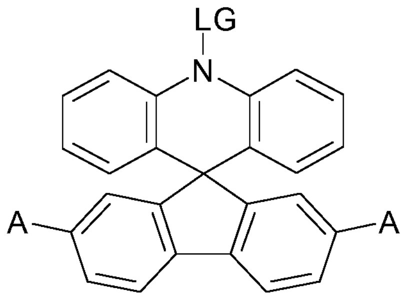

Formel (26)

Insbesondere bevorzugte TADF-Verbindungen sind dabei solche der Formel (27).

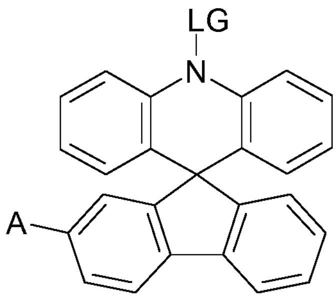

Formel (27)

Ganz besonders bevorzugte TADF-Verbindungen sind dabei auch solche der Formeln (28) und (29).

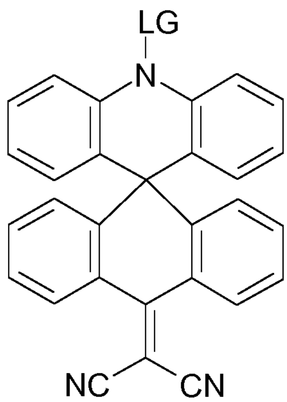

Formel (28)

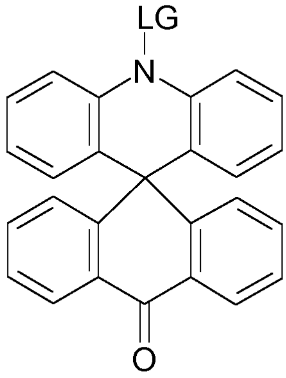

Formel (29)

Die Verbindungen der Formeln (2) bis (29) können dabei mit einem oder mehreren Resten $R^1$, die gleich oder verscheiden sein können bei jedem Auftreten, substituiert sein.

Die folgende Übersicht enthält eine exemplarische Übersicht erfindungsgemäßer TADF-Verbindungen.

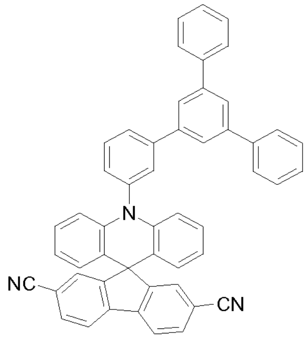

Formel (E-1)

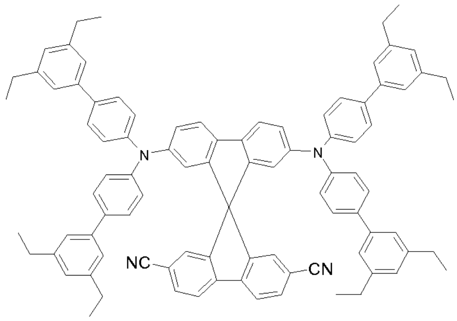

Formel (E-2)

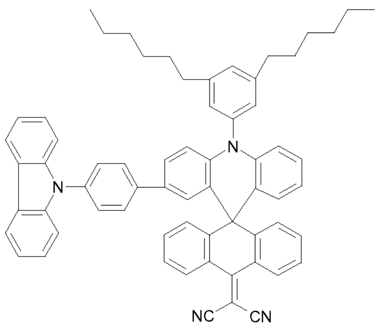

Formel (E-3)

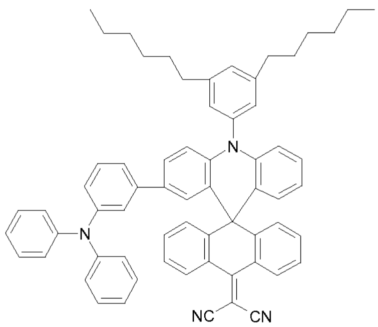

Formel (E-4)

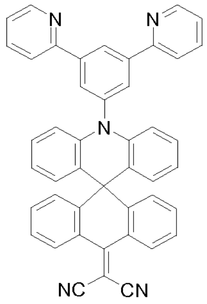

Formel (E-5)

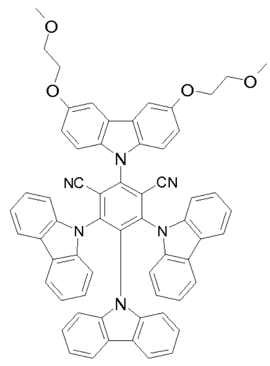

Formel (E-6)

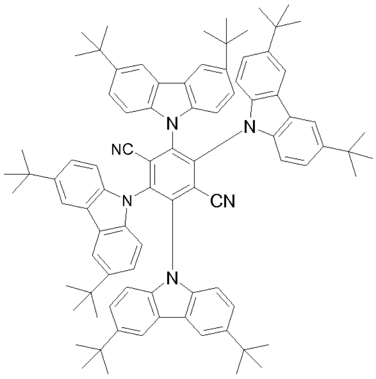

Formel (E-7)

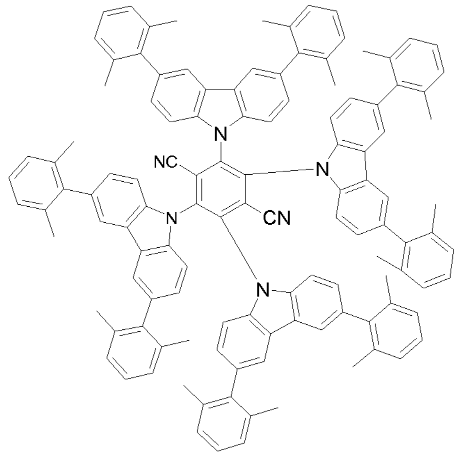

Formel (E-8)

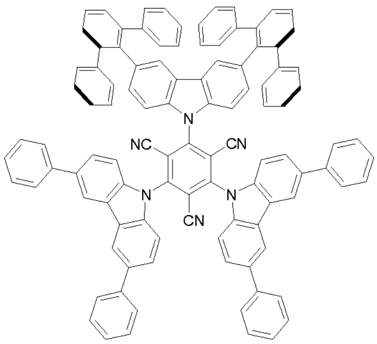

Formel (E-9)

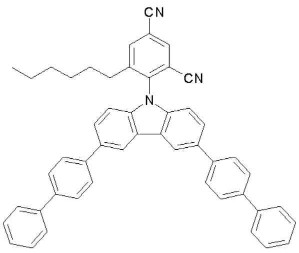

Formel (E-10)

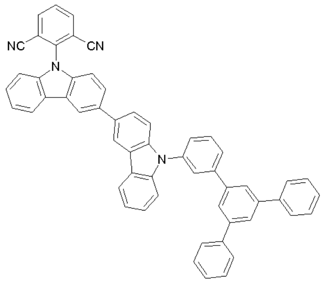

Formel (E-11)

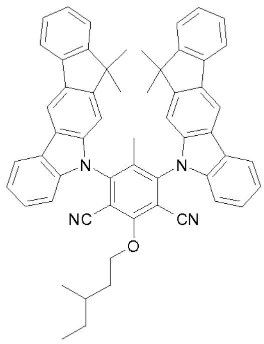

Formel (E-12)

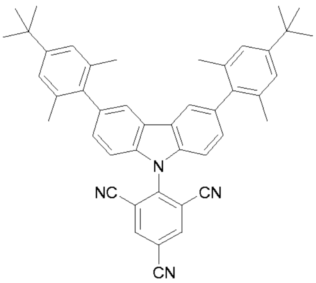

Formel (E-13)

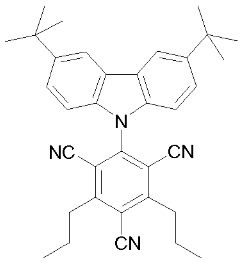

Formel (E-14)

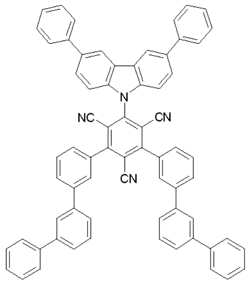

Formel (E-15)

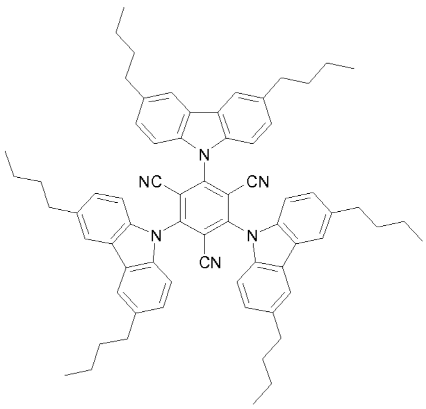

Formel (E-16)

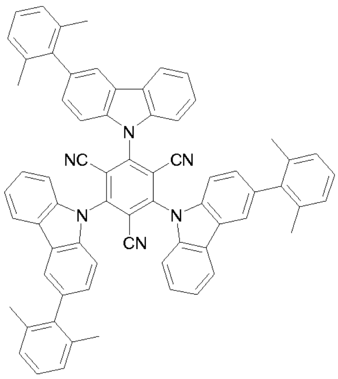

Formel (E-17)

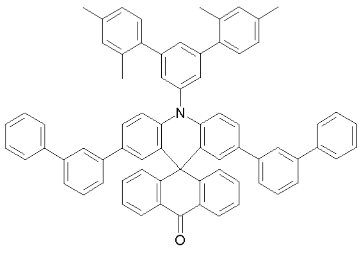

Formel (E-18)

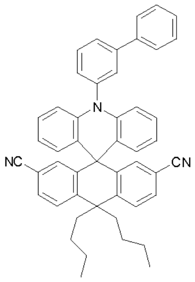

Formel (E-19)

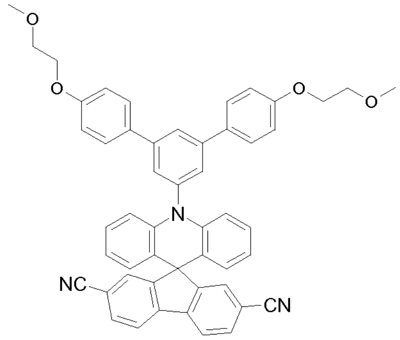

Formel (E-20)

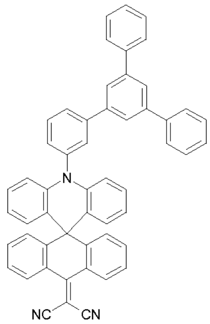

Formel (E-21)

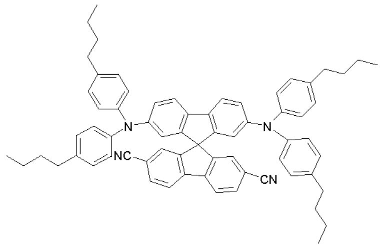

Formel (E-22)

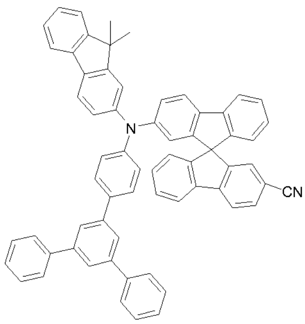

Formel (E-23)

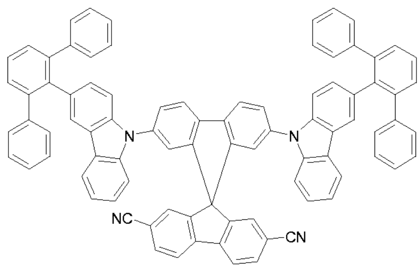

Formel (E-24)

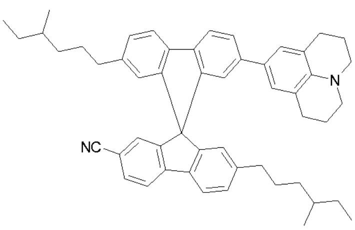

Formel (E-25)*

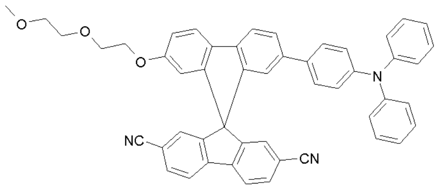

Formel (E-26)

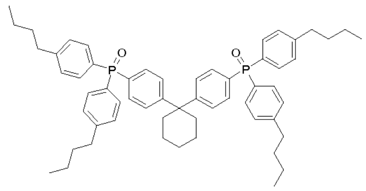

Formel (E-27)*

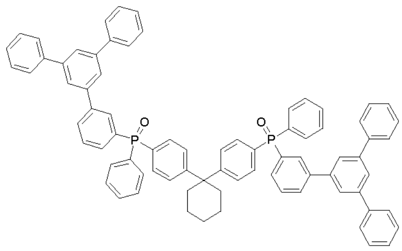

Formel (E-28)*

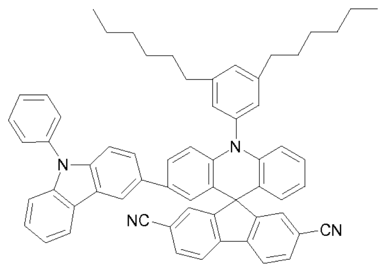

Formel (E-29)

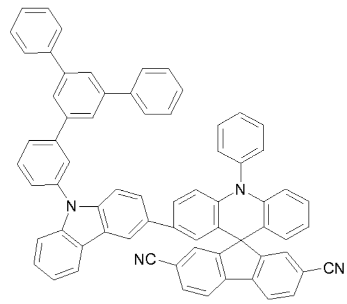

Formel (E-30)

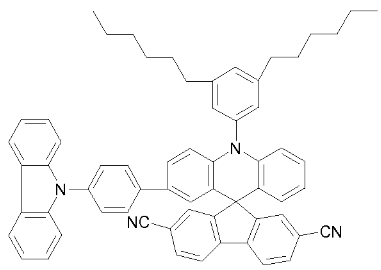

Formel (E-31)

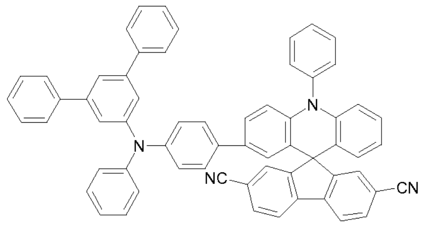

Formel (E-32)

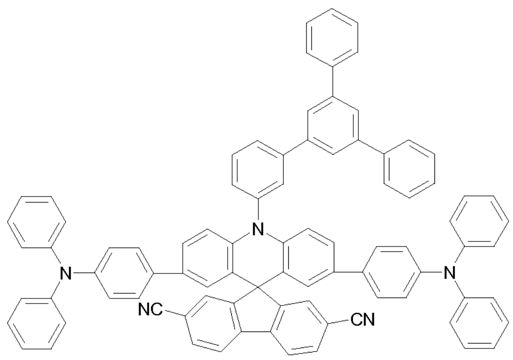

Formel (E-33)

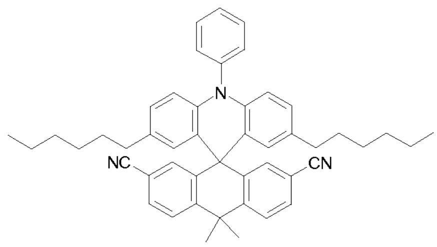

Formel (E-34)

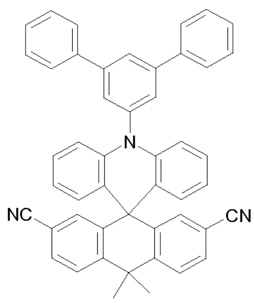

Formel (E-35)

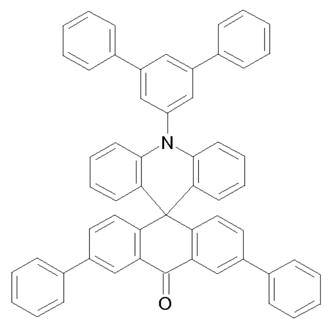

Formel (E-36)

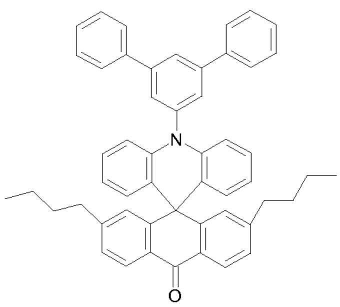

Formel (E-37)

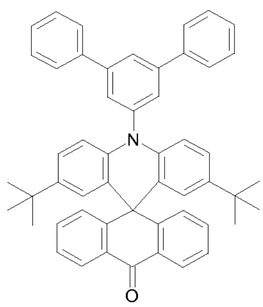

Formel (E-38)

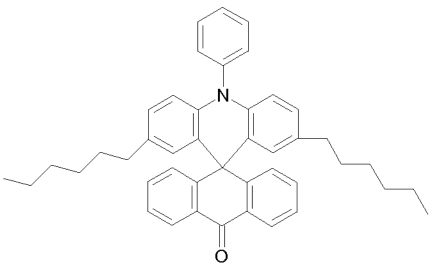

Formel (E-39)

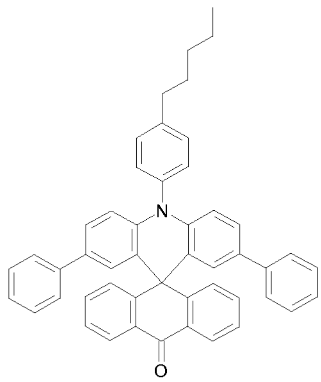

Formel (E-40)

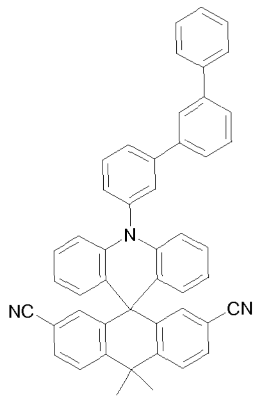

Formel (E-41)

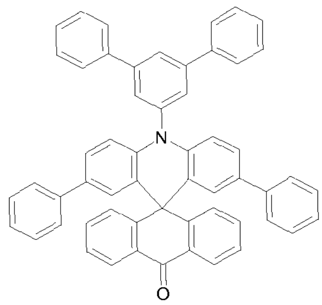

Formel (E-42)

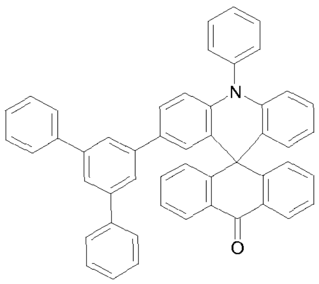

Formel (E-43)

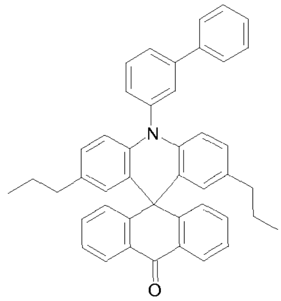

Formel (E-44)

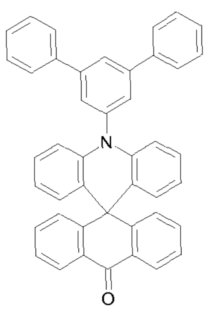

Formel (E-45)

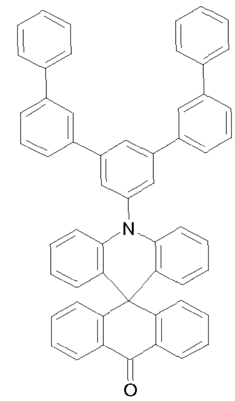

Formel (E-46)

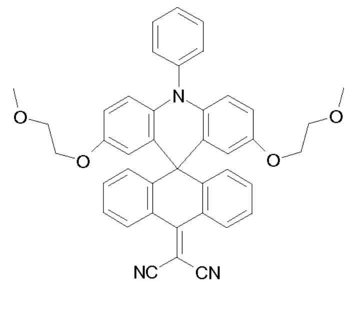

Formel (E-47)

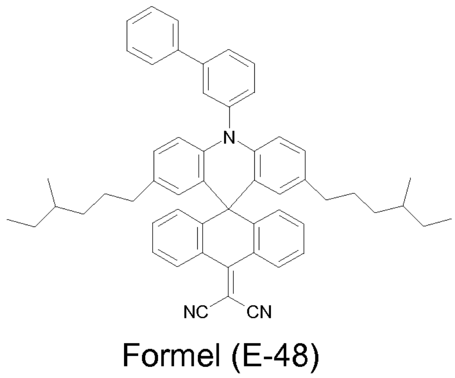

Formel (E-48)

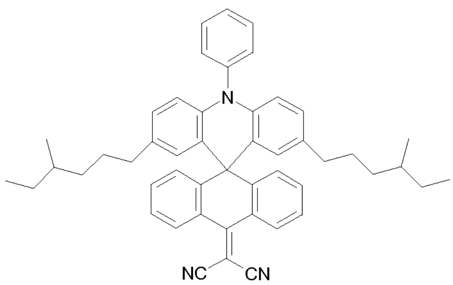

Formel (E-49)

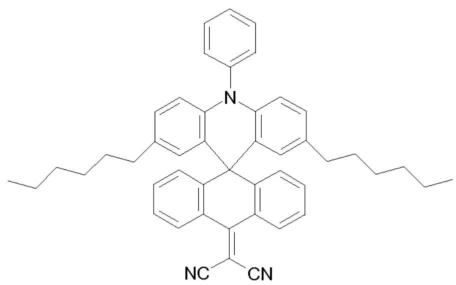

Formel (E-50)

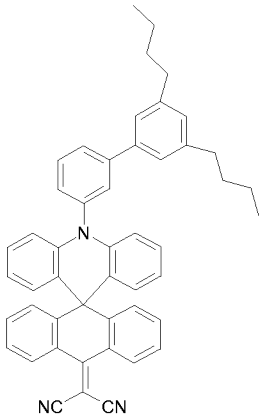

Formel (E-51)

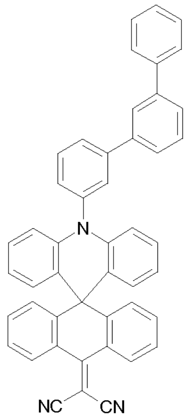

Formel (E-52)

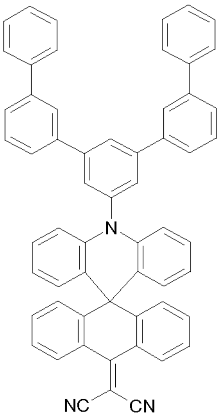

Formel (E-53)

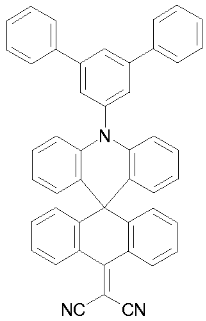

Formel (E-54)

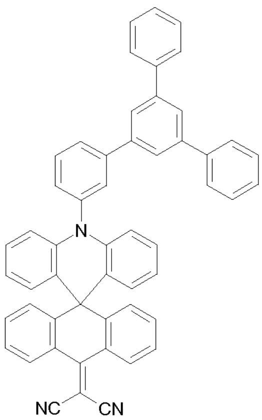

Formel (E-55)

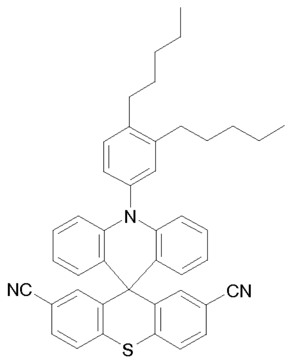

Formel (E-56)

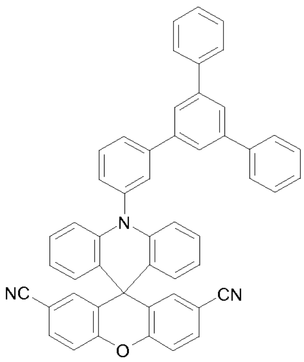

Formel (E-57)

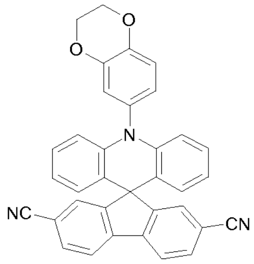

Formel (E-58)

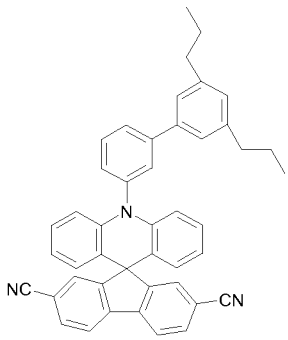

Formel (E-59)

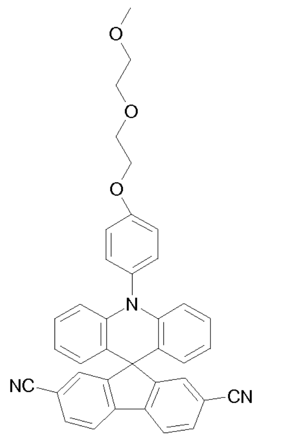

Formel (E-60)

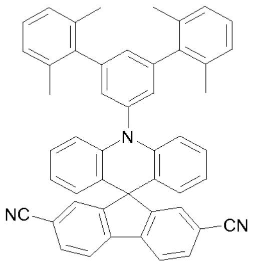

Formel (E-61)

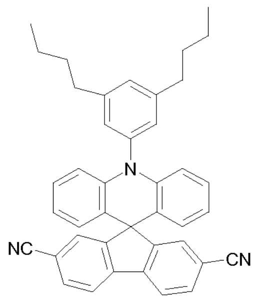

Formel (E-62)

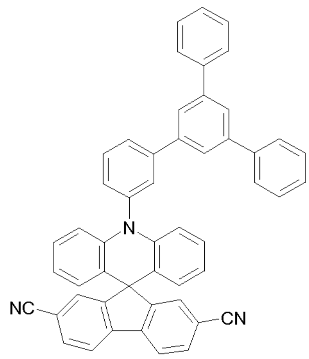

Formel (E-63)

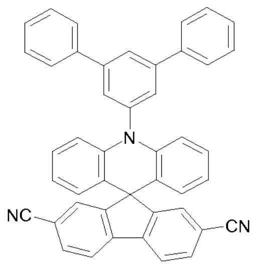

Formel (E-64)

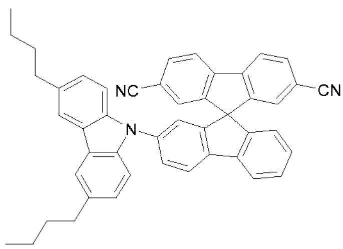

Formel (E-65)

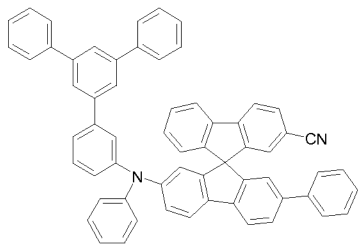

Formel (E-66)

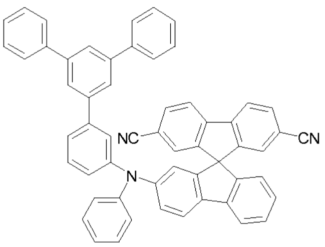

Formel (E-67)

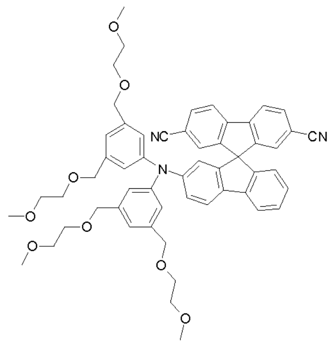

Formel (E-68)

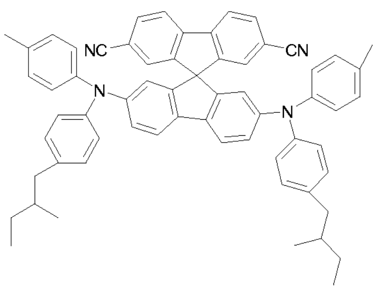

Formel (E-69)

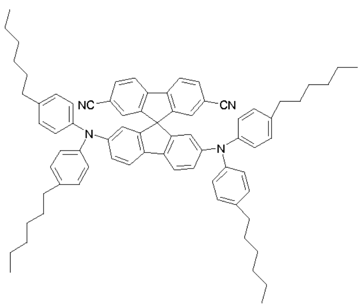

Formel (E-70)

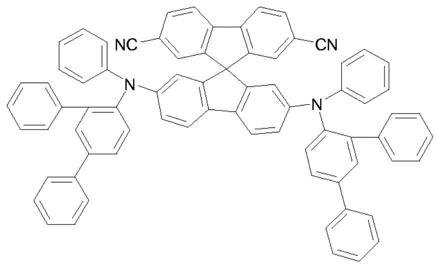

Formel (E-71)

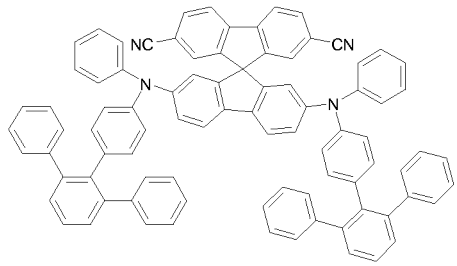

Formel (E-72)

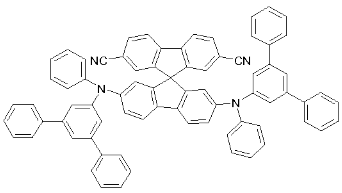

Formel (E-73)

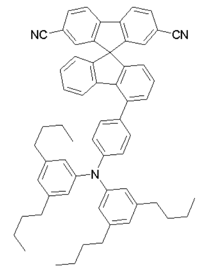

Formel (E-74)

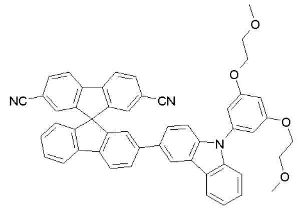

Formel (E-75)

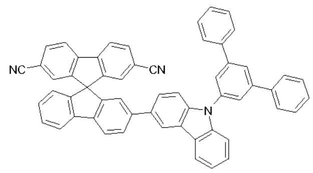

Formel (E-76)

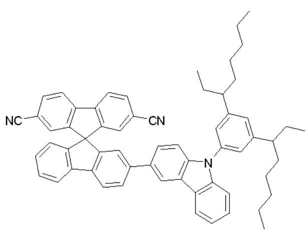

Formel (E-77)

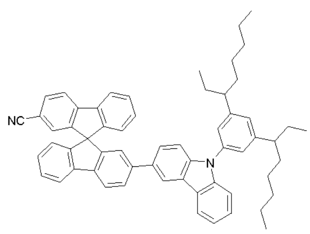

Formel (E-78)

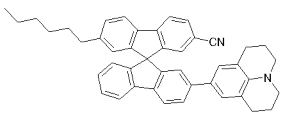

Formel (E-79)*

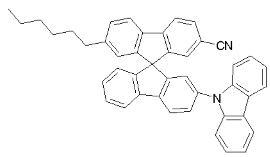

Formel (E-80)

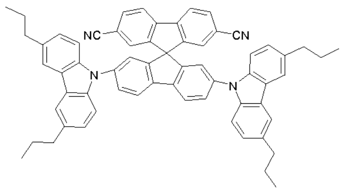

Formel (E-81)

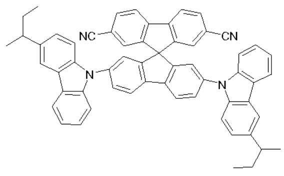

Formel (E-82)

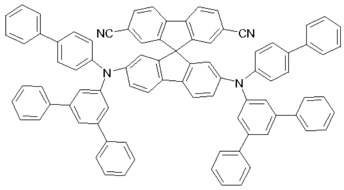

Formel (E-83)

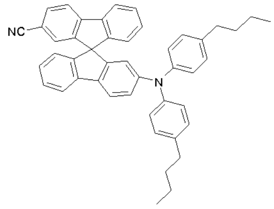

Formel (E-84)

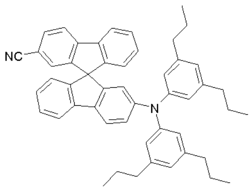

Formel (E-85)

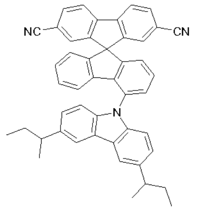

Formel (E-86)

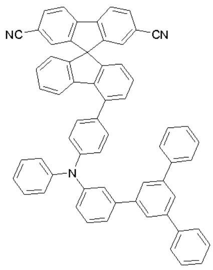

Formel (E-87)

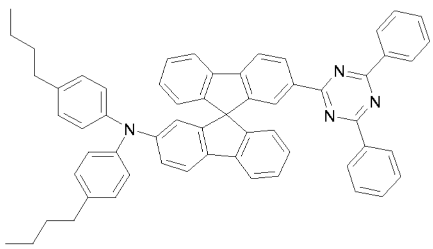

Formel (E-88)

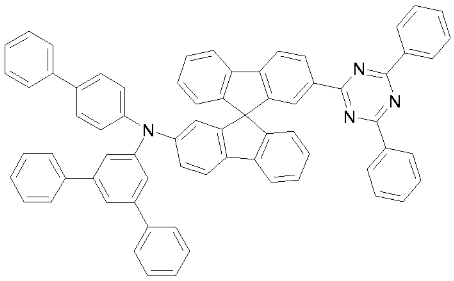

Formel (E-89)

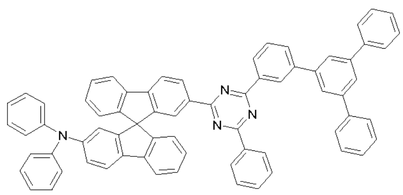

Formel (E-90)

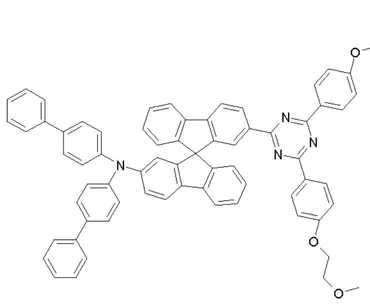

Formel (E-91)

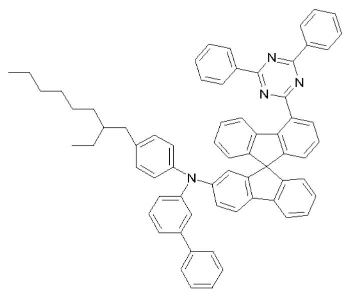

Formel (E-92)

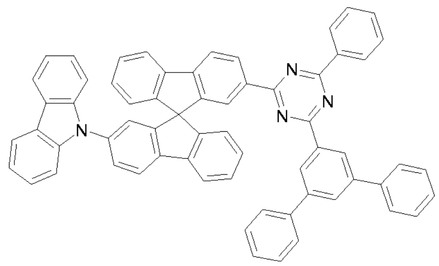

Formel (E-93)

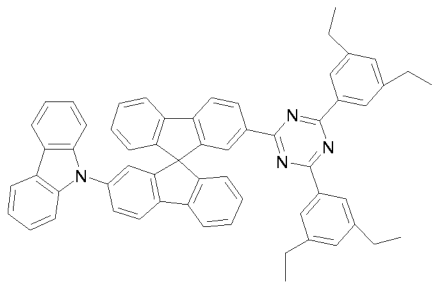

Formel (E-94)

Die erfindungsgemäßen Verbindungen können nach Verfahren hergestellt werden, die dem Fachmann gut bekannt sind. In den Beispielen werden verschiedene Verfahren offenbart.

Die erfindungsgemäßen organischen TADF-Verbindungen oder Zusammensetzungen enthaltend die erfindungsgemäßen organischen TADF-Verbindungen können in elektronischen Vorrichtungen, insbesondere in organischen Elektrolumineszenzvorrichtungen wie den organischen lichtemittierenden Dioden (OLEDs) und den organischen lichtemittierenden elektrochemischen Zellen (OLECs) eingesetzt werden.

Die vorliegende Erfindung betrifft daher auch eine Zusammensetzung enthaltend wenigstens eine erfindungsgemäße TADF-Verbindung und wenigstens ein weiteres organisches funktionelles Material und/oder ein anorganisches Nanoteilchen.

Das weitere organische funktionelle Material ist dabei bevorzugt ausgewählt aus der Gruppe der elektronleitenden Materialien (ETM), elektroninjizierenden Materialien (EIM), elektronenblockierenden Materialien (EBM), lochleitenden Materialien (HTM), lochinjizierenden Materialien (HIM), lochblockierenden Materialien (HBM), fluoreszierenden Emittern, phosphoreszierenden Emittern und Matrixmaterialien, wobei Matrixmaterialien besonders bevorzugt sind.

Dem Fachmann sind diese organischen funktionellen Materialien gut bekannt und er kann ohne Schwierigkeiten aus einer Vielzahl ihm bekannter Materialien und unter Heranziehung gängiger Überlegungen die jeweils geeigneten Materialien für die Zusammensetzung auswählen.

Fluoresziernde Emitter (auch fluoreszierende Dotanden genannt) im Sinne der vorliegenden Erfindung sind Verbindungen, bei denen die Lichtemission durch einen spin-erlaubten Übergang erfolgt, vorzugsweise handelt es sich hierbei um einen Übergang aus einem angeregten Singulett-Zustand.

Vom Begriff phosphoreszierende Emitter (auch phosphoreszierende Dotanden genannt) sind typischerweise Verbindungen umfasst, bei denen die Lichtemission durch einen spin-verbotenen Übergang erfolgt, beispielsweise einen Übergang aus einem Triplett-Zustand oder einem Zustand mit einer höheren Spinquantenzahl, beispielsweise einem Quintett-Zustand, vorzugsweise handelt es sich bei den phosphoreszierenden Emittern um Verbindungen, die Licht aus einem angeregten Triplett-Zustand emittieren.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Die TADF-Verbindung wird dabei bevorzugt als lumineszierende Verbindung in der Emissionsschicht einer Elektrolumineszenzvorrichtung verwendet.

Eine lumineszente Verbindung im Sinne der vorliegenden Erfindung ist eine Verbindung, die in der Lage ist,

unter optischer Anregung in einer Umgebung, wie sie in der organischen Elektrolumineszenzvorrichtung vorliegt, bei Raumtemperatur Licht zu emittieren. Dabei weist die Verbindung bevorzugt eine Lumineszenzquanteneffizienz von mindestens 40 % auf, besonders bevorzugt von mindestens 50 %, ganz besonders bevorzugt von mindestens 60 % und insbesondere bevorzugt von mindestens 70 %. Dabei wird die Lumineszenzquanteneffizienz bestimmt in einer Schicht in Mischung mit dem Matrixmaterial, wie sie in der organischen Elektrolumineszenzvorrichtung verwendet werden soll. Wie die Bestimmung der Lumineszenzquantenausbeute im Sinne der vorliegenden Erfindung durchgeführt wird, ist im Beispielteil ausführlich allgemein beschrieben.

Die organische TADF-Verbindung liegt vorzugsweise in der emittierenden Schicht einer organischen Elektrolumineszenzvorrichtung vor. Die erfindungsgemäßen TADF-Verbindungen können dabei sowohl als Emitter als auch als Matrix eingesetzt werden, wobei bevorzugt ist, wenn sie als Emitter verwendet werden. Wenn sie als Emitter in einer Emissionsschicht verwendet werden, liegen sie bevorzugt in einer Matrix vor. Die Matrix kann hierbei aus nur einem Material oder aus mehreren Materialien (mixed Matrix) bestehen. Dabei trägt das Matrixmaterial nicht oder nicht wesentlich zur Emission der Mischung bei.

Um Exziplexbildung in der emittierenden Schicht zu vermeiden, ist es bevorzugt, wenn für LUMO(TADF), also das LUMO der TADF-Verbindung, und das HOMO(Matrix) gilt:

$$\text{LUMO(TADF)} - \text{HOMO(Matrix)} > S_1\text{(TADF)} - 0.4\ \text{eV};$$

ganz bevorzugt:

$$\text{LUMO(TADF)} - \text{HOMO(Matrix)} > S_1\text{(TADF)} - 0.3\ \text{eV};$$

und besonders bevorzugt:

$$\text{LUMO(TADF)} - \text{HOMO(Matrix)} > S_1\text{(TADF)} - 0.2\ \text{eV}.$$

Dabei ist $S_1$(TADF) der erste angeregte Singulettzustand $S_1$ der TADF-Verbindung.

Damit die TADF-Verbindung die emittierende Verbindung in der Mischung der emittierenden Schicht ist, ist es bevorzugt, dass die niedrigste Triplettenergie der Matrix maximal 0.1 eV niedriger ist als die Triplettenergie des Moleküls, welches TADF zeigt. Insbesondere bevorzugt ist $T_1$ (Matrix) $\geq T_1$ (TADF). Besonders bevorzugt gilt: $T_1$ (Matrix) - $T_1$ (TADF) $\geq$ 0.1 eV, ganz besonders bevorzugt $T_1$ (Matrix) - $T_1$ (TADF) $\geq$ 0.2 eV. Dabei steht $T_1$ (Matrix) für die niedrigste Triplettenergie der Matrixverbindung und $T_1$ (TADF) für die niedrigste Triplettenergie der Verbindung, die TADF zeigt. Dabei wird die Triplettenergie der Matrix, nach dem im Beispielteil ausführlich allgemein offenbarten Verfahren, durch quantenchemische Rechnung bestimmt.

Beispiele für geeignete Matrixmaterialien sind Lactame, z.B. gemäß WO 2011/116865A1, WO 2013/064206A1, WO 2014/056567A1, WO 2014/094964A1,Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, oder verbrückte Carbazolderivate, z. B. gemäß US 2009/0136779, WO 2010/050778, WO 2011/042107 oder WO 2011/088877.

Die erfindungsgemäßen TADF-Verbindungen und die erfindungsgemäßen Zusammensetzungen eignen sich sehr gut, um elektronische Vorrichtungen, insbesondere organische Elektrolumineszenzvorrichtungen leicht und kostengünstig aus Lösung herzustellen. Zur Herstellung aus der Lösung werden Formulierungen benötigt. Die so hergestellten elektronischen Vorrichtungen weisen dabei sehr gute Leistungsdaten auf. Weiterhin zeigt es sich, dass die so hergestellten elektronischen Vorrichtungen weniger Ausfallraten bei der Herstellung der Vorrichtungen aufweisen. Die Formulierungen eignen sich somit für eine kostengünstige und zuverlässige Massenproduktion elektronischer Vorrichtungen für die kommerzielle Anwendung.

Daher betrifft die vorliegende Erfindung auch eine Formulierung enthaltend wenigstens eine erfindungsgemäße TADF-Verbindung oder wenigstens eine erfindungsgemäße Zusammensetzung sowie wenigstens ein Lösungsmittel.

Unter Formulierung wird vorliegend eine Zusammensetzung verstanden, die neben dem wenigstens einen organischen TADF-Molekül, den möglichen weiteren organischen funktionellen Materialien noch ein organisches Lösungsmittel oder organisches Lösungsmittelgemisch enthält. Das Lösungsmittel oder Lösungsmittelgemisch ist dabei bevorzugt im Überschuss vorhanden.

Unter Lösungsmittelgemisch wird vorliegend eine Mischung von wenigstens zwei unterschiedlichen Lösungsmitteln verstanden.

Weiterhin bevorzugt ist, dass die Formulierung in flüssiger Form vorliegt. Die Formulierung kann dabei eine echte Lösung, eine Emulsion, Miniemulsion, Dispersion oder Suspension darstellen, wobei ganz bevorzugt ist, wenn die Formulierung eine echte Lösung ist.

Die Formulierung wird zur Herstellung einer Schicht organischer elektronischer Vorrichtungen, insbesondere der Emissionsschicht organischer Elektrolumineszenzvorrichtungen, verwendet. Während der Herstellung der Schicht wird das Lösungsmittel oder das Lösungsmittelgemisch entfernt, so dass die TADF-Verbindung(en) und die potentiellen weiteren organischen funktionellen Materialien in der Schicht im Überschuss gegenüber dem verbleibenden Lösungsmittel oder Lösungsmittelgemisch vorliegen. Vorzugsweise findet sich das Lösungsmittel oder Lösungsmittelgemisch, wenn überhaupt, nur noch in Spuren in der Schicht der organischen elektronischen Vorrichtung. Ganz bevorzugt ist, wenn das Lösungsmittel oder Lösungsmittelgemisch vollständig entfernt wurde und daher nicht mehr in der aufgebrachten Schicht aufzufinden ist.

Bevorzugt ist, wenn das verwendetet Lösungsmittel oder die Lösungsmittel (im Fall eines Lösungsmittelgemischs) eine Oberflächenspannung von wenigstens 28 mN/m, bevorzugt wenigstens 30 mN/m, ganz bevorzugt wenigstens 32 mN/m und ganz besonders bevorzugt wenigstens 35 mN/m aufweist.

Weiterhin bevorzugt ist, wenn die Siede- oder Sublimationstemperatur des Lösungsmittels oder der Lösungsmittel kleiner als 300°C und bevorzugt weniger als 260°C beträgt.

Ganz bevorzugt ist, wenn die Viskosität des Lösungsmittel oder der verschiedenen Lösungsmittel eines Lösungsmittelgemischs größer als 3mPa*s und bevorzugt größer als 5mPa*s ist.

Weiterhin bevorzugt ist, wenn das Molekulargewicht des Lösungsmittels oder der Lösungsmittel kleiner oder gleich 1000 g/mol, bevorzugt kleiner oder gleich 700 g/mol, ganz bevorzugt kleiner oder gleich 500 g/mol und besonders bevorzugt kleiner oder gleich 300 g/mol ist.

Wie bereits erläutert liegt das Lösungsmittel bzw. sind die Lösungsmittel eines Lösungsmittelgemischs in der Formulierung im Überschuss vor im Vergleich zu der TADF-Verbindung.

Die Formulierung der vorliegenden Erfindung kann bevorzugt 0,01 bis 20 Gew.-%, ganz bevorzugt 0,05 bis 10 Gew.-%, besonders bevorzugt 0,1 bis 5 Gew.-% und insbesondere bevorzugt 0,5 bis 5 Gew.-% niedermolekulare organische Halbleitermaterialien enthalten, wobei unter niedermolekularen organischen Halbleitermaterialien die erfindungsgemäßen TADF-Verbindungen sowie die oben bereits genannten weiteren organischen funktionellen Materialien verstanden werden, deren Molekulargewicht kleiner oder gleich 4000 g/mol, bevorzugt kleiner oder gleich 3000 g/mol, ganz bevorzugt kleiner oder gleich 2000 g/mol, ganz besonders bevorzugt kleiner oder gleich 1500 und insbesondere bevorzugt kleiner oder gleich 1000 g/mol ist.

Der Fachmann kann aus einer Vielzahl von Lösungsmitteln und Lösungsmittelgemischen auswählen, um die erfindungsgemäßen Formulierungen bereitzustellen.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethyhenglycolbutylmethyl ether, Triethyleneglycolbutylmethyl ether, Diethylenglycoldibutyl ether, Triethylenglycoldimethyl ether, Diethylenglycolmonobutylether, Tripropyleneglycol dimethyl ether, Tetraethyleneglycol dimethyl ether, 2-isopropyl naphthalin, Pentylbenzol, Hexylbenzol, Heptylbenzol, octylbenzol, 1,1-Bis(3,4-Dimethylphenyl)ethan oder Mischungen diesen Lösemitteln.

Die Formulierung der vorliegenden Erfindung enthält ein oder mehrere organische Lösungsmittel, vorzugsweise mindestens ein aromatisches Lösungsmittel. Die Lösungsmittel sind vorzugsweise aus der Gruppe bestehend aus aromatischen Kohlenwasserstoffen, wie Toluol, o-, m- oder p-Xylol, Phenoxytoluole, Trimethylbenzole (z.B. 1,2,3-, 1,2,4- und 1,3,5-Trimethylbenzole), Tetralin, anderen Mono-, Di-, Tri- und Tetraalkylbenzolen (z.B. Diethylbenzole, Methylcumol, Tetramethylbenzole usw.), aromatischen Ethern (z.B. Anisol, Alkylanisole, z.B. 2-, 3- und 4-Isomere des Methylanisols, 2,3-, 2,4-, 2,5-, 2,6-, 3,4- und 3,5-Isomere des Dimethylanisols), Naphthalinderivaten, Alkylnaphthalinderivaten (z.B. 1- und 2- Methylnaphthalin), Di- und Tetrahydronaphthalinderivaten ausgewählt. Ebenfalls bevorzugt sind aromatische Ester (z.B. Alkylbenzoate), aromatische Ketone (z.B. Acetophenon, Propiophenon), Alkylketone (z.B. Cyclohexanon), heteroaromatische Lösungsmittel (z.B. Thiophen, Mono-, Di- und Trialkylthiophene, 2-Alkylthiazole, Benzthiazole usw., Pyridine), Halogenarylene und Anilinderivate. Diese Lösungsmittel können Halogenatome enthalten.

Besonders bevorzugt sind: 3-Fluor-trifluormethylbenzol, Trifluormethylbenzol, Dioxan, Trifluormethoxybenzol, 4-Fluor-benzoltrifluorid, 3-Fluorpyridin, Toluol, 2-Fluortoluol, 2-Fluor-benzoltrifluorid, 3-Fluortoluol, Pyridin, 4-Fluortoluol, 2,5-Difluortoluol, 1-Chlor-2,4-difluorbenzol, 2-Fluorpyridin, 3-Chlorfluorbenzol, 1-Chlor-2,5-difluorbenzol, 4-Chlorfluorbenzol, Chlorbenzol, 2-Chlorfluorbenzol, p-Xylol, m-Xylol, o-Xylol, 2,6-Lutidin, 2-Fluor-m-xylol, 3-Fluor-o-xylol, 2-Chlorbenzoltrifluorid, Dimethylformamid, 2-Chlor-6-fluortoluol, 2-Fluoranisol, Anisol, 2,3-Dimethylpyrazin, Brombenzol, 4-Fluoranisol, 3-Fluoranisol, 3-Trifluormethylanisol, 2-Methylanisol, Phenetol, Benzoldioxol, 4-Methylanisol, 3-Methylanisol, 4-Fluor-3-methylanisol, 1,2-Dichlorbenzol, 2-Fluorbenzolnitril, 4-Fluorveratrol, 2,6-Dimethylanisol, Anilin, 3-Fluorbenzolnitril, 2,5-Dimethylanisol, 3,4-Dimethylanisol, 2,4-Dimethylanisol, Benzolnitril, 3,5-Dimethylanisol, N,N-Dimethylanilin, 1-Fluor-3,5-Dimethoxybenzol, Phenylacetat, N-Methylanilin, Methylbenzoat, N-Methylpyrrolidon, Morpholin, 1,2-Dihydronaphthalin, 1,2,3,4-Tetrahydronaphthalin, o-Tolunitril, Veratrol, Ethylbenzoat, N,N-Diethylanilin, Propylbenzoat, 1-Methylnaphthalin, Butylbenzoat, 2-Methylbiphenyl, 2-Phenylpyridin oder 2,2'-Bitolyl.

Besonders bevorzugt sind aromatische Kohlenwasserstoffe, insbesondere Toluol, Phenoxytoluol, Dimethylbenzole (Xylole), Trimethylbenzole, Tetralin und Methylnaphthaline, aromatische Ether, insbesondere Anisol, und aromatische Ester, insbesondere Methylbenzoat.

Insbesondere bevorzugt sind aromatische Ether, insbesondere Anisol und Derivate davon, wie Alkylanisole, und aromatische Ester, insbesondere Methylbenzoat.

Diese Lösungsmittel können als Mischung von zwei, drei oder mehr verwendet werden.

Bevorzugte organische Lösungsmittel können Löslichkeitsparameter nach Hansen von $H_d$ im Bereich von 17,0 bis 23,2 $MPa^{0,5}$, $H_p$ im Bereich von 0,2 bis 12,5 $MPa^{0,5}$ und $H_h$ im Bereich von 0,9 bis 14,2 $MPa^{0,5}$ aufweisen. Besonders bevorzugte organische Lösungsmittel weisen Löslichkeitsparameter nach Hansen von $H_d$ im Bereich von 18,5 bis 21,0 $MPa^{0,5}$, $H_p$ im Bereich von 2,0 bis 6,0 $MPa^{0,5}$ und $H_h$ im Bereich von 2,0 bis 6,0 $MPa^{0,5}$ auf.

Tabelle 2: Löslichkeitsparameter nach Hansen von brauchbaren Lösungsmitteln

| **Lösungsmittel** | $H_d[MPa^{0,5}]$ | $H_h[MPa^{0,5}]$ | $H_p[MPa^{0,5}]$ |
|---|---|---|---|
| 1,2,3,4-Tetrahydro-1-naphthol | 19,6 | 9,2 | 12,8 |
| 1,2,3,4-Tetrahydronaphthalin | 19,1 | 2,3 | 4,0 |
| 1,2,3,4-Tetramethylbenzol | 18,7 | 1,8 | 1,6 |
| 1,2,3,5-Tetramethylbenzol | 18,7 | 1,8 | 1,6 |
| 1,2,3-Trimethylbenzol | 19,0 | 2,9 | 1,6 |
| 1,2,4,5-Tetramethylbenzol | 18,7 | 1,8 | 1,6 |
| 1,2,4-Trichlorbenzol | 20,5 | 6,9 | 2,7 |
| 1,2,4-Trimethylbenzol | 19,0 | 2,9 | 1,6 |
| 1,2-Dihydronaphthalin | 20,1 | 5,5 | 4,9 |
| 1,2-Dimethylnaphthalin | 21,0 | 1,7 | 5,2 |
| 1,3,3-Trimethyl-2-methylenindol | 17,9 | 1,0 | 3,0 |
| 1,3-Benzodioxol | 19,7 | 7,4 | 7,9 |
| 1,3-Diisopropylbenzol | 17,5 | 0,2 | 1,1 |
| 1,3-Dimethylnaphthalin | 21,0 | 1,7 | 5,2 |
| 1,4-Benzodioxan | 19,5 | 8,7 | 7,2 |
| 1,4-Diisopropylbenzol | 17,5 | 0,6 | 1,6 |
| 1,4-Dimethylnaphthalin | 21,0 | 1,7 | 5,2 |
| 1,5-Dimethyltetralin | 19,3 | 5,5 | 2,6 |
| 1-Benzothiophen | 19,7 | 12,3 | 6,3 |
| 1-Bromnaphthalin | 23,1 | 10,3 | 6,1 |
| 1-Chlormethylnaphthalin | 22,1 | 9,9 | 5,3 |
| 1-Ethylnaphthalin | 20,7 | 7,8 | 4,4 |
| 1-Methoxynaphthalin | 21,4 | 10,5 | 7,5 |

(fortgesetzt)

| **Lösungsmittel** | $H_d$[$MPa^{0,5}$] | $H_h$[$MPa^{0,5}$] | $H_p$[$MPa^{0,5}$] |
|---|---|---|---|
| 1-Methylnaphthalin | 21,7 | 8,4 | 4,5 |
| 1-Methylindan | 19,4 | 5,7 | 2,5 |
| 1-Methylindol | 19,2 | 8,1 | 10,2 |
| 2,3,3-Trimethoxyindolenin | 19,6 | 6,8 | 4,2 |
| 2,3-Benzofuran | 21,3 | 5,5 | 5,6 |
| 2,3-Dihydrobenzofuran | 19,9 | 9,5 | 6,6 |
| 2,3-Dimethylanisol | 18,9 | 4,6 | 4,5 |
| 2,4-Dimethylanisol | 18,9 | 4,6 | 4,5 |
| 2,5-Dimethylanisol | 18,9 | 4,6 | 4,5 |
| 2,6-Diisopropylnaphthalin | 18,3 | 3,5 | 2,2 |
| 2,6-Dimethylanisol | 18,9 | 4,6 | 4,5 |
| 2,6-Dimethylnaphthalin | 20,1 | 5,0 | 3,0 |
| 2-Brom-3-(brommethyl)thiophen | 19,3 | 7,3 | 6,6 |
| 2-Brommethylnaphthalin | 22,0 | 9,4 | 7,2 |
| 2-Bromnaphthalin | 23,1 | 10,3 | 6,1 |
| 2-Ethoxynaphthalin | 20,5 | 10,0 | 7,0 |
| 2-Ethylnaphthalin | 20,7 | 7,8 | 4,4 |
| 2-Isopropylanisol | 17,7 | 4,3 | 5,4 |
| 2-Methylchinolin | 20,0 | 7,8 | 4,0 |
| 2-Methylanisol | 18,3 | 5,1 | 6,2 |
| 2-Methylindol | 17,8 | 9,7 | 4,8 |
| 2-Phenoxyethanol | 18,7 | 8,5 | 13,0 |
| 3,4-Dimethylanisol | 18,9 | 4,6 | 4,5 |
| 3,5-Dimethylanisol | 18,9 | 4,6 | 4,5 |
| 3-Bromchinolin | 21,4 | 8,7 | 5,1 |
| 3-Isopropylbiphenyl | 19,1 | 1,3 | 1,9 |
| 3-Methylanisol | 18,7 | 5,7 | 5,4 |
| 4-Benzylaceton | 18,3 | 8,8 | 5,0 |
| 4-Isopropylbiphenyl | 19,0 | 2,5 | 1,9 |
| 4-Methoxybenzylalkohol | 19,0 | 8,5 | 13,3 |
| 4-Methylanisol | 18,6 | 5,9 | 7,2 |
| 4-Phenyl-2-butanon | 18,3 | 8,8 | 5,0 |
| 5,6,7,8-Tetrahydro-1-naphthol | 19,6 | 7,2 | 10,9 |
| 5,6,7,8-Tetrahydro-2-naphthol | 19,6 | 7,2 | 10,9 |
| 5,6,7,8-Tetrahydro-2-naphthylamin | 20,1 | 7,9 | 8,6 |
| 5,6.7,8-Tetrahydro-1-naphthylamin | 20,1 | 7,9 | 8,6 |
| 5-Decanolid | 17,1 | 7,8 | 3,8 |
| 5-Methoxyindan | 19,8 | 9,8 | 4,0 |

(fortgesetzt)

| **Lösungsmittel** | $H_d$[$MPa^{0,5}$] | $H_h$[$MPa^{0,5}$] | $H_p$[$MPa^{0,5}$] |
|---|---|---|---|
| 5-Methoxyindol | 17,4 | 12,3 | 7,8 |
| 5-tert.-Butyl-m-xylol | 17,6 | 3,4 | 2,2 |
| 6-Methoxy-1,2,3,4-tetrahydronapthalin | 19,4 | 6,8 | 5,4 |
| 6-Methylchinolin | 21,7 | 8,4 | 4,5 |
| 8-Methylchinolin | 21,7 | 8,4 | 4,5 |
| Acetophenon | 18,8 | 10,8 | 5,5 |
| Anisol | 18,5 | 5,5 | 5,2 |
| $\alpha$-Pinen | 17,4 | 3,0 | 3,2 |
| Benzonitril | 19,2 | 11,9 | 4,7 |
| Benzothiazol | 21,3 | 5,5 | 5,6 |
| Benzylacetat | 18,2 | 7,3 | 6,4 |
| Benzylalkohol | 19,1 | 6,7 | 14,2 |
| Brombenzol | 19,8 | 7,6 | 4,3 |
| Butylbenzol | 17,6 | 2,6 | 1,7 |
| Butylbenzoat | 17,7 | 5,9 | 5,2 |
| Cyclohexylbenzol | 18,6 | 1,0 | 1,6 |
| Decahydronaphthalin | 17,5 | 0,4 | 1,0 |
| Diphenylether | 19,9 | 2,9 | 3,3 |
| Ethylphenylketon (Propiophenon) | 18,3 | 8,9 | 5,3 |
| Ethylbenzol | 18,2 | 2,7 | 2,1 |
| Ethylbenzoat | 18,1 | 6,6 | 5,9 |
| Furfurylalkohol | 18,1 | 6,7 | 11,9 |
| gamma-Terpinen | 18,0 | 2,5 | 2,8 |
| Hexylbenzol | 17,4 | 2,9 | 1,6 |
| Indan | 19,7 | 7,3 | 5,8 |
| Inden | 20,3 | 4,4 | 5,4 |
| Isoamylbenzol | 17,1 | 3,7 | 1,8 |
| Isobutyl benzol | 17,1 | 2,9 | 1,6 |
| Isopropylbenzol (Cumol) | 17,8 | 2,0 | 1,1 |
| m-Cymol | 18,1 | 2,0 | 2,1 |
| Mesitylen | 19,0 | 2,9 | 1,6 |
| Methylbenzoat | 18,5 | 7,9 | 6,4 |
| Methylphenylacetat | 18,2 | 7,3 | 6,4 |
| m-Xylol | 18,8 | 3,1 | 2,7 |
| n-Butoxybenzol | 17,5 | 4,4 | 4,1 |
| n-Butylbenzol | 17,6 | 2,6 | 1,7 |
| n-Propylbenzoat | 17,8 | 6,6 | 6,3 |
| n-Propylbenzol | 17,8 | 3,4 | 2,8 |

(fortgesetzt)

| **Lösungsmittel** | $H_d$[$MPa^{0,5}$] | $H_h$[$MPa^{0,5}$] | $H_p$[$MPa^{0,5}$] |
|---|---|---|---|
| o-Dichlorbenzol | 19,5 | 8,7 | 3,3 |
| o-Diethylbenzol | 17,7 | 0,7 | 1,9 |
| o-Ethyltoluol | 18,0 | 1,9 | 2,8 |
| o-Xylol | 18,4 | 2,0 | 2,9 |
| Pentylbenzol | 17,4 | 3,0 | 1,8 |
| p-Ethyltoluol | 18,3 | 3,5 | 2,8 |
| Phenetol | 18,1 | 4,6 | 4,6 |
| Phenylacetat | 18,5 | 7,9 | 6,4 |
| p-Isopropyltoluol (p-Cymol) | 18,0 | 2,5 | 2,8 |
| Propiophenon | 18,3 | 8,9 | 5,3 |
| Propylbenzoat | 17,8 | 6,6 | 6,3 |
| p-Xylol | 18,7 | 3,3 | 3,3 |
| sec.-Butylbenzol | 17,2 | 2,2 | 1,6 |
| t-Butylbenzol | 17,2 | 1,3 | 2,9 |
| Tetralin | 19,1 | 2,3 | 4,0 |
| Thiophen | 18,8 | 5,2 | 7,4 |
| Toluol | 18,6 | 4,0 | 2,2 |
| Veratrol | 18,2 | 6,3 | 7,5 |
| $H_d$ bezieht sich auf den Dispersionsbeitrag<br>$H_p$ bezieht sich auf den polaren Beitrag<br>$H_h$ bezieht sich auf den Wasserstoffbindungsbeitrag | | | |

Vorzugsweise besitzt das Lösungsmittel bei dem verwendeten Druck, ganz bevorzugt bei Atmosphärendruck (1013 hPa), einen Siedepunkt oder eine Sublimationstemperatur von < 300°C, besonders bevorzugt ≤ 260°C, insbesondere bevorzugt ≤ 220°C. Die Verdampfung kann auch beschleunigt werden, z.B. durch Einsatz von Wärme und/oder vermindertem Druck. Durch die Verwendung von Lösungsmitteln mit einem Siedepunkt von mindestens 100°C, vorzugsweise mindestens 130°C lassen sich unerwartete Verbesserungen erreichen.

Üblicherweise kann das organische Lösungsmittel eine Oberflächenspannung von mindestens 28 mN/m, vorzugsweise mindestens 30 mN/m, besonders bevorzugt mindestens 32 mN/m und insbesondere bevorzugt mindestens 35 mN/m aufweisen. Die Oberflächenspannung kann mit einem Kontaktwinkel-Goniometer FTA (First Ten Angstrom) 125 bei 25°C gemessen werden. Einzelheiten zum Verfahren sind bei First Ten Angstrom, wie von Roger P. Woodward, Ph.D., "Surface Tension Measurements Using the Drop Shape Method" veröffentlicht, erhältlich. Vorzugsweise kann die Pendant-Drop-Methode zur Bestimmung der Oberflächenspannung verwendet werden.

Für eine grobe Schätzung kann die Oberflächenspannung unter Verwendung der Löslichkeitsparameter nach Hansen nach der in Hansen Solubility Parameters: A User's Handbook, zweite Auflage, C. M. Hansen (2007), Taylor and Francis Group, LLC (HSPiP-Handbuch) dargelegten Formel berechnet werden.

$$\text{Oberflächenspannung} = 0{,}0146 \text{ x}(2{,}28\text{x } \delta H_d^2 + \delta H_p^2 + \delta H_h^2) \text{ x MVol}^{0{,}2},$$

wobei:

$H_d$ sich auf den Dispersionsbeitrag bezieht,
$H_p$ sich auf den polaren Beitrag bezieht,
$H_h$ sich auf den Wasserstoffbindungsbeitrag bezieht,
MVol sich auf das molare Volumen bezieht.

Die Löslichkeitsparameter nach Hansen können nach dem Programm Hansen Solubility Parameters in Practice (HSPiP) (2. Auflage) wie bei Hanson und Abbot et al. erhältlich bestimmt werden.

Vorzugsweise kann das Lösungsmittel eine relative Verdampfungsgeschwindigkeit (Butylacetat = 100) von mindestens 0,01, vorzugsweise von mindestens 0,1, bevorzugt von mindestens 0,5, besonders bevorzugt von mindestens 5, ganz besonders bevorzugt von mindestens 10 und insbesondere bevorzugt von mindestens 20 aufweisen. Die relative Verdampfungsgeschwindigkeit kann nach DIN 53170: 2009-08 bestimmt werden. Für eine grobe Schätzung kann die relative Verdampfungsgeschwindigkeit unter Verwendung der Löslichkeitsparameter nach Hansen mit dem HSPiP-Programm wie vor- und nachstehend genannt berechnet werden.

Die Formulierung der vorliegenden Erfindung enthält vorzugsweise mindestens 70 Gew.-%, besonders bevorzugt mindestens 80 Gew.-% und insbesondere bevorzugt mindestens 90 Gew.-% eines oder mehrerer organischer Lösungsmittel bezogen auf die Gesamtmasse der Formulierung.

Bei einer weiteren bevorzugten Ausführungsform enthält die Formulierung der vorliegenden Erfindung mindestens ein Polymermaterial als inertes Bindemittel. Das bedeutet, dass das Polymer keine Halbleitereigenschaften besitzt oder mit einer der Halbleiterverbindungen in der Zusammensetzung chemisch reagiert. Die geringen Leitungseigenschaften des inerten polymeren Bindemittels können als niedrige Dielektrizitätskonstante bestimmt werden. Bevorzugte Bindemittel gemäß der vorliegenden Erfindung sind Materialien mit niedriger Dielektrizitätskonstante, also solche, die bei 1.000 Hz eine Dielektrizitätskonstante ($\varepsilon$) von 3,3 oder weniger besitzen. Das organische Bindemittel besitzt bei 1.000 Hz vorzugsweise eine Dielektrizitätskonstante von weniger als 3,0, besonders bevorzugt 2,9 oder weniger. Vorzugsweise besitzt das organische Bindemittel bei 1.000 Hz eine Dielektrizitätskonstante von größer 1,7. Mit besonderem Vorzug liegt die Dielektrizitätskonstante des Bindemittels im Bereich von 2,0 bis 2,9. Der Ausdruck "chemisch reagieren" wie vor- und nachstehend verwendet bezeichnet eine mögliche Oxidation oder andere chemische Reaktion des nicht leitenden Zusatzes mit den organischen lichtemittierenden Materialien und/oder Ladungstransportmaterialien unter den für die Herstellung, die Lagerung, den Transport und/oder die Verwendung der Formulierung und der OLED-Vorrichtung verwendeten Bedingungen.

Vorzugsweise weist das polymere Bindemittel ein gewichtsmittleres Molekulargewicht im Bereich von 1.000 bis 50.000.000 g/mol, besonders bevorzugt 1.500 bis 10.000.000 g/mol und insbesondere bevorzugt 2.000 bis 5.000.000 g/mol auf. Mit Polymeren mit einem gewichtsmittleren Molekulargewicht von vorzugsweise $\geq$ 10.000 g/mol, besonders bevorzugt $\geq$ 100.000 g/mol lassen sich überraschende Effekte erzielen.

Das Polymer kann insbesondere einen Polydispersitätsindex $M_w/M_n$ im Bereich von 1,0 bis 10,0, besonders bevorzugt im Bereich von 1,1 bis 5,0 und insbesondere bevorzugt im Bereich von 1,2 bis 3 besitzen.

Üblicherweise ist das polymere Bindemittel im Lösungsmittel der vorliegenden Zusammensetzung wie vor- und nachstehend beschrieben dispergierbar oder löslich. Vorzugsweise ist das polymere Bindemittel in dem organischen Lösungsmittel löslich und beträgt die Löslichkeit des polymeren Bindemittels in dem Lösungsmittel mindestens 1 g/l, besonders bevorzugt mindestens 5 g/l und insbesondere bevorzugt mindestens 10 g/l.

Gemäß einer besonderen Ausführungsform der vorliegenden Erfindung kann die Zusammensetzung vorzugsweise 0,1 bis 10 Gew.-%, besonders bevorzugt 0,25 bis 5 Gew.-% und insbesondere bevorzugt 0,5 bis 4 Gew.-% an polymerem Bindemittel enthalten.

Gemäß einer besonderen Ausführungsform enthalten die polymeren Bindemittel vorzugsweise von Styrol und/oder Olefinen abgeleitete Wiederholungseinheiten. Bevorzugte polymere Bindemittel können mindestens 80 Gew.-%, vorzugsweise 90 Gew.-% und besonders bevorzugt 99 Gew.-% von Styrolmonomeren und/oder Olefinen abgeleitete Wiederholungseinheiten enthalten.

Styrolmonomere sind in der Technik gut bekannt. Diese Monomere umfassen Styrol, substituierte Styrole mit einem Alkylsubstituenten in der Seitenkette, wie $\alpha$-Methylstyrol und $\alpha$-Ethylstyrol, substituierte Styrole mit einem Alkylsubstituenten am Ring, wie Vinyltoluol und p-Methylstyrol, halogenierte Styrole wie Monochlorstyrole, Dichlorstyrole, Tribromstyrole und Tetrabromstyrole.

Olefine sind Monomere, die aus Wasserstoff- und Kohlenstoffatomen bestehen. Diese Monomere umfassen Ethylen, Propylen, Butylen, Isopren und 1,3-Butadien.

Gemäß einem besonderen Aspekt der vorliegenden Erfindung handelt es sich bei dem polymeren Bindemittel um Polystyrol mit einem gewichtsmittleren Molekulargewicht im Bereich von 50.000 bis 2.000.000 g/mol, vorzugsweise 100.000 bis 750.000 g/mol, besonders bevorzugt im Bereich von 150.000 bis 600.000 g/mol und insbesondere bevorzugt im Bereich von 200.000 bis 500.000 g/mol.

Gemäß einer weiteren Ausführungsform der vorliegenden Erfindung handelt es sich bei dem polymeren Bindemittel um Poly-4-methylstyrol mit einem gewichtsmittleren Molekulargewicht im Bereich von 40.000 bis 120.000 g/mol, besonders bevorzugt im Bereich von 60.000 bis 100.000 g/mol.

Insbesondere kann es sich bei dem Bindemittel um Poly-$\alpha$-methylstyrol mit einem gewichtsmittleren Molekulargewicht im Bereich von 1.000 bis 20.000 g/mol, besonders bevorzugt im Bereich von 1.500 bis 6.000 g/mol handeln.

Brauchbare und bevorzugte polymere Bindemittel weisen Löslichkeitsparameter nach Hansen von $H_d$ im Bereich von 15,7 bis 23,0 $MPa^{0,5}$, $H_p$ im Bereich von 0,0 bis 20,0 $MPa^{0,5}$ und $H_h$ im Bereich von 0,0 bis 12,5 $MPa^{0,5}$ auf.

Besonders bevorzugte polymere Bindemittel weisen Löslichkeitsparameter nach Hansen von $H_d$ im Bereich von 17,0 bis 21,0 $MPa^{0,5}$, $H_p$ im Bereich von 1,0 bis 5,0 $MPa^{0,5}$ und $H_h$ im Bereich von 2,0 bis 10,0 $MPa^{0,5}$ auf. Insbesondere bevorzugte polymere Bindemittel weisen Löslichkeitsparameter nach Hansen von $H_d$ im Bereich von 19,0 bis 21,0 $MPa^{0,5}$, $H_p$ im Bereich von 1,0 bis 3,0 $MPa^{0,5}$ und $H_h$ im Bereich von 2,5 bis 5,0 $MPa^{0,5}$ auf.

Die Löslichkeitsparameter nach Hansen können nach dem Programm Hansen Solubility Parameters in Practice (HSPiP) (2. Auflage) wie bei Hanson und Abbot et al. erhältlich bestimmt werden.

Beispiele brauchbarer polymerer Bindemittel werden in Tabelle 1 der WO 2011/076325 A1 offenbart.

Gemäß einer bevorzugten Ausführungsform der vorliegenden Erfindung handelt es sich bei dem inerten Bindemittel um ein Polymer mit einer Glasübergangstemperatur im Bereich von -70 bis 160°C, vorzugsweise 0 bis 150°C, besonders bevorzugt 50 bis 140°C und insbesondere bevorzugt 70 bis 130°C. Die Glasübergangstemperatur lässt sich durch Messen der DSC des Polymers (DIN EN ISO 11357, Erhitzungsgeschwindigkeit 10°C pro Minute) bestimmen.

Die Formulierung gemäß der vorliegenden Erfindung kann zusätzlich eine oder mehrere weitere Komponenten enthalten, wie beispielsweise oberflächenaktive Verbindungen, Gleitmittel, Netzmittel, Dispergiermittel, Hydrophobiermittel, Haftmittel, Fließverbesserer, Entschäumer, Entgasungsmittel, Verdünnungsmittel, die reaktiv oder nicht reaktiv sein können, Hilfsstoffe, Farbmittel, Farbstoffe oder Pigmente, Sensibilisierer, Stabilisatoren, Nanopartikel oder Inhibitoren. Diese weiteren Komponenten sollten jedoch nicht oxidierend oder anders in der Lage sein, chemisch mit dem organischen Halbleitermaterial zu reagieren oder einen elektrisch dotierenden Effekt auf das organische Halbleitermaterial auszuüben.

Überraschende Verbesserungen lassen sich mit flüchtigen Netzmitteln erzielen. Der Ausdruck "flüchtig", wie er vor- und nachstehend verwendet wird, bedeutet, dass das Mittel aus dem/den organischen Halbleitermaterial(ien) durch Verdampfen entfernt werden kann, nachdem diese(s) Material(ien) auf ein Substrat einer OLED-Vorrichtung unter Bedingungen (wie Temperatur und/oder verminderter Druck), die diese Materialien oder die OLED-Vorrichtung nicht wesentlich beschädigen, abgeschieden wurden. Das bedeutet vorzugsweise, dass das Netzmittel bei dem verwendeten Druck, sehr bevorzugt bei Atmosphärendruck (1013 hPa) vorzugsweise einen Siedepunkt oder eine Sublimationstemperatur von < 350°C, besonders bevorzugt ≤ 300°C, insbesondere bevorzugt ≤ 250°C besitzt. Die Verdampfung kann auch beschleunigt werden, z.B. durch Einsatz von Wärme und/oder vermindertem Druck.

Überraschende Wirkungen lassen sich mit Formulierungen erreichen, die flüchtige Komponenten mit ähnlichen Siedepunkten enthalten. Vorzugsweise liegt der Unterschied des Siedepunktes des Netzmittels und des organischen Lösungsmittels im Bereich von -50°C bis 50°C, besonders bevorzugt im Bereich von -30°C bis 30°C und insbesondere bevorzugt im Bereich von -20°C bis 20°C.

Bevorzugte Netzmittel sind nichtaromatische Verbindungen. Weiteren Vorzug haben nichtionische Verbindungen als Netzmittel. Besonders brauchbare Netzmittel weisen eine Oberflächenspannung von höchstens 35 mN/m, vorzugsweise von höchstens 30 mN/m und besonders bevorzugt von höchstens 25 mN/m auf. Die Oberflächenspannung kann mit einem Kontaktwinkel-Goniometer FTA (First Ten Angstrom) 125 bei 25°C gemessen werden. Einzelheiten zum Verfahren sind bei First Ten Angstrom, wie von Roger P. Woodward, Ph.D., "Surface Tension Measurements Using the Drop Shape Method" veröffentlicht, erhältlich. Vorzugsweise kann die Pendant-Drop-Methode zur Bestimmung der Oberflächenspannung verwendet werden.

Gemäß einem besonderen Aspekt der vorliegenden Erfindung beträgt der Unterschied der Oberflächenspannung des organischen Lösungsmittels und des Netzmittels bevorzugt mindestens 1 mN/m, vorzugsweise mindestens 5 mN/m und besonders bevorzugt mindestens 10 mN/m.

Gemäß einem besonderen Aspekt der vorliegenden Erfindung kann das Netzmittel eine relative Verdampfungsgeschwindigkeit (Butylacetat = 100) von mindestens 0,01, bevorzugt von mindestens 0,1, vorzugsweise von mindestens 0,5, besonders bevorzugt von mindestens 5, ganz besonders bevorzugt von mindestens 10 und insbesondere bevorzugt von mindestens 20 aufweisen.

Unerwartete Verbesserungen lassen sich mit Zusammensetzungen erzielen, die Lösungsmittel und Netzmittel mit einer ähnlichen relativen Verdampfungsgeschwindigkeit (Butylacetat = 100) enthalten.

Vorzugsweise liegt der Unterschied der relativen Verdampfungsgeschwindigkeit (Butylacetat = 100) des Netzmittels und des organischen Lösungsmittels im Bereich von -20 bis 20, besonders bevorzugt im Bereich von -10 bis 10. Gemäß einer bevorzugten Ausführungsform der vorliegenden Erfindung kann das Verhältnis der relativen Verdampfungsgeschwindigkeit (Butylacetat = 100) des Netzmittels zur relativen Verdampfungsgeschwindigkeit (Butylacetat = 100) des organischen Lösungsmittels im Bereich von 230:1 bis 1:230, vorzugsweise von 20:1 bis 1:20 und besonders bevorzugt von 5:1 bis 1:5 liegen.

Unerwartete Verbesserungen lassen sich durch Netzmittel erzielen, die ein Molekulargewicht von mindestens 100 g/mol, vorzugsweise mindestens 150 g/mol, besonders bevorzugt mindestens 180 g/mol und insbesondere bevorzugt mindestens 200 g/mol aufweisen.

Geeignete und bevorzugte Netzmittel, die nicht oxidieren oder auf andere Weise chemisch mit den OSC-Materialien reagieren, sind aus der Gruppe bestehend aus Siloxanen, Alkanen, Aminen, Alkenen, Alkinen, Alkoholen und/oder halogenierten Derivaten dieser Verbindungen ausgewählt. Des weiteren können Fluorether, Fluorester

und/oder Fluorketone verwendet werden. Besonders bevorzugt sind diese Verbindungen aus Methylsiloxanen mit 6 bis 20 Kohlenstoffatomen, insbesondere 8 bis 16 Kohlenstoffatomen; C7-C14 Alkanen, C7-C14 Alkenen, C7-C14 Alkinen, Alkoholen mit 7 bis 14 Kohlenstoffatomen, Fluorethern mit 7 bis 14 Kohlenstoffatomen, Fluorestern mit 7 bis 14 Kohlenstoffatomen und Fluorketonen mit 7 bis 14 Kohlenstoffatomen ausgewählt. Insbesondere bevorzugte Netzmittel sind Methylsiloxane mit 8 bis 14 Kohlenstoffatomen.

Beispiele von Verbindungen, die als Netzmittel brauchbar und bevorzugt sind, sind in der in WO 2011/076325 A1 offenbart.

Bevorzugt enthält die Formulierung vorzugsweise höchstens 5 Gew.-%, besonders bevorzugt höchstens 3 Gew.-% und insbesondere bevorzugt höchstens 1 Gew.-% an benetzenden Hilfsstoffen. Bevorzugt enthält die Zusammensetzung 0,01 bis 5 Gew.-%, besonders bevorzugt 0,05 bis 3 Gew.-% und insbesondere bevorzugt 0,1 bis 1 Gew.-% Netzmittel.

Die Formulierungen können mit Hilfe von Verfahren hergestellt werden die dem Fachmann gut geläufig sind. Typischerweise werden die einzelnen Komponenten der Formulierung gemischt und gerührt, ggf. auch unter Zufuhr von Wärme. Häufig wird die Formulierung auch entgast oder mit Inertgasen übersättigten Lösungsmitteln hergestellt. Insgesamt ist darauf zu achten, dass nur Lösungsmittel und andere Komponenten sehr hoher Reinheit verwendet werden, um eine Kontamination der elektronischen Vorrichtungen mit schädlichen Verbindungen zu vermeiden. Insbesondere ist darauf zu achten, dass der Wasser-, Sauerstoff und Halogengehalt in der Formulierung niedrig gehalten wird, da insbesondere die Leistungsdaten organischer Elektrolumineszenzvorrichtungen durch deren Anwesenheit stark beeinträchtigt werden können.

Die erfindungsgemäße organische TADF-Verbindung kann, wie bereits ausgeführt, als Emitter in elektronischen Vorrichtungen eingesetzt werden. In einer weiteren Ausführungsform der vorliegenden Erfindung kann die TADF-Verbindung auch als Matrixmaterial verwendet werden. In diesem Fall überträgt die TADF-Verbindung seine Energie auf einen Emitter, der letztlich Strahlung aus der elektronischen Vorrichtung emittiert. Hierfür eignet sich grundsätzlich jeder Emitter, also sowohl organische fluoreszierende oder organische phosphoreszierende und auch anorganische Emitter, wie beispielsweise, die Quantenpunkte (quantum dots) und Quantenstäbchen (quantum rods), die gemeinsam auch als Nanoteilchen bezeichnet werden. Die Verwendung von TADF-Verbindungen als Matrix ermöglicht elektronische Vorrichtungen mit besonders vorteihaften Leistungsdaten. Besonders bevorzugt ist, wenn die Emitter ein Nanoteilchen ist.

Es ist daher sehr vorteilhaft, wenn die Formulierung nanokristalline Verbindungen oder Nanoteilchen enthält. Zu den Nanokristallen und Nanoteilchen zählen die Quantenpunkte (quantum dots, QDs) und Quantenstäbchen (quantum rods, QRs). Mit Hilfe dieser Verbindungen lassen sich effiziente OLEDs und andere organische elektronische Vorrichtungen kostengünstig herstellen, die eine sehr geringe Ausfallrate aufweisen und eine Emission mit schmalen Banden zeigen. Weiterhin lassen sich emittierende Vorrichtungen erhalten, die ausgezeichnete Farbreinheiten und Farbqualitäten (CRI - color rendering index) zeigen. Weiterhin kann die Emissionsfarbe (CIE 1931 RGB) solcher OLEDs sehr leicht und genau eingestellt werden.

Ganz besonders bevorzugt ist, wenn die Formulierung neben Nanoteilchen und TADF-Verbindung(en) noch weitere Matrixverbindungen enthält, wobei es sich bevorzugt um die oben genannten elektronentransportierenden Matrixverbindungen oder um die oben genannten lochtransportierneden Matrixverbindungen handelt.

Sowohl Quantenpunkte als auch Quantenstäbchen können sehr einfach hergestellt werden. Die Größe der Teilchen, die bestimmender Faktor für die Farbe der emittierten Strahlung darstellt, ist leicht einzustellen. Weiterhin sind die Quantenpunkte und Quantenstäbchen löslich in vielen gängigen Lösungsmitteln und eignen sich sehr gut für lösungsbasierte Herstellprozesse.

Die ersten monodispersen kolloidalen Quantenpunkte enthaltend ein halbleitendes Material basierten auf CdE (E = S, Se, Te) und wurden mit Hilfe der sogenannten TOPO (Trioctylphosphinoxid) Methode hergestellt (J. Am. Chem. Soc. 115[19], 8706-8715, 1993).

Dem Fachmann ist eine Vielzahl von Methoden zur Herstellung von Quantenpunkten und Quantenstäbchen bekannt, wobei bevorzugt lösungsbasierte Methoden kolloidaler Systeme zum kontrollierten Wachstum anorganischer Quantenpunkte eingesetzt werden (Science 271:933 (1996); J. Am. Chem. Soc. 30:7019-7029 (1997); J. Am. Chem. Soc. 115:8706 (1993)).

Geeignete Halbleiter, die in Quantenpunkten und Quantenstäbchen verwendet werden sind ausgewählt aus der Gruppe bestehend aus

Elementen der Gruppen II bis VI, so wie Z.B. CdSe, CdS, CdTe, ZnSe, ZnO, ZnS, ZnTe, HgS, HgSe, HgTe und Mischungen davon, wie z.B. CdZnSe;

Elementen der Gruppe III bis V, wie z.B. InAs, InP, GaAs, GaP, InN, GaN, InSb, GaSb, AlP, AlAs, AlSb und Mischungen davon, wie z.B. InAsP, CdSeTe, ZnCdSe, InGaAs;

Elementen der Gruppe IV bis VI, wie z.B. PbSe, PbTe and PbS und Mischungen davon;

Elementen der Gruppe III bis VI, wie z.B. InSe, InTe, InS, GaSe und Mischungen davon, wie z.B. InGaSe, InSeS;

Elementen der Gruppe IV Halbleiter, wie z.B. Si and Ge und Mischungen davon, sowie Mischungen der zuvor genannten Materialien.

Weiter geeignete Halbleiter für Quantenpunkte und Quantenstäbchen umfassen solche, die in US10/796,832 offenbart sind und umfassen jede Art von Halbleitern umfassend Elemente der Gruppen II bis VI, III bis V, IV bis VI und IV Halbleiter. Eine Auswahl besonders geeigneter Halbleiter ist Si, Ge, Sn, Se, Te, B, C (auch Diamand), P, BN, BP, BAs, AlN, AlP, AlAs, AlS, AlSb, BaS, BaSe, BaTe, CaS, CaSe, CaTe, GaN, GaP, GaAs, GaSb, InN, InP, InAs, InSb, AlN, AlP, AlAs, AlSb, GaN, GaP, GaAs, GaSb, ZnO, ZnS, ZnSe, ZnTe, CdS, CdSe, CdTe, HgS, HgSe, HgTe, BeS, BeSe, BeTe, MgS, MgSe, GeS, GeSe, GeTe, SnS, SnSe, SnTe, PbO, PbS, PbSe, PbTe, CuF, CuCl, CuBr, CuI, $Si_3N_4$, $Ge_3N_4$, $Al_2O_3$, $(Al, Ga, In)_2$ $(S, Se, Te)_3$, $Al_2CO$, und eine geeignete Kombination von zwei oder mehr der genannten Halbleiter..

Es ist bevorzug, wenn die Quantenpunkte oder Quantenstäbchen ausgewählt werden aus Elementen der Gruppen II-VI, III-V, IV-VI und IV Halbleitern, ganz bevorzugt aus ZnO, ZnS, ZnSe, ZnTe, CdS, CdSe, CdTe, HgS, HgSe, HgTe, MgS, MgSe, GeS, GeSe, GeTe, SnS, SnSe, SnTe, PbO, PbS, PbSe, PbTe, GaN, GaP, GaAs, GaSb, InN, InP, InAs, InSb, AlN, AlP, AlAs, AlSb, GaN, GaP, GaAs, GaSb, und einer Kombination aus diesen.

In QDs and QRs, photoluminescence and electroluminescence arise from the band edge states of the nanoparticle. The radiative band-edge emission from nanoparticles competes with non-radiative decay channel originating from surface electronic states, as reported by X. Peng, et al., J. Am. Chem. Soc. Vol119:7019-7029 (1997).

Als besonders vorteilhaft haben sich auch Quantenpunkte und Quantenstäbchen gezeigt, die eine Core-Shell Struktur aufweisen (J. Am. Chem. Soc. Vol119:7019-7029 (1997).

Core-Shell Strukturen können erhalten werden, wenn organometallische Vorstufen, welche die Shell Materialien enthalten, als Vorstufen verwendet werden. Diese Vorstufen werden zu einer Reaktionsmischung mit dem Core-Nanoteilchen gegeben. Weitere Einzelheiten zum Herstellungsverfahren sind dem Fachmann aus dem Stand der Technik gut bekannt.

In einer bevorzugten Ausführungsform wird ZnS als Shell-Material verwendet.

Weiter bevorzugt ist, wenn die Quantenpunkte und Quantenstäbchen halbleitende Materialien der Gruppe II-VI, Mischungen davon und Core-Shell Strukturen davon darstellen. Ganz bevorzugt sind dabei CdSe, CdS, CdTe, ZnSe, ZnS, ZnTe, Mischungen davon.

Die Quantenpunkte und Quantenstäbchen können weitere Liganden enthalten, die an die Oberfläche der Teilchen gebunden sind. Geeignete Liganden hierfür sind im Stand der Technik gut bekannt und sind, beispielsweise, offenbart in US 10/656910 und US 60/578236. Hierdurch können verschiedene Eigenschaften der Quantenpunkte und Quantenstäbchen verbessert werden. Hierdurch kann die Löslichkeit in bestimmten Lösungsmittel Matrixmaterialien und Polymeren verbessert werden. Weitere bevorzugte Liganden sind in US 2007/0034833A1 und US 20050109989A1 offenbart.

Die hierin verwendeten Begriffe Quantenpunkt und Quantenstäbchen stehen für Nanoteilchen, die im Wesentlichen eine monodisperse Größenverteilung aufweisen. Die Dimension der Teilchen beträgt 500 nm und weniger, bis hin zu einer Dimension von ca. 1 nm. Monodispers bedeutet, dass die Teilchen eine Größenverteilung von +-10% der genannten Dimension der Teilchen aufweist.

Typische Strukturen und Verfahren zur Herstellung von Quantenstäbchen sind in US 2013/0135558 A1 offenbart.

Die erfindungsgemäßen Formulierungen können zur Herstellung von Schichten in elektronischen Vorrichtungen verwendet werden, wobei die Schichten, die mittels der erfindungsgemäßen Formulierung hergestellt wird aus Lösung aufgebracht wird.

Bei der Herstellung organischer elektronischer Vorrichtungen, wie z.B. den OLEDs, wird zwischen zwei grundsätzlich verschiedenen Verfahren unterscheiden. Beim ersten Verfahren werden die relevanten Verbindungen im Vakuum aufgedampft. Dieses Verfahren ist sehr aufwendig und kostenintensiv. Bei dem zweiten Verfahren werden die relevanten Verbindungen aus der Lösung aufgebracht.

Bestimmte elektronischen Vorrichtungen sind aus Mehrschichtsystemen aufgebaut. Bei der Herstellung solcher Mehrschichtsysteme können beide oben genannte Verfahren eingesetzt werden. Häufig werden einzelne Schichten aufgedampft, während andere Schichten aus Lösung prozessiert werden.

Ein weiterer Gegenstand der vorliegenden Erfindung ist daher ein Verfahren zur Herstellung einer organischen elektronischen Vorrichtung, dadurch gekennzeichnet, dass wenigstens eine Schicht der elektronischen Vorrichtung mit Hilfe der erfindungsgemäßen Formulierung aus Lösung hergestellt wird.

Typische Verfahren zur Herstellung von Schichten aus der Lösung sind z. B. das Spincoating, oder ein beliebiges Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck). Hierfür sind Formulierungen erforderlich.

Bei den organischen elektronischen Vorrichtungen handelt es sich bevorzugt um eine organische integrierte Schaltung (OIC), einen organischen Feld-Effekt-Transistor (OFET), einen organischen Dünnfilmtransistor (OTFT), eine organisch Elektrolumineszenzvorrichtung, eine organische Solarzelle (OSC), einen organischen optischen Detektor, einen organischen Photorezeptor, bevorzugt aber um eine organische Elektrolumineszenzvorrichtung.

Ganz bevorzugte organische elektrolumineszierende Vorrichtungen sind die organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

Die organische Elektrolumineszenzvorrichtung enthält Kathode, Anode, emittierende Schicht und auf Kathodenseite daran angrenzend mindestens eine Elektronentransportschicht. Außer diesen Schichten kann sie noch weitere Schichten enthalten, beispielsweise jeweils eine oder mehrere Lochinjektionsschichten, Lochtransportschichten, weitere Elektronentransportschichten, Elektroneninjektionsschichten, Exzitonenblockierschichten, Elektronenblockierschichten und/oder Ladungserzeugungsschichten (Charge-Generation Layers). Es sei aber darauf hingewiesen, dass nicht notwendigerweise jede dieser Schichten vorhanden sein muss.

In den weiteren Schichten der erfindungsgemäßen organischen Elektrolumineszenzvorrichtung, insbesondere in den Lochinjektions- und -transportschichten und in den Elektroneninjektions- und -transportschichten, können alle Materialien verwendet werden, wie sie üblicherweise gemäß dem Stand der Technik eingesetzt werden. Dabei können die Lochtransportschichten auch p-dotiert bzw. die Elektronentransportschichten auch n-dotiert sein. Dabei wird unter einer p-dotierten Schicht eine Schicht verstanden, in der freie Löcher erzeugt wurden und deren Leitfähigkeit dadurch erhöht ist. Eine umfassende Diskussion von dotierten Transportschichten in OLEDs findet sich in Chem. Rev. 2007, 107, 1233. Besonders bevorzugt ist der p-Dotand in der Lage, das Lochtransportmaterial in der Lochtransportschicht zu oxidieren, hat also ein ausreichend hohes Redoxpotential, insbesondere ein höheres Redoxpotential als das Lochtransportmaterial. Als Dotanden sind prinzipiell alle Verbindungen geeignet, welche Elektronenakzeptorverbindungen darstellen und die Leitfähigkeit der organischen Schicht durch Oxidation des Hosts erhöhen können. Der Fachmann kann im Rahmen seines allgemeinen Fachwissens ohne größeren Aufwand geeignete Verbindungen identifizieren. Insbesondere geeignet als Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709 und US 2010/0096600 offenbarten Verbindungen.

Der Fachmann kann daher ohne erfinderisches Zutun alle für organische Elektrolumineszenzvorrichtungen bekannten Materialien in Kombination mit der emittierenden Schicht enthaltend die erfindungsgemäßen TADF-Verbindungen einsetzen.

Insbesondere kann es bevorzugt sein, wenn zwischen der erfindungsgemäßen Elektronentransportschicht und der Kathode weitere Elektronentransportschichten und/oder Elektroneninjektionsschichten vorliegen.

Eine Elektronentransportschicht im Sinne der vorliegenden Erfindung ist eine Schicht, die zwischen der Kathode oder der Elektroneninjektionsschicht und der emittierenden Schicht angeordnet ist. Eine Elektroneninjektionsschicht im Sinne der vorliegenden Erfindung ist eine Schicht, die direkt an die Kathode angrenzt und die eine Schichtdicke von nicht mehr als 5 nm, bevorzugt 0.5 bis 5 nm, aufweist.

Dabei enthalten bevorzugt alle Elektronentransportschichten, also alle Schichten, die zwischen der Kathode bzw., wenn vorhanden, der Elektroneninjektionsschicht und der emittierenden Schicht vorliegen, mindestens eine Verbindung mit einem LUMO $\leq$ -2.55 eV.

Damit die Emission der TADF-Verbindung nicht an der direkt angrenzenden Elektronentransportschicht gelöscht wird, ist es bevorzugt, dass die niedrigste Triplettenergie dieser Elektronentransportschicht maximal 0.1 eV niedriger ist als die Triplettenergie des Moleküls, welches TADF zeigt. Insbesondere bevozugt ist $T_1$(ETL) $\geq$ $T_1$(TADF). Besonders bevorzugt gilt: $T_1$(ETL) - $T_1$(TADF) $\geq$ 0.1 eV, ganz besonders bevorzugt $T_1$(ETL) - $T_1$(TADF) $\geq$ 0.2 eV. Dabei steht $T_1$(ETL) für die niedrigste Triplettenergie der Elektronentransportschicht, die direkt an die emittierende Schicht angrenzt, und $T_1$(TADF) für die niedrigste Triplettenergie der TADF-Verbindung. Dabei wird die Triplettenergie der Materialien der Elektronentransportschicht durch im Beispielteil ausführlich und allgemein offenbarte quantenchemische Verfahren ermittelt. Wenn die Elektronentransportschicht mehr als eine Verbindung enthält, gilt die Bedingung für die Triplettenergie bevorzugt für jede der Verbindungen.

Die oben genannten Bedingungen für die Triplettenergie sind nur für die direkt an die emittierende Schicht angrenzende Elektronentransportschicht bevorzugt. Sind weitere Elektronentransportschichten vorhanden, die kathodenseitig der direkt an die emittierende Schicht angrenzende Elektronentransportschicht angeordnet sind, so ist für diese weiteren Elektronentransportschichten die Triplettenergie nicht von Bedeutung, so dass hier auch Elektronentransportmaterialien mit einer geringeren Triplettenergie gewählt werden können, beispielsweise Anthracenderivate.

Die auf Kathodenseite direkt an die emittierende Schicht angrenzende Elektronentransportschicht kann auch als Lochblockierschicht wirken, kann also außer elektronentransportierenden Eigenschaften auch gleichzeitig lochblockierende Eigenschaften aufweisen. Dies ist abhängig von der Lage des HOMO-Niveaus der Schicht. Insbesondere wirkt die Schicht dann als Lochblockierschicht, wenn für das HOMO der Schicht gilt: HOMO(EML) - HOMO(ETL) > 0.2 eV, bevorzugt HOMO(EML) - HOMO(ETL) > 0.3 eV. HOMO(ETL) ist das HOMO des Materials der Elektronentransportschicht. Besteht diese Schicht aus mehreren Materialien, so ist HOMO(ETL) das am höchsten liegende HOMO dieser Materialien. HOMO(EML) ist das HOMO des Materials der emittierenden Schicht. Besteht diese Schicht aus mehreren Materialien, so ist HOMO(EML) das am höchsten liegende HOMO dieser Materialien. Dabei wird das HOMO (highest

occupied molecular orbital) jeweils durch quantenchemische Rechnungen bestimmt, wie hinten im Beispielteil allgemein erläutert.

Zur Klarstellung sei betont, dass es sich bei den Werten für HOMO und LUMO definitionsgemäß um negative Zahlenwerte handelt. Bei dem am höchsten liegenden HOMO bzw. dem höchsten HOMO handelt es sich daher um das betragsmäßig kleinste HOMO, und bei dem am tiefsten liegenden LUMO bzw. dem tiefsten LUMO handelt es sich um das betragsmäßig größte LUMO.

Die Elektronentransportschicht kann als Reinschicht vorliegen, d. h. nur aus einer Verbindung bestehen, die dann vorzugsweise ein LUMO $\leq$ -2.55 eV aufweist. Die Schicht kann auch als Mischung vorliegen, wobei dann mindestens eine der Verbindungen vorzugsweise ein LUMO $\leq$ -2.55 eV aufweist. Diese Verbindung liegt in der Schicht in einem Anteil von bevorzugt mindestens 30 Vol.-%, besonders bevorzugt mindestens 50 Vol.-%, ganz besonders bevorzugt mindestens 70 Vol.-% vor. Insbesondere bevorzugt liegt die Schicht als Reinschicht vor, d. h. sie besteht nur aus einer Verbindung, die vorzugsweise ein LUMO $\leq$ -2.55 eV aufweist. Wenn die Elektronentransportschicht eine Mischung aus zwei oder mehr Materialien enthält, ist es bevorzugt, wenn jedes dieser Materialien ein LUMO $\leq$ -2.55 eV aufweist.

Geeignete Elektronentransportmaterialien zur Verwendung in der Elektronentransportschicht sind ausgewählt aus den Stoffklassen der Triazine, der Pyrimidine, der Lactame, der Metallkomplexe, insbesondere der Be-, Zn- bzw. Al-Komplexe, der aromatischen Ketone, der aromatischen Phosphinoxide, der Azaphosphole, der Azaborole, welche mit mindestens einem elektronenleitenden Substituenten substituiert sind, der Benzimidazole, Oxadiazole und der Chinoxaline.

Als Kathode der erfindungsgemäßen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Ebenso bevorzugt sind Metalllegierungen, insbesondere Legierungen aus einem Alkalimetall oder Erdalkalimetall und Silber, besonders bevorzugt eine Legierung aus Mg und Ag. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, CsF, $CS_2CO_3$, $BaF_2$, MgO, NaF, etc.). Ebenso kommen organische Alkali- oder Erdalkalikomplexe in Frage, wie z. B. Lithiumchinolinat (LiQ). Die Schichtdicke dieser Schicht, die als Elektroneninjektionsschicht anzusehen ist, beträgt bevorzugt zwischen 0.5 und 5 nm.

Als Anode der erfindungsgemäßen Elektrolumineszenzvorrichtung sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Dabei muss mindestens eine der Elektroden transparent oder teiltransparent sein, um die Auskopplung von Licht zu ermöglichen. Bevorzugte transparente oder teiltransparente Anodenmaterialien sind leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Es ist aber auch möglich, dass der Anfangsdruck noch geringer ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Ein weiterer Gegenstand der vorliegenden Erfindung ist daher ein Verfahren zur Herstellung einer erfindungsgemäßen organischen Elektrolumineszenzvorrichtung, dadurch gekennzeichnet, dass mindestens eine Schicht mit einem Sublimationsverfahren aufgebracht wird und/oder dass mindestens eine Schicht mit einem OVPD (Organic Vapour Phase Deposition) Verfahren oder mit Hilfe einer Trägergassublimation aufgebracht wird und/oder dass mindestens eine Schicht aus Lösung, durch Spincoating oder mit einem Druckverfahren aufgebracht wird.

Die erfindungsgemäßen TADF-Verbindungen, Zusammensetzungen, Formulierungen und Vorrichtungen zeichnen sich durch einen oder mehrere der folgenden überraschenden Vorteile gegenüber dem Stand der Technik aus:

1. Die erfindungsgemäßen organischen TADF-Verbindungen lassen sich sehr leicht herstellen.

2. Die erfindungsgemäßen organischen TADF-Verbindungen weisen eine verbesserte Löslichkeit in organischen Lösungsmitteln auf, die typischerweise zur Herstellung elektronischers Vorrichtungen eingesetzt werden.

3. Vorrichtungen, insbesondere organische Elektrolumineszenzvorrichtungen hergestellt mit Hilfe der erfindungsgemäßen Formulierungen weisen vergleichbare Effizienzen und Spannungen auf im Vergleich zu Vorrichtungen aus dem Stand der Technik.

4. Organische elektronische Vorrichtungen, insbesondere organische Elektrolumineszenzvorrichtungen hergestellt mit Hilfe der erfindungsgemäßen Formulierungen sind leicht und kostengünstig herzustellen und eignen sich daher besonders gut für die Massenproduktion kommerzieller Produkte.

5. Mit Hilfe der erfindungsgemäßen Formulierungen können auf sehr einfache und kostengünstige Weise Schichten organischer elektronischer Vorrichtungen, insbesondere in organischen Elektrolumineszenzvorrichtungen hergestellt werden, die mehrere Komponenten, beispielsweise mehrere Matrixverbindungen, enthalten.

6. Mit Hilfe der erfindungsgemäßen Formulierungen können die Herstellverfahren sehr leicht an neue Anforderungen angepasst werden.

7. Die Verwendung der erfindungsgemäßen Formulierungen zur Herstellung organischer elektronischer Vorrichtungen führt zu sehr zuverlässigen Produktionsprozessen, insbesondere auch aufgrund einer verringerten Ausfallrate.

8. Die Anzahl der Kurzschlüsse in den elektronischen Vorrichtungen enthaltend die erfindungsgemäßen Verbindungen oder Zusammensetzungen sind deutlich verringert gegenüber den Vorrichtungen aus dem Stand der Technik.

9. Die Stabilität der Emitter in der Emissionsschicht kann durch Verwendung der TADF-Verbindungen gegenüber dem Stand der Technik verbessert werden.

10. Die erfindungsgemäßen Verbindungen können in höheren Konzentrationen in elektronischen Vorrichtungen eingesetzt werden, was sich vorteilhaft auf die Effizienz der Vorrichtungen auswirkt.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbarte Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße organische Elektrolumineszenzvorrichtungen herstellen.

## Beispiele

### Beispiel 1

**Bestimmungsverfahren**

**Quantenchemisches Verfahren zur Bestimmung von Orbitalenergien und elektronischer Zustände**

Die HOMO- und LUMO- Energien sowie das Triplettniveau und die Singulettniveaus der Materialien werden über quantenchemische Rechnungen bestimmt. Hierzu wird vorliegend das Programm-paket "Gaussian09, Revision D.01" (Gaussian Inc.) verwendet. Zur Berech-nung organischer Substanzen ohne Metalle (mit Methode "org." bezeichnet) wird zuerst eine Geometrieoptimierung mit der semi-empirischen Methode AM1 (Gaussian-Eingabezeile "# AM1 opt") mit der Ladung (Charge) 0 und der Multiplizität (Multiplicity) 1 durchgeführt. Im Anschluss erfolgt auf Grundlage der optimierten Geo-me-trie eine Energierechnung (single point) für den elektronischen Grundzustand und das Triplett-Niveau. Hierbei wird die TDDFT-Methode (time dependent density functional theory) B3PW91 mit dem Basissatz 6-31 G(d) (Gaussian-Eingabezeile "# B3PW91/6-31G(d) td=(50-50,nstates=4)") verwendet (Ladung 0, Multiplizität 1). Für metallorganische Verbindungen (mit Methode "M-org." bezeichnet) wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt, wie oben beschrieben, analog zu der der organischen Substanzen, mit dem Unterschied, dass für das Metallatom der Basissatz "LanL2DZ" und für die Liganden der Basissatz "6-31 G(d)" verwendet wird (Gaussian-Eingabezeile "#B3PW91/gen pseudo=lanl2 td=(50-50,nstates=4)"). Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Daraus wird der anhand von Cyclovoltammetriemessungen kalibrierte HOMO- und LUMO-Wert in Elektronenvolt wie folgt bestimmt:

$$\mathrm{HOMO(eV)} = (\mathrm{HEh}*27.212)*0.8308-1.118$$

$$\mathrm{LUMO(eV)} = (\mathrm{LEh}*27.212)*1.0658 -0.5049$$

Diese Werte sind im Sinne dieser Anmeldung als HOMO bzw. als LUMO der Materialien anzusehen.

Das Triplett-Niveau $T_1$ eines Materials ist definiert als die relative Anregungsenergie (in eV) des Triplettzustands mit der niedrigsten Energie, der sich aus der quanten-chemischen Energierechnung ergibt.

Das Singulett-Niveau $S_1$ eines Materials ist definiert als die relative Anregungsenergie (in eV) des Singulettzustands mit der zweitniedrigsten Energie, der sich aus der quanten-chemischen Energierechnung ergibt.

Der energetisch niedrigste Singulettzustand wird als $S_0$ bezeichnet.

Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian09, Revision D.01" verwendet.

In Tabelle 3 sind die HOMO- und LUMO-Energiewerte sowie $S_1$ und $T_1$ der verschiedenen Materialien angegeben.

**Bestimmung der Überlappung von Orbitalen**

Die Überlappung der Molekülorbitale, die bei bestimmten elektronischen Übergängen beteiligt sind (Charge-Transfer-Zustände) wird mit Hilfe des Parameters A beschrieben. Dabei ist dem Fachmann die Bedeutung des Parameters A gut bekannt. Die Bestimmung des Parameters mittels Verfahren, die im Stand der Technik beschrieben sind, bereitet dem Fachmann keinerlei Schwierigkeiten. Im Rahmen der vorliegenden Erfindung wird der Parameter A anhand der PBHT-Methode gemäß D. J. Tozer et al. (J. Chem. Phys. 128, 044118 (2008)) bestimmt, die beispielsweise in dem Programmpaket Q-Chem 4.1 von Q-Chem, Inc. implementiert ist. Dabei werden die Molekülorbitale gemäß dem oben beschriebenen Verfahren berechnet. Anschließend werden die räumlichen Überlappungen für alle möglichen Paare von

besetzten Molekülorbitalen, $\varphi_i$, und unbesetzten (virtuellen) Molekülorbitalen, $\varphi_a$, gemäß folgender Gleichung ermittelt

$$O_{ia} = \langle |\varphi_i| \, || \, |\varphi_a| \rangle$$

wobei für die Berechnung die Beträge der Orbitale verwendet werden.

Der Parameter A ergibt sich dann aus der gewichteten Summe über alle Paare ia von besetzten und unbesetzten Molekülorbitalen gemäß

$$\Lambda = \frac{\sum_{ia} \kappa_{ia}^2 O_{ia}}{\sum_{ia} \kappa_{ia}^2}$$

wobei der Wert von $\kappa_{ia}$ gemäß Tozer et al. aus den Orbitalkoeffizienten in den Anregungsvektoren der gelösten TD-Eigenwertgleichung (Time-Dependent) ermittelt wird und wobei $0 \leq A \leq 1$ gilt.

**Bestimmung der PL-Quanteneffizienz (PLQE)**

Von den in den verschiedenen OLEDs verwendeten Emissionsschichten wird ein 50 nm dicker Film auf ein geeignetes transparentes Substrat, vorzugsweise Quarz, aufgebracht, d. h. die Schicht enthält dieselben Materialien in derselben Konzentration wie in der OLED. Hierbei werden die gleichen Herstellungsbedingungen wie bei der Herstellung der Emissionsschicht für die OLEDs verwendet. Von diesem Film wird ein Absorptionsspektrum im Wellenlängenbereich von 350-500 nm gemessen. Hierzu wird das Reflexionsspektrum R(λ) sowie das Transmissionsspektrum T(λ) der Probe unter einem Einfallswinkel von 6° (also nahezu senkrechter Einfall) bestimmt. Als Absorptionsspektrum im Sinne dieser Anmeldung wird A(λ)=1-P(λ)-T(λ) definiert.

Gilt A(λ) ≤ 0.3 im Bereich 350-500 nm, so wird die zum Maximum des Absorptionsspektrums gehörige Wellenlänge im Bereich 350-500 nm als $\lambda_{exc}$ definiert. Gilt für irgendeine Wellenlänge A(λ) > 0.3, so wird als $\lambda_{exc}$ die größte Wellenlänge definiert, bei der A(λ) von einem Wert kleiner 0.3 zu einem Wert größer 0.3 oder von einem Wert größer 0.3 zu einem Wert kleiner 0.3 wechselt.

Zur Bestimmung der PLQE wird ein Messplatz Hamamatsu C9920-02 verwendet. Das Prinzip beruht auf der Anregung der Probe mit Licht definierter Wellenlänge und der Messung der absorbierten und emittierten Strahlung. Die Probe befindet sich während der Messung in einer Ulbrichtkugel ("integrating sphere"). Das Spektrum des Anregungslichts ist in etwa gaußförmig mit einer Halbwertsbreite < 10 nm und Peakwellenlänge $\lambda_{exc}$ wie oben definiert.

Die PLQE wird nach dem für den genannten Messplatz üblichen Auswerteverfahren bestimmt. Es ist strengstens darauf zu achten, dass die Probe zu keinem Zeitpunkt mit Sauerstoff in Berührung kommt, da die PLQE von Materialien mit kleinem energetischen Abstand zwischen $S_1$ und $T_1$ durch Sauerstoff sehr stark reduziert wird (H. Uoyama et al., Nature 2012, Vol. 492, 234).

In Tabelle 2 ist die PLQE für die Emissionsschichten der OLEDs wie oben definiert zusammen mit der verwendeten Anregungswellenlänge angegeben.

**Bestimmung der Abklingzeit**

Zur Bestimmung der Abklingzeit wird eine Probe verwendet, die wie oben unter "Bestimmung der PL-Quanteneffizienz (PLQE)" beschrieben hergestellt wurde. Die Messung erfolgt im Vakuum. Die Probe wird bei Raumtemperatur durch einen Laserpuls geeigneter Intensität angeregt (Wellenlänge 266 nm, Pulsdauer ca. 1.5 ns). Nach der Anregung (definiert als t = 0) wird der zeitliche Verlauf der emittierten Photolumineszenz gemessen. Für die Messdaten ab dem Zeitpunkt t = 250 ns wird die Abklingzeit $t_a = t_e$ -250 ns bestimmt. Dabei ist $t_e$ derjenige Zeitpunkt nach t = 250 ns, bei dem die Intensität erstmals auf 1/e ihres Wertes bei t = 250 ns abgefallen ist.

**Bestimmung der Löslichkeit**

Um zu untersuchen, ob bei einem Material eine Toluol-Löslichkeit von 10 mg/ml oder mehr gegeben ist, wird wie folgt verfahren: 20 mg des Materials werden als Feststoff in einem Probenfläschchen vorgelegt. Bei Raumtemperatur (20°C) werden 2 ml Toluol zugegeben. Das Fläschchen wird verschlossen und der Inhalt auf einem beheizbaren Magnetrührer 1h bei 60°C gerührt. Dabei wird mittels eines Aluminiumblocks mit Löchern, in die die Fläschchen genau hineinpassen, für guten thermischen Kontakt gesorgt. Nach 1h wird das Fläschchen entnommen und auf Raumtemperatur abkühlen gelassen. Befindet sich dann eine klare Lösung ohne größere Partikel im Fläschchen, so sind mindestens 10 mg/ml des Materials in Toluol löslich.

Löslichkeiten mit andere Löslichkeitsgrenzen werden ganz dazu bestimmt. Die Einwaage wird auch auf 2ml Lösungsmittel angepasst.

## Beispiel 2

### Synthese der Verbindungen 6a bis 6f

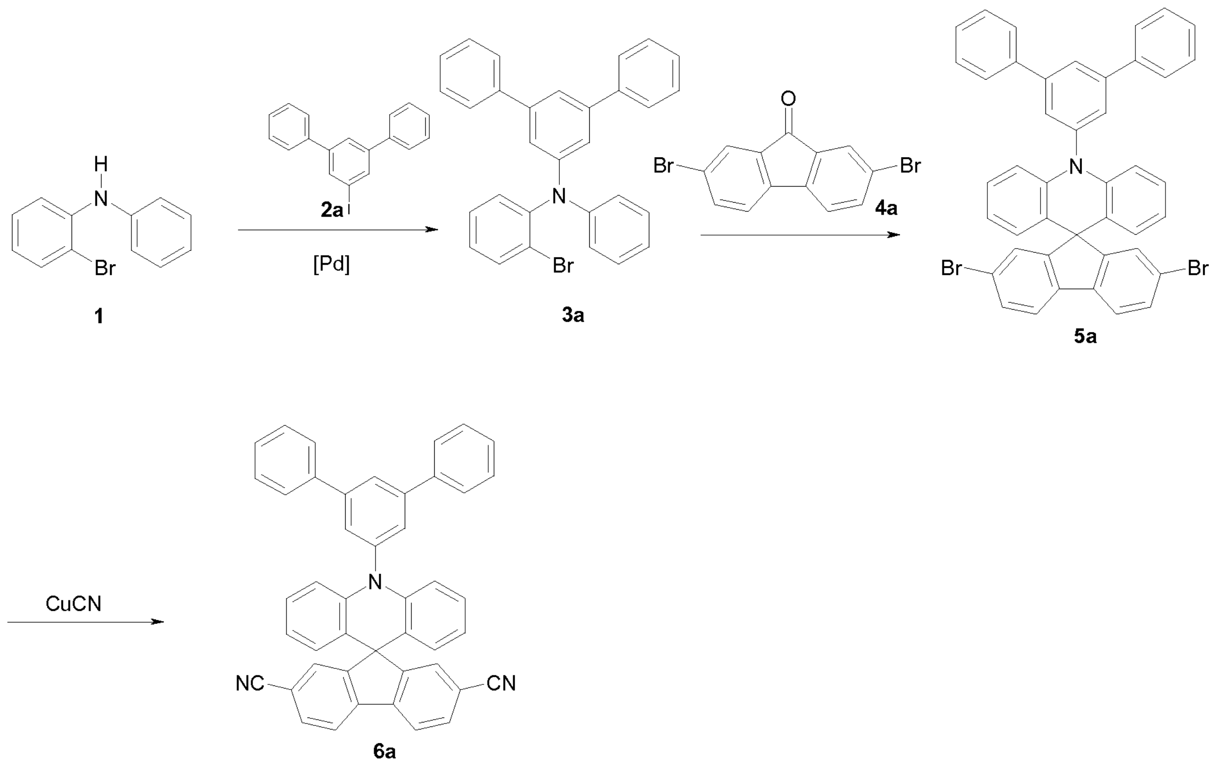


1 3a 5a 6a

### Stufe 1

(2-Bromo-phenyl)-phenyl-[1,1';3',1"]terphenyl-5'-yl-amin 3a

Zu einer Suspension aus 50.0 g (200 mmol, 1.0 eq) *N*-Phenyl-2-bromoanilin [61613-22-7] und 85.5 g (240 mmol, 1.2 eq) 5'-Iodo-1,1':3',1"-terphenyl [87666-86-2] in 500 ml Toluol werden 23.1 g (240 mmol, 1.2 eq) Natrium-t*ert*-butoxide gegeben und das Gemisch für 30 Minuten entgast. Anschließend werden 1.35 g (6.0 mmol, 0.03 eq) Palladium(II)-acetat und 10 ml (10 mmol, 0.05 eq) einer 1 N Tri-*tert*-butyl-phosphonatlösung in Toluol zugegeben. Der Ansatz wird über Nacht unter Rückfluss erhitzt und nach beendeter Reaktion mit Wasser gewaschen. Die wässrige Phase wird zweimal mit Toluol extrahiert, die vereinten organischen Phasen über Natriumsulfat getrocknet und das Lösungsmittel am Rotationsverdampfer entfernt. Es werden 89.6 g (188 mmol, 94%) eines bräunlichen Öls erhalten.

Analog werden hergestellt:

| Nr. | Edukt 2 | Produkt | Ausbeute |
|---|---|---|---|
| **3b** | 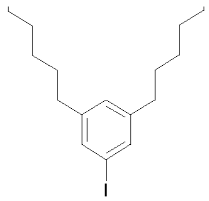 | 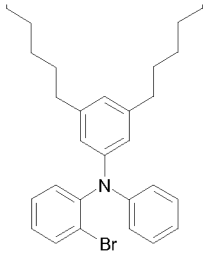 | 87% |
| **3c** | 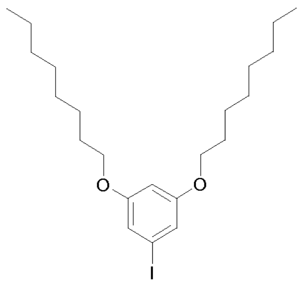 [1264968-31-1] | 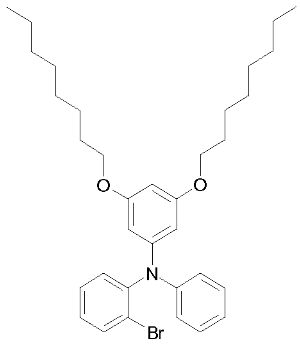 | 64% |
| **3d** | 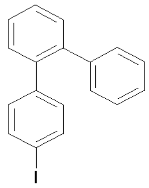 [24253-38-1] | 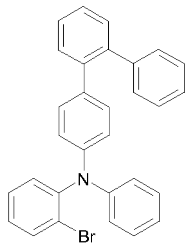 | 92% |
| **3e** | 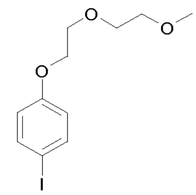 [1227071-97-7] | 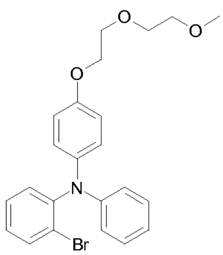 | 59% |
| **3f** | 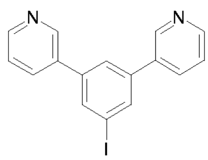 [1214386-08-9] | 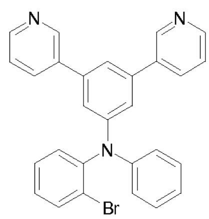 | 89% |
| **3g** | 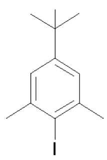 [5122-20-3] | 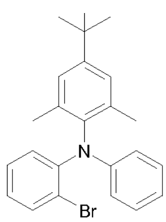 | 95% |

(fortgesetzt)

| Nr. | Edukt 2 | Produkt | Ausbeute |
|---|---|---|---|
| 3h | 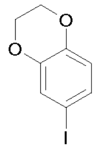 [57744-67-9] | 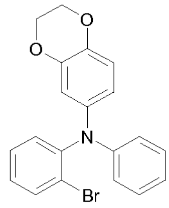 | 32% |
| 3i | 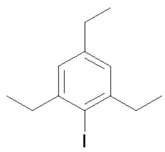 [2100-21-2] | 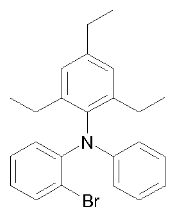 | 84% |
| 3j | 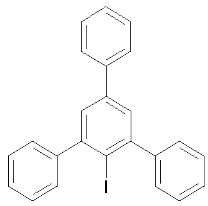 [97388-36-8] | 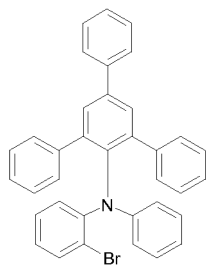 | 65% |

**Stufe 2**

2',7'-Dibromo-10H-Terphenyl-10H-spiro(acridin-9,9'-fluoren) **5a**

Eine Lösung aus 89.0 g (187 mmol, 1.0 eq) des Amins **3a** wird in 1 L getrocknetem THF gelöst und mit 75 ml n-BuLi (187 mmol, 1.0 eq, 2.5 M in Hexan) bei -78°C versetzt. Nach beendeter Zugabe wird das Reaktionsgemisch für eine Stunde bei dieser Temperatur gerührt und anschließend eine Lösung aus 63.2 g (187 mmol, 1.0 eq) **4a** in 400 ml getrocknetem THF zugegeben. Das Reaktionsgemisch wird für weitere 30 Minuten bei -78°C gerührt und über Nacht auf Raumtemperatur erwärmt. Anschließend wird die Reaktion mit Wasser gequencht, die organische Phase nach Zugabe von etwas Diethylether abgetrennt und mit Wasser und ges. Kochsalzlösung gewaschen. Die organische Phase wird über Natriumsulfat getrocknet und das Lösungsmittelgemisch am Rotationsverdampfer entfernt. Das isolierte Zwischenprodukt wird in eine Mischung aus 100 ml konz. HCl und 1 L Essigsäure gegeben und für 2 h refluxiert. Das Reaktionsgemisch wird abgekühlt, mit Wasser verdünnt und mit Dichlormethan extrahiert. Die vereinten organischen Phasen werden mit ges. Natriumhydrogencarbonatlösung und Wasser gewaschen, anschließend über Natriumsulfat getrocknet und das Lösungsmittel im Vakuum entfernt. Der erhaltene Feststoff wird aus Toluol/Heptan umkristallisiert. Es werden 79.2 g (110 mmol, 59%) eines farblosen Feststoffs erhalten.

Analog werden hergestellt:

| Nr. | Edukt 3 | Edukt 4 | Produkt 5 | Ausbeute |
|---|---|---|---|---|
| **5b** | 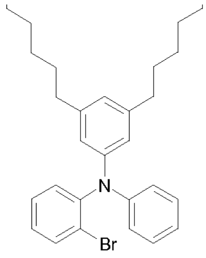 | 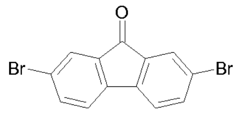 [14348-75-5] | 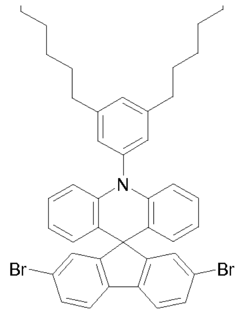 | 41% |
| **5c** | 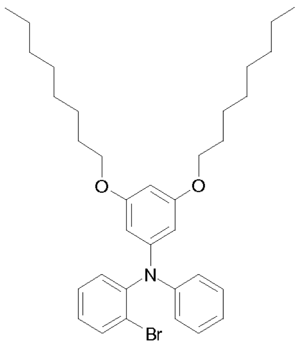 | 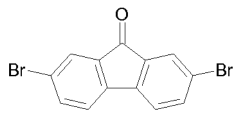 [14348-75-5] | 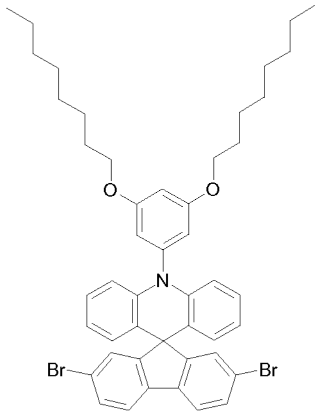 | 32% |
| **5d** | 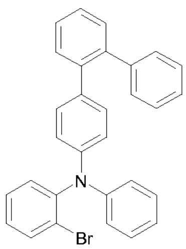 | 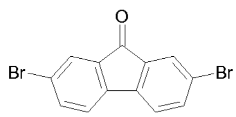 [14348-75-5] | 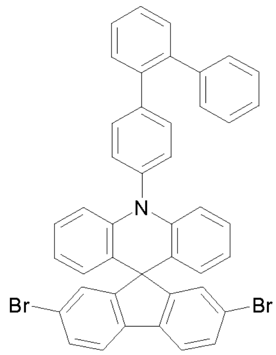 | 22% |
| **5e** | 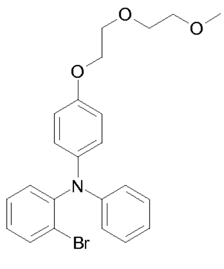 | 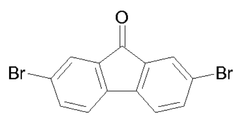 [14348-75-5] | 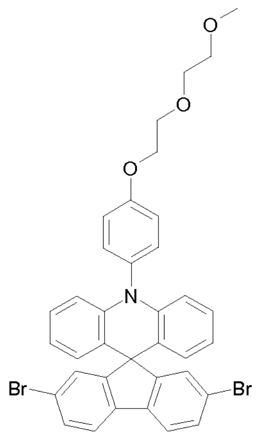 | 54% |

(fortgesetzt)

| Nr. | Edukt 3 | Edukt 4 | Produkt 5 | Ausbeute |
|---|---|---|---|---|
| **5f** | 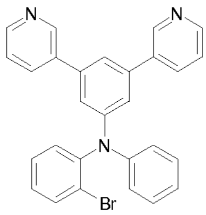 | 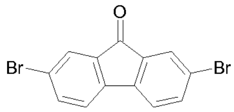 [14348-75-5] | 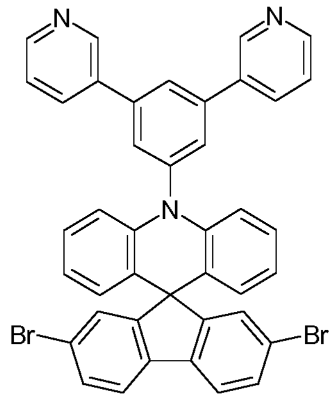 | 67% |
| **5g** | 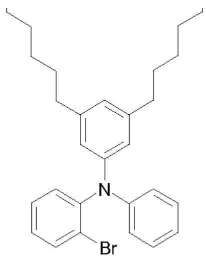 | 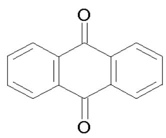 [84-65-1] | 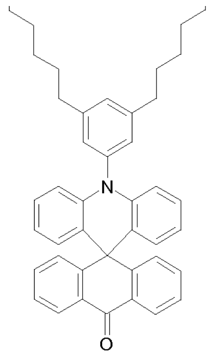 | 71% |
| **5h** | 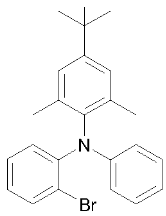 | 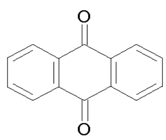 [84-65-1] | 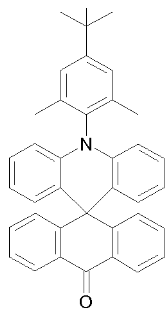 | 78% |
| **5i** | 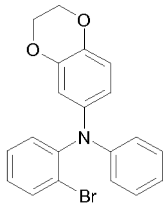 | 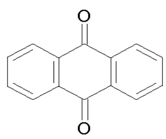 [84-65-1] | 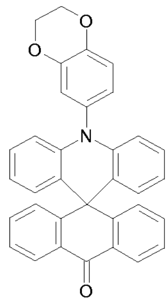 | 18% |

(fortgesetzt)

| Nr. | Edukt 3 | Edukt 4 | Produkt 5 | Ausbeute |
|---|---|---|---|---|
| 5j | 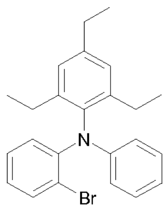 | 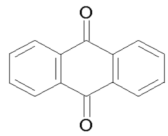 [84-65-1] | 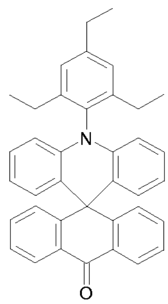 | 63% |
| 5k | 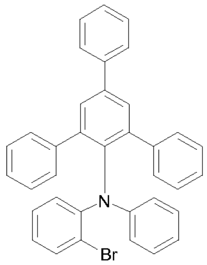 | 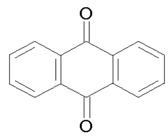 [84-65-1] | 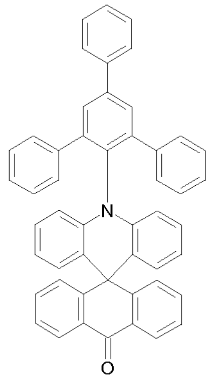 | 44% |
| 5k | 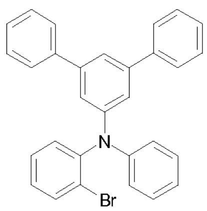 | 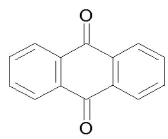 [84-65-1] | 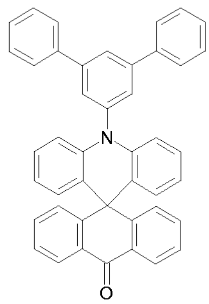 | 37% |

**Stufe 3**

2',7'-Cyano-10H-Terphenyl-10H-spiro(acridin-9,9'-fluoren) **6a**

Ein Gemisch aus 79.0 g (110 mmol, 1.0 eq) des Dibromids **5a** und 24.6 g (275 mmol, 2.5 eq) Kupfer(I)-cyanid [544-92-3] in 1.2 L NMP werden bei 170°C für 24 h gerührt. Nach beendeter Reaktion wird die Lösung auf 2 N Natronlauge gegeben und mit Natriumhypochlorit versetzt. Die erhaltene Lösung wird für eine halbe Stunde gerührt und mit Toluol extrahiert. Die vereinten organischen Phasen werden mit Wasser gewaschen, über Natriumsulfat getrocknet und das Lösungsmittel am Rotationsverdampfer entfernt (Rohausbeute: 56.3 g, 92.4 mmol, 84%). Der erhaltene Feststoff wird aus Toluol/Heptan mehrfach umkristallisiert, bis eine Reinheit von >99.9% (HPLC) erreicht wird. Abschließend wird das Produkt mittels Sublimation aufgereinigt. Es werden 28.1 g (46.2 mmol, 42%) der hochreinen Zielverbindung erhalten.

Analog werden hergestellt:

| Nr. | Edukt 5 | Produkt | Ausbeute |
|---|---|---|---|
| 6b | 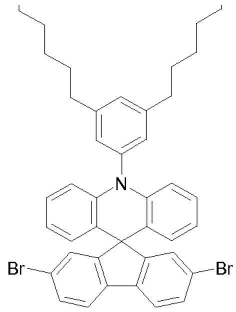 | 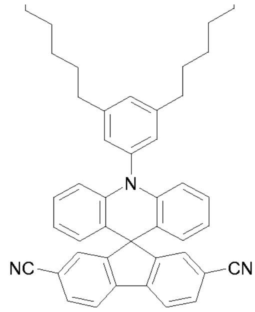 nicht sublimiert | 54% |
| 6c | 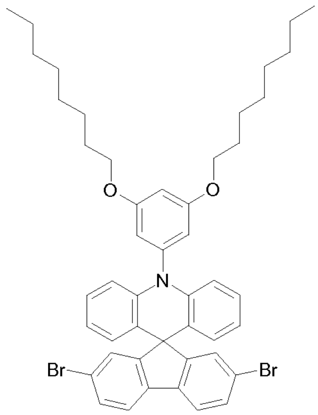 | 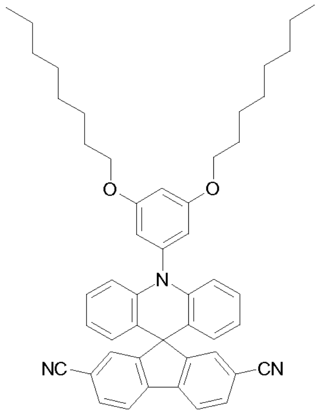 nicht sublimiert | 47% |
| 6d | 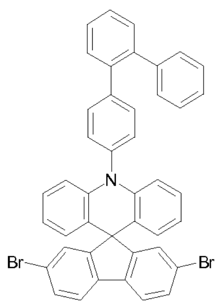 | 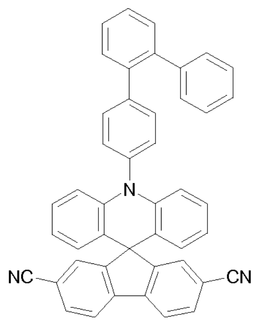 | 68% |
| 6e | 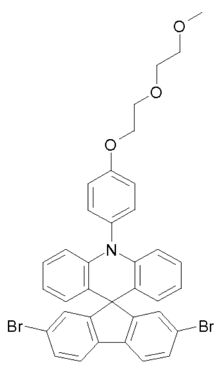 | 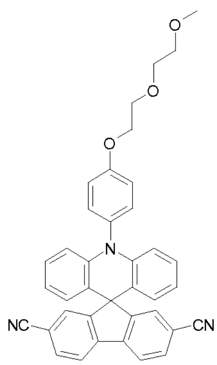 nicht sublimiert | 33% |

(fortgesetzt)

| Nr. | Edukt 5 | Produkt | Ausbeute |
|---|---|---|---|
| **6f** | 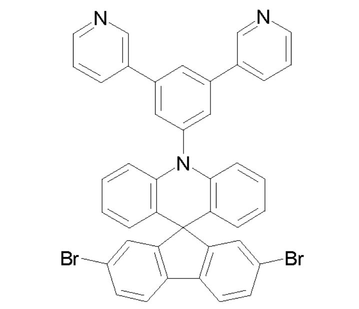 | 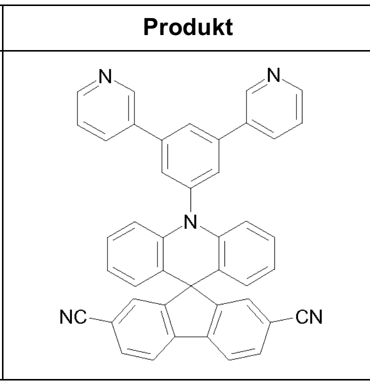 | 71% |

**Beispiel 3**

**Synthese der Verbindung 8a**

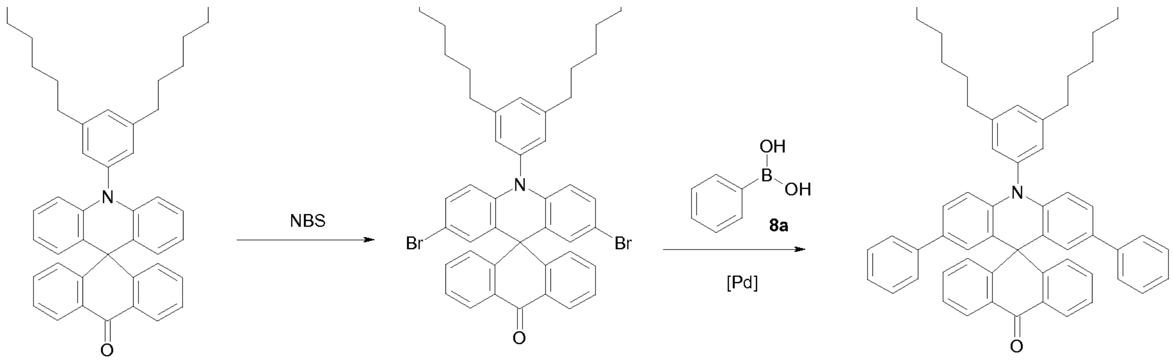


**5g** **7a** **8a**

**Stufe 1**

Es werden 20 g (33 mol, 1.0 eq) des Edukts **5g** in 500 ml DMF vorgelegt und langsam mit 12 g (68 mmol, 2.05 eq) N-Bromsuccinimid versetzt. Nach drei Stunden bei Raumtemperatur wird der ausgefallene Feststoff abgesaugt und mit Ethanol gewaschen. Es werden 24 g (31 mmol, 96%) eines beigen Feststoffs **7a** erhalten.

**Stufe 2**

Es werden 24 g (31 mmol, 1.0 eq) des Dibromids **7a,** 9.5 g (78 mmol, 2.5 eq) Phenylboronsäure und 26 g (124 mmol, 4.0 eq) Trikaliumphosphat vorgelegt und mit 375 ml Toluol, 150 ml Dioxan und 375 ml Wasser versetzt. Nachdem das Gemisch für 30 Minuten entgast wird, werden 70 mg (0.31 mmol, 0.01 eq) Palladium(II)-acetat und 570 mg (1.9 mmol, 0.06 eq) Tri-o-tolylphosphin zugegeben und über Nacht bei 85°C gerührt. Nach beendeter Reaktion werden die Phasen im Scheidetrichter getrennt, die organische Phase mit Toluol extrahiert und die vereinten organischen Phasen mit Wasser gewaschen. Die organische Phase wird anschließend über Natriumsulfat getrocknet und das Lösungsmittel am Rotationsverdampfer entfernt. Es werden 22 g (29 mmol, 93%) eines farblosen Feststoffs erhalten, der mehrfach aus Toluol/Heptan umkristallisiert wird, bis eine HPLC-Reinheit von >99.9% erreicht wird. Abschließend wird das erhaltene Zielprodukt mittels Sublimation aufgereinigt. Es werden 11.5 g (15 mmol, 48%) der hochreinen Verbindung **8a** erhalten.

**Beispiel 4**

**Synthese der Verbindungen 9a bis 9f**

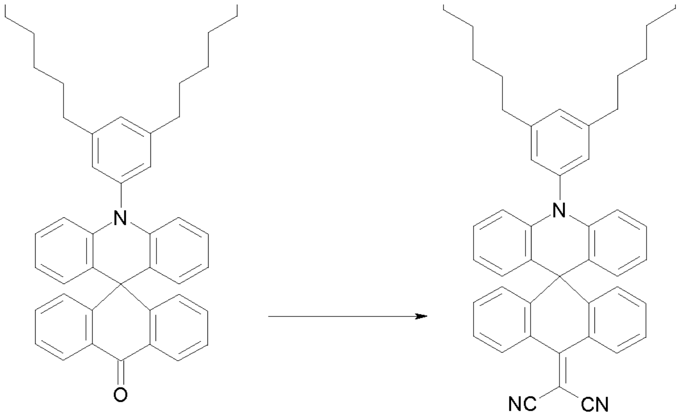

**5g** **9a**

30 g (50 mmol, 1.0 eq) des nicht-aufgereinigten Rohproduktes **5g** werden unter Schutzgas zusammen mit 16.5 g (250 mmol, 5.0 eq) Malonsäuredinitril in 1.5 L Dichlormethan gelöst. Anschließend werden langsam 250 ml (250 mmol, 5.0 eq) Titan(IV)-chlorid als 1 M Lösung in Dichlormethan, gefolgt von 20 ml (500 mmol, 10 eq) getrocknetem Pyridin zugegeben. Das Reaktionsgemisch wird für 24 Stunden unter Rückfluss gekocht und erneut dieselben Mengen Malonsäuredinitril, Titan(IV)-chlorid und getrocknetes Pyridin zugegeben. Nachdem erneut für 24 Stunden unter Rückfluss gekocht wird, werden 500 ml Dichlormethan zugegeben und die organische Phase sukzessive mit 10% HCl und 5% NaOH gewaschen. Die organische Phase wird über Natriumsulfat getrocknet und das Lösungsmittel am Rotationsverdampfer entfernt. Der erhaltene Feststoff 11.7 g (18 mmol, 36%) wird mit Toluol/Heptan umkristallisiert bis eine HPLC-Reinheit von >99.9% erreicht wird, abschließend wird eine Sublimation durchgeführt. Es werden 3.6 g (5.5 mmol, 11%) des hochreinen Produkts **9a** erhalten.

Analog werden hergestellt:

| **Nr.** | **Edukt 8** | **Produkt** 9 | **Ausbeute** |
|---|---|---|---|
| **9b** | 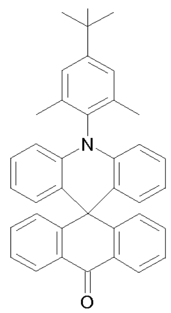 | 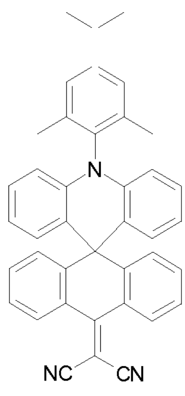 | 14% |

(fortgesetzt)

| Nr. | Edukt 8 | Produkt 9 | Ausbeute |
|---|---|---|---|
| 9c | 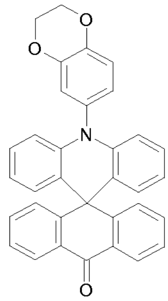 | 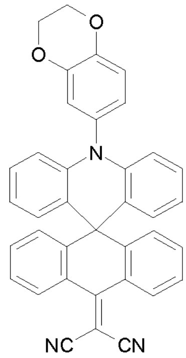 | 9% |
| 9d | 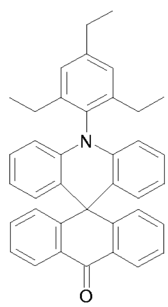 | 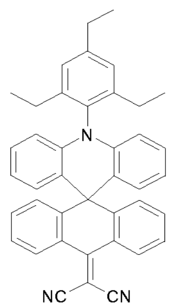 | 24% |
| 9e | 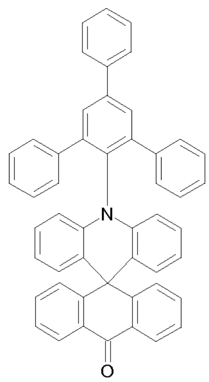 | 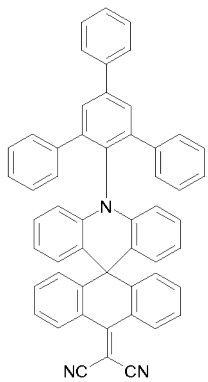 | 21% |
| 9f | 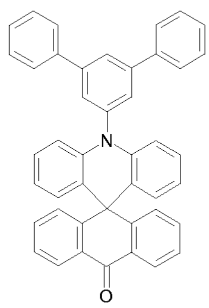 | 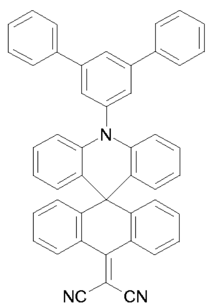 | 13% |

**Beispiel 5**

**Synthese der Verbindungen 14a bis 14i**

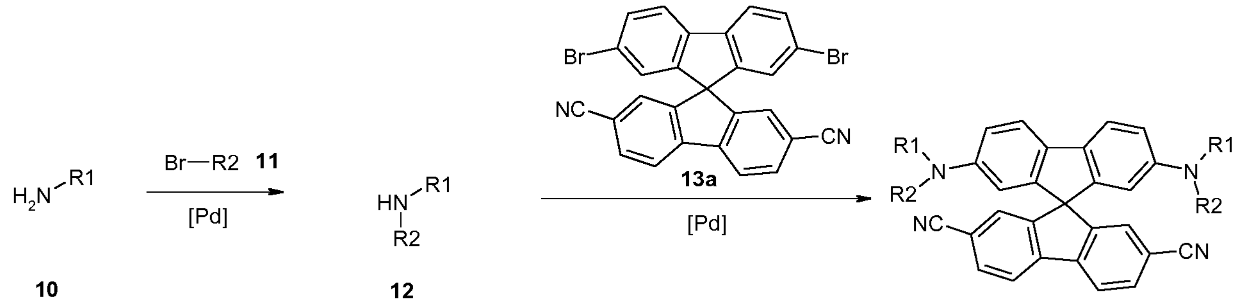


10 12 14

**Stufe** 1

Bildung der sekundären Amine **12a bis 12h**

Die Synthese wird entsprechend der Vorschrift für Verbindung **3a** durchgeführt.

Analog werden erhalten:

| Nr. | Edukt 10 | Edukt 11 | Produkt 12 | Ausbeute |
|---|---|---|---|---|
| **12a** | 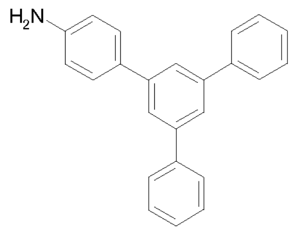 [343239-58-7] | 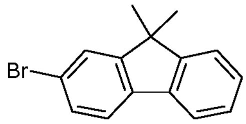 [28320-31-2] | 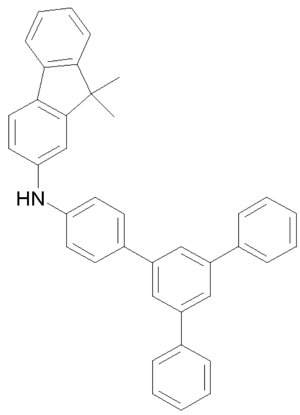 | 64% |
| **12b** | 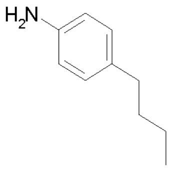 [104-13-2] | 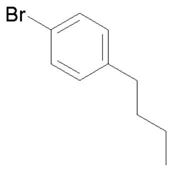 [41492-05-1] | 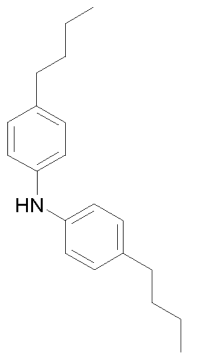 | 57% |
| **12c** | 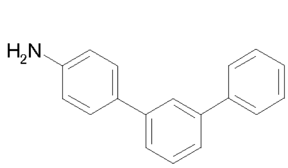 [78626-54-7] | 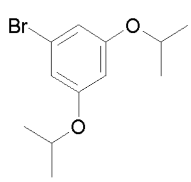 [1310418-54-2] | 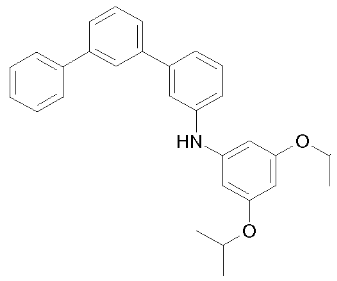 | 61% |

(fortgesetzt)

| Nr. | Edukt 10 | Edukt 11 | Produkt 12 | Ausbeute |
|---|---|---|---|---|
| **12d** | 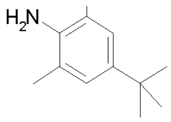 [42014-60-8] | 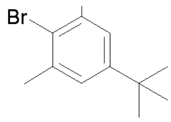 [5345-05-1] | 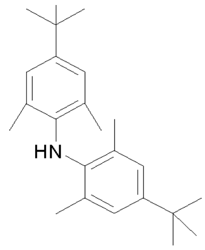 | 48% |
| **12e** | 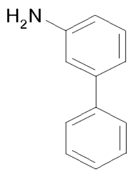 [2243-47-2] | 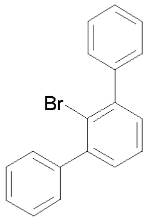 [126866-29-3] | 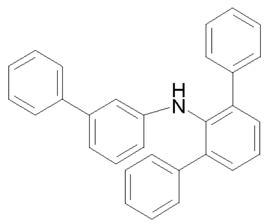 | 56% |
| **12f** | 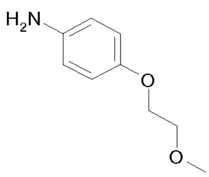 [33311-29-4] | 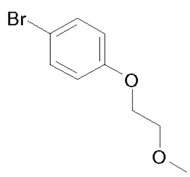 [39255-23-7] | 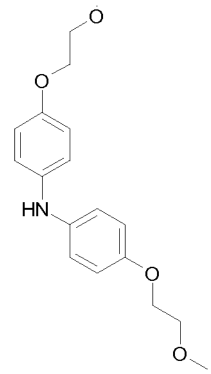 | 67% |
| **12g** | 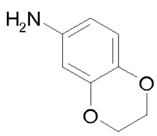 [22013-33-8] | 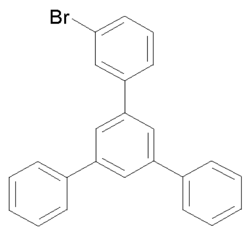 [126866-29-3] | 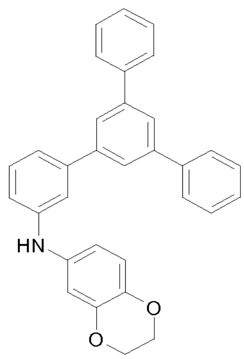 | 31% |
| **12h** | 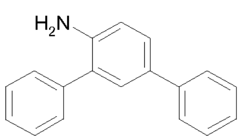 [63344-48-9] | 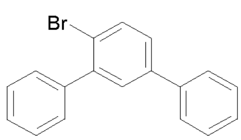 [60631-83-6] | 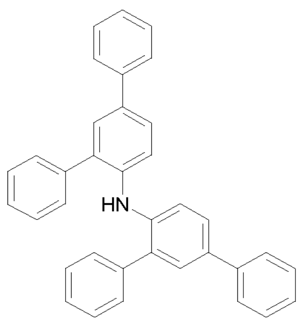 | 45% |

**Stufe 2**

Darstellung der Zielverbindungen **14a bis 14i**

Es werden 5.7 g (11 mmol, 1.0 eq) 2,7-Dibromo-2',7'-dicyano-9,9'spirobifluoren und 17 g (33 mmol, 3.0 eq) des Amins **12a** in 100 ml trockenem Toluol vorgelegt und für 30 Minuten mit Argon entgast. Anschließend werden 62 mg (0.28mmol, 0.025 eq) Palladium(II)-acetat und 0.55 ml (0.55 mmol, 0.05 eq) tri-*tert*-Butylphosphin (1 M in Toluol) zugegeben und der Ansatz über Nacht unter Rückfluss erhitzt. Nach beendeter Reaktion wird Wasser zugegeben, die organische Phase abgetrennt und erneut mit Wasser gewaschen. Anschließend werden die vereinten wässrigen Phasen mit Toluol extrahiert. Die organischen Phasen werden über Natriumsulfat getrocknet und das Lösungsmittel am Rotationsverdampfer entfernt. Es werden 11.5 g (8.3 mmol, 75%) eines beigen Feststoffs erhalten, welcher durch Umkristallisation mit Heptan/Toluol weiter aufgereinigt wird bis eine HPLC-Reinheit >99.9% erreicht wird. Abschließend wird der Feststoff für fünf Stunden unter Vakuum bei 300°C getempert. Es werden 8.9 g (6.4 mmol, 58%) der Zielverbindung **14a** erhalten.

Analog werden erhalten:

| Nr. | Edukt 12 | Edukt 13 | Produkt 14 | Ausbeute |
|---|---|---|---|---|
| **14b** | 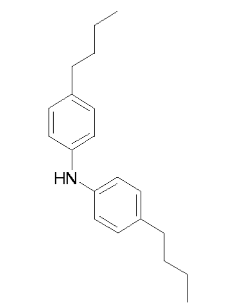 | 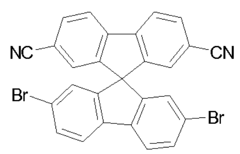 [873657-58-0] | 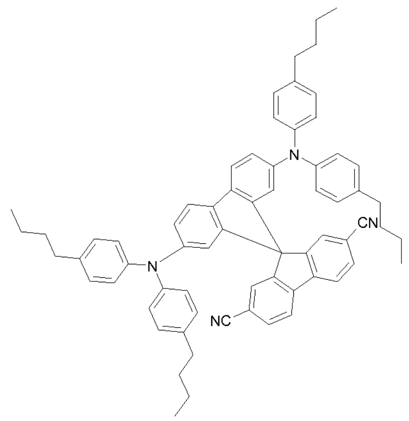 | 58% |
| **14c** | 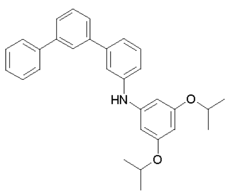 | 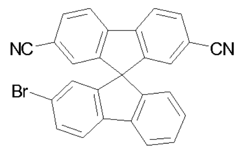 [876173-76-1] | 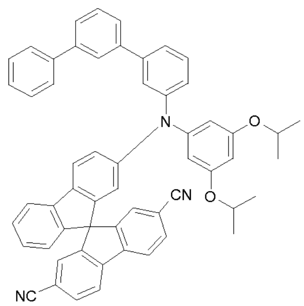 | 31% |
| **14d** | 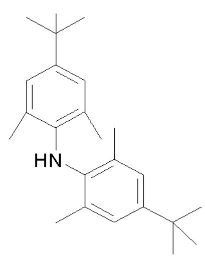 | 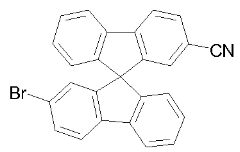 [1428263-48-2] | 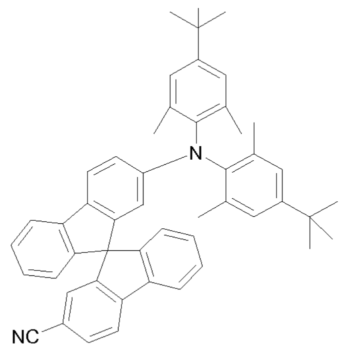 | 46% |

(fortgesetzt)

| Nr. | Edukt 12 | Edukt 13 | Produkt 14 | Ausbeute |
|---|---|---|---|---|
| 14e | 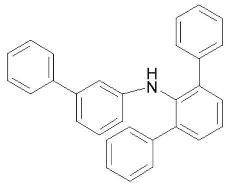 | 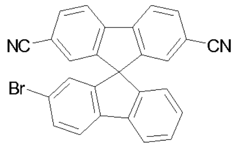 [876173-76-1] | 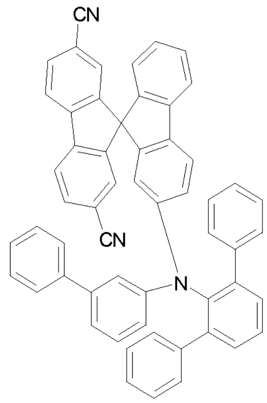 | 67% |
| 14f | 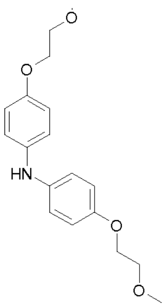 | 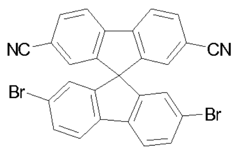 [873657-58-0] | 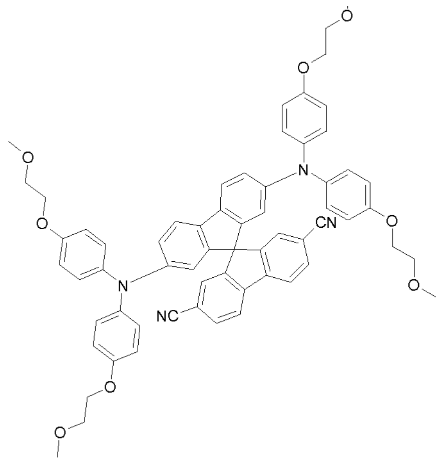 | 18% |
| 14g | 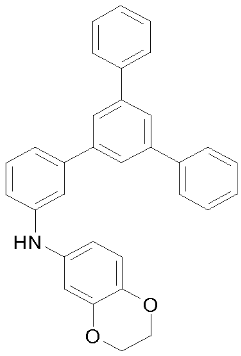 | 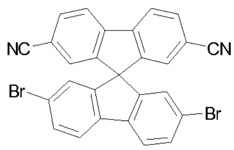 [873657-58-0] | 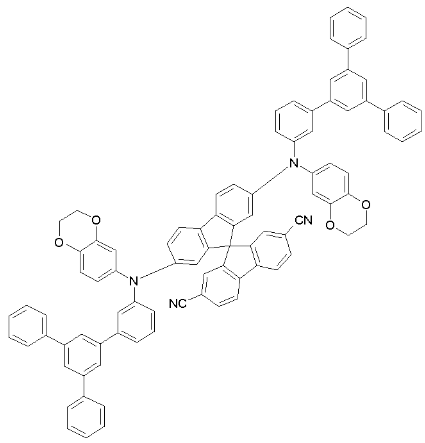 | 25% |
| 14h | 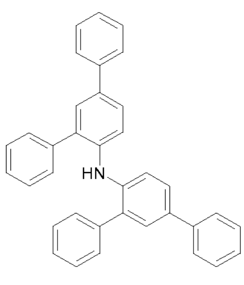 | 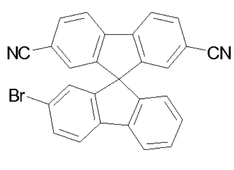 [876173-76-1] | 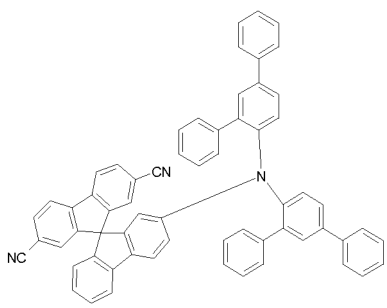 | 49% |

(fortgesetzt)

| Nr. | Edukt 12 | Edukt 13 | Produkt 14 | Ausbeute |
|---|---|---|---|---|
| 14i | 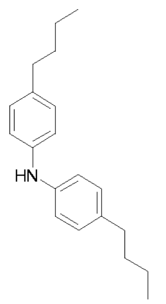 | 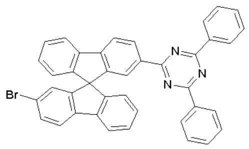 [1421827-56-6] | 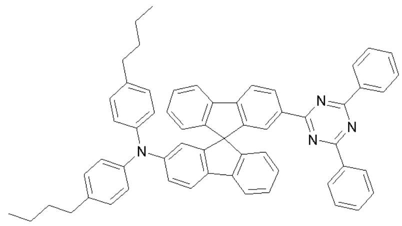 | 69% |

## Beispiel 6

**Synthese der Verbindungen 19a bis 19f**

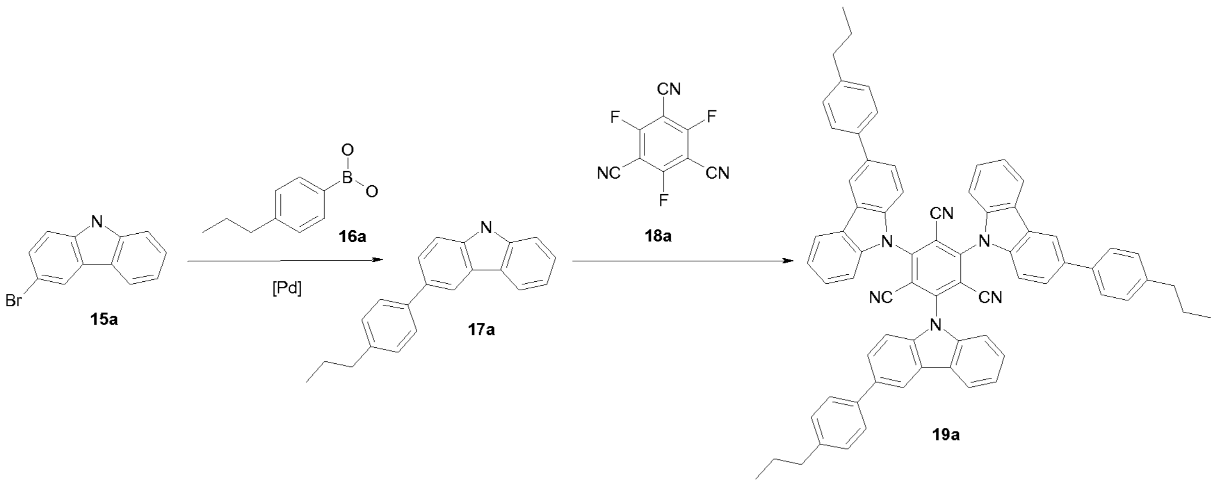


15a 16a 17a 18a 19a

**Stufe 1**

3-(4-Propyl-phenyl)-9H-carbazol **17a**

25 g (100 mmol, 1.0 eq) 3-Brom-9H-carbazol werden zusammen mit 20 g (120 mmol, 1.2 eq) 4-Propylphenylboronsäure [134150-01-9] und 23 g Trikaliumphosphat (106 mmol, 1.06 eq) in je 350 ml Toluol, Dioxan und Wasser vorgelegt und für 30 Minuten entgast. Anschließend werden 450 mg (2.0 mmol, 0.02 eq) Palladium(II)-acetat und 1.5 g (5.0 mmol, 0.05 eq) Tri-o-tolylphosphin zugegeben und das Reaktionsgemisch über Nacht auf Rückfluss erhitzt. Nach beendeter Reaktion wird die organische Phase abgetrennt und mit Wasser gewaschen. Die wässrige Phase wird mit Toluol extrahiert und die vereinten organischen Phasen über Natriumsulfat getrocknet. Der erhaltene Feststoff wird mit Ethanol ausgewaschen. Nach Trocknung werden 26 g (91 mmol, 91%) eines leicht beigen Feststoffs **17a** erhalten.

Analog werden hergestellt:

| Nr. | Edukt 15 | Edukt 16 | Produkt 17 | Ausbeute |
|---|---|---|---|---|
| **17b** | 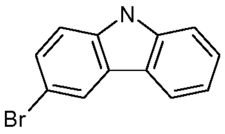 [1592-95-6] | 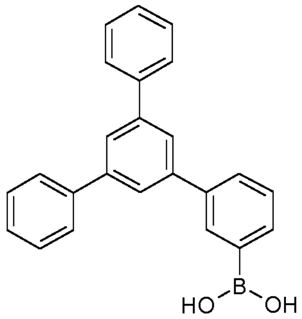 [1233200-59-3] | 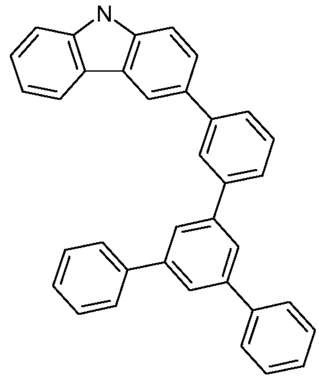 | 88% |
| **17c** | 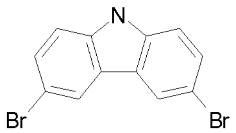 [6825-20-3] | 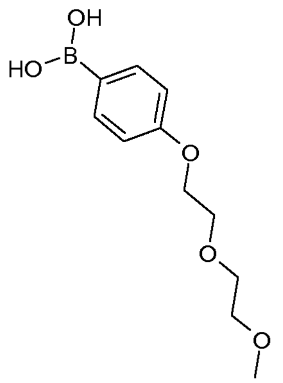 [1333122-38-5] | 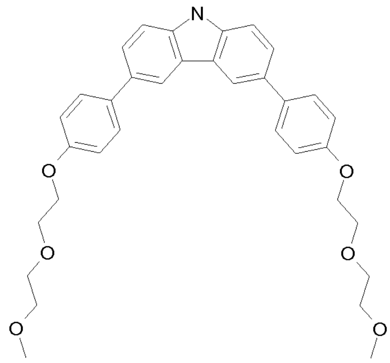 | 64% |

**Stufe 2**

Synthese der Zielverbindung **18a**

**Variante A:**

Eine gut gerührte Suspension von 3.6 g (91 mmol, 4.0 eq) Natriumhydrid, 60 Gew.-% ige Dispersion in Mineralöl, in 500 ml THF wird unter Eiskühlung bei ca. +10°C portionsweise mit 26 g (91 mmol, 4.0 eq) der Verbindung **17a** versetzt - Vorsicht Wasserstoffentwicklung! Schäumen! Nach beendeter Zugabe wird die Mischung 30 Minuten nachgerührt und dann portionsweise unter Eiskühlung so mit 6.6 g (23 mmol, 1.0 eq) 1,3,5-Tricyano-2,4,6-trifluorbenzol [363897-9] versetzt, dass die Temperatur +20°C nicht übersteigt. Nach beendeter Zugabe rührt man 2 h bei +10°C nach, entfernt dann das Kältebad, lässt auf 20-25°C erwärmen, rührt 2 h nach und erwärmt dann noch 12 h auf 40°C. Nach Abkühlen auf Raumtemperatur wird die Reaktion durch Zutropfen von 30 ml Methanol beendet und die Reaktionsmischung im Vakuum fast bis zur Trockene eingeengt. Der Rückstand wird zweimal mit je 225 ml eines Gemischs aus 175 ml Methanol und 50 ml Wasser und dann einmal mit 100 ml Methanol heiß ausgerührt. Die Reinigung erfolgt durch mehrfache Umkristallisation aus Heptan/Toluol bis eine HPLC-Reinehit >99.9% erreicht wird. Es werden 4.8 g (4.8 mmol, 21%) eines farblosen Feststoffs **18a** erhalten.

**Variante B:**

Eine gut gerührte Suspension von 3.6 g (91 mmol, 4.0 eq) des Carbazols **17a,** 6.6 g (23 mmol, 1.0 eq) 1,3,5-Tricyano-2,4,6-trifluorbenzol, 24.4 g (115 mmol, 5.0 eq) Trikaliumphosphat, wasserfrei und 200 g Glaskugeln werden in 500 ml Dimethylacetamid 16 h bei 160 °C gerührt. Nach Erkalten gibt man 1000 ml Wasser zu, saugt vom ausgefallenen Feststoff ab, wäscht diesen zweimal mit je 300 ml Wasser, zweimal mit je 200 ml Methanol und trocknet dann im Vakuum. Die weitere Reinigung erfolgt analog zu der in Variante A beschriebenen Aufreinigung. Ausbeute: 7.8 g (7.8 mmol, 34%), Reinheit: 99.9 % n. HPLC.

Analog werden folgende Verbindungen dargestellt:

| Nr. | Edukt 17 | Edukt 18 | Produkt 19 | Ausbeute |
|---|---|---|---|---|
| **19b** | 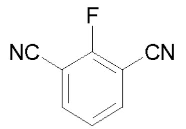 [23039-06-7] | 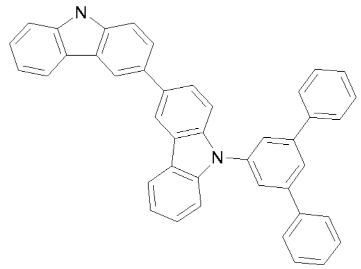 [1415349-61-9] | 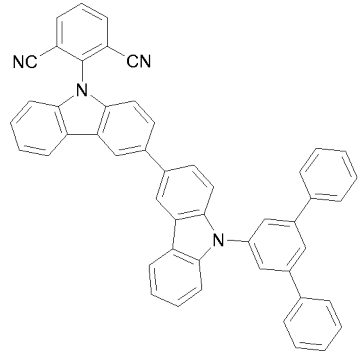 | **A** 41% |
| **19c** | 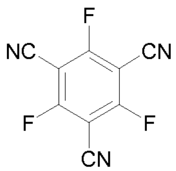 363897-9 | 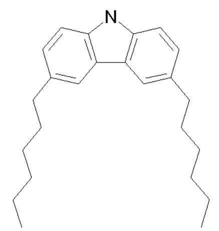 [1131605-21-4] | 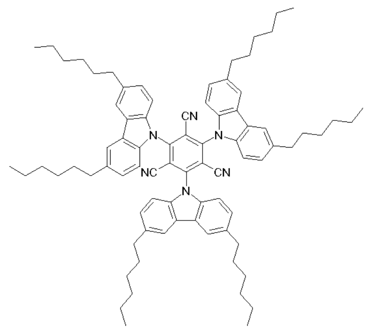 | **A** 24% |
| **19d** | 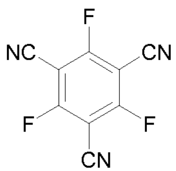 363897-9 | 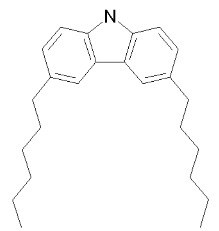 [1131605-21-4] | 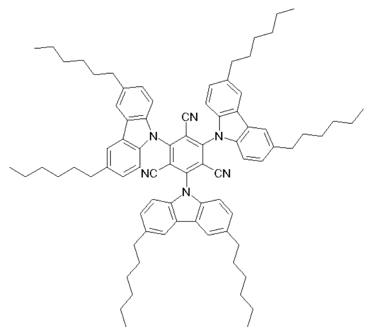 | **B** 22% |

(fortgesetzt)

| Nr. | Edukt 17 | Edukt 18 | Produkt 19 | Ausbeute |
|---|---|---|---|---|
| **19e** | 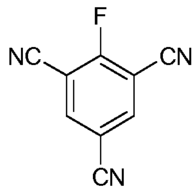 [13519-91-0] | 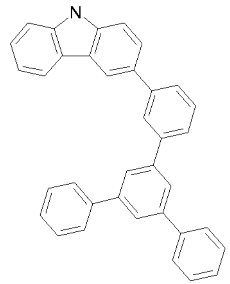 | 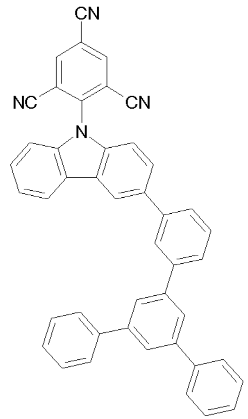 | **A** 37% |
| **19f** | 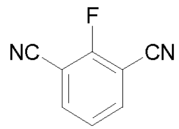 [23039-06-7] | 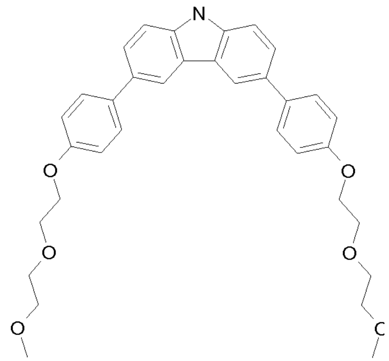 | 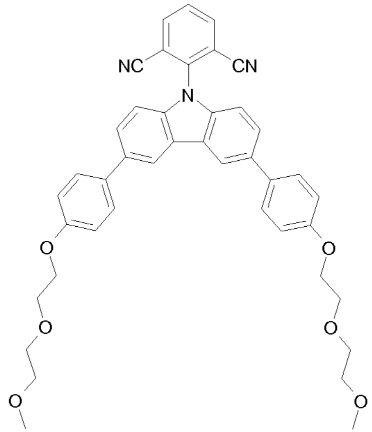 | **B** 14% |

*A und B in der Spalte Ausbeute beziehen sich auf die für die jeweiligen Verbindungen durchgeführten Varianten.*

## Beispiel 7

### Löslichkeiten

Für eine gute Verarbeitbarkeit aus Lösung ist eine hohe Löslichkeit in gängigen Lösungsmitteln von entscheidender Bedeutung. Gängige mittels Spin-coating aufgebrachte Lösungen in Toluol enthalten eine Mischung der OLED-aktiven Substanzen mit einer Gesamtkonzentration von ca. 20 mg/ml. Bei der Verwendung von höhersiedenden Lösungsmitteln wie z.B. Mesitylen müssen sogar noch höhere Konzentrationen verwendet werden, um gleiche Schichtdicken zu erreichen. Hat eine Einzelsubstanz nur eine Löslichkeit von 2 mg/ml in Toluol, so können nur Mischungen mit maximal 10% dieser Komponente aus Lösung verarbeitet werden; aus höhersiedenden Lösungsmitteln sogar noch geringere Materialanteile. Eine Löslichkeit von 5 mg/ml erlaubt dagegen die Prozessierung von Mischungen mit bis zu 25% dieser Komponente, eine Löslichkeit von 10 mg/ml entsprechend bis zu 50%.

Die Löslichkeiten ausgewählter Materialien finden sich in Tabelle 1.

**Tabelle 1:** Löslichkeiten ausgewählter Materialien

| Bsp. | Material | Löslichkeit in Toluol > 2mg/ml | Löslichkeit in Toluol > 5mg/ml | Löslichkeit in Toluol > 10mg/ml |
|---|---|---|---|---|
| V1-1 | **20a** 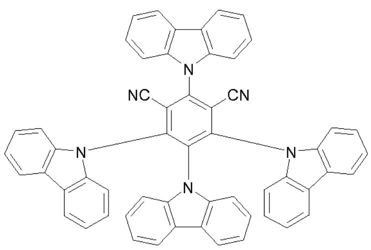 CAS 1416881-52-1 | ja | Nein | Nein |
| V1-2 | **20b** 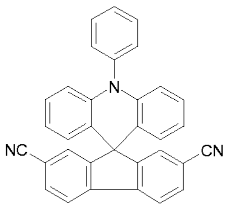 CAS 1206626-92-7 | Nein | Nein | Nein |
| V1-3 | **20c** 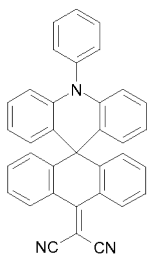 CAS 1418575-73-1 | ja | Nein | Nein |
| E1-1 | 6a | ja | ja | Nein |
| E1-2 | 9a | ja | ja | Ja |
| E1-3 | 9e | ja | ja | Nein |
| E1-4 | 19d | ja | ja | Ja |

## Beispiel 8

### Devicebeispiele

Die Materialien, die für folgenden Beispiele benötigt werden, sind in Tabelle 2 dargestellt. Die zugehörigen HOMO und LUMO Energieniveaus sowie $S_1$ und $T_1$ sind in Tabelle 3 angegeben.

### OLEDs mit aus Lösung prozessierter Emissionsschicht

Die Herstellung vollständig lösungsbasierter OLEDs ist in der Literatur bereits vielfach beschrieben, z.B. in WO 2004/037887. Die Herstellung vakuumbasierter OLEDs ist ebenfalls vielfach vorbeschrieben, u.a. in WO 2004/058911.

In den im Folgenden diskutierten Beispielen wurden lösungsbasiert und vakuumbasiert aufgebrachte Schichten innerhalb einer OLED kombiniert, so dass die Prozessierung bis einschließlich zur Emissionsschicht aus Lösung und in den darauffolgenden Schichten (Lochblockierschicht und Elektronentransportschicht) aus dem Vakuum erfolgte. Die vorbeschriebenen allgemeinen Verfahren werden dafür wie folgt auf die hier beschriebenen Gegebenheiten (Schichtdickenvariation, Materialien) angepasst und kombiniert:

Der generelle Aufbau ist wie folgt: Substrat / ITO (50 nm) / Lochinjektionsschicht(HIL) / Lochtransportschicht (HTL) / Emissionsschicht (EML) / Lochblockierschicht (HBL) / Elektronentransportschicht (ETL) / Kathode (Aluminium, 100 nm)

Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden 20 min. mit einem UV-Ozon Plasma behandelt und anschließend zur besseren Prozessierung mit PEDOT:PSS beschichtet (Poly(3,4-ethylendioxy-2,5-thiophen) : Polystyrolsulfonat, bezogen von Heraeus Precious Metals GmbH & Co. KG, Deutschland). PEDOT:PSS wird an Luft aus Wasser aufgeschleudert und nachfolgend an Luft bei 180°C für 10 Minuten ausgeheizt, um Restwasser zu entfernen. Auf diese Substrate wird eine vernetzbare Lochtransportschicht aufgebracht. Sie besteht aus einem Polymer der folgenden Strukturformel,

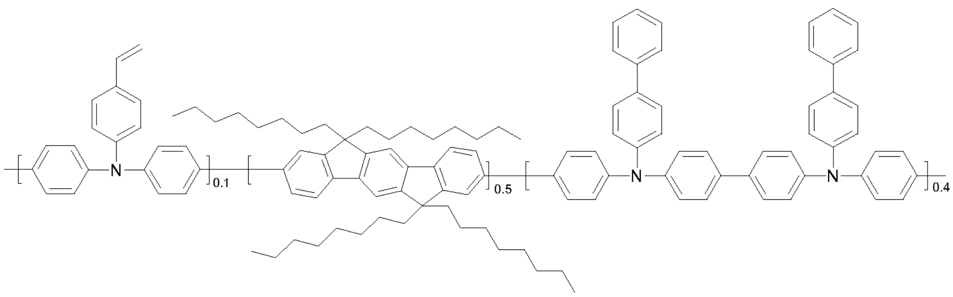

das gemäß WO 2010/097155 synthetisiert wurde. Das Material wird in Toluol gelöst. Der Feststoffgehalt der Lösung liegt bei 5 g/l. Die Schichten werden in einer Inertgasatmosphäre, im vorliegenden Fall Argon, aufgeschleudert und 60 Minuten bei 180°C ausgeheizt.

Im Anschluss wird die Emissionsschicht aufgebracht. Diese setzt sich immer aus mindestens einem Matrixmaterial (Hostmaterial, Wirtsmaterial) und einem emittierenden Dotierstoff (Dotand, Emitter) zusammen. Eine Angabe wie IC2(60%):WB1 (30%):6a(10%) bedeutet, dass das Material IC2 in einem Gewichtsanteil von 60%, WB1 in einem Gewichtsanteil von 30% und 6a in einem Gewichtsanteil von 10% in der Lösung vorliegt, aus der die Emissionsschicht hergestellt wird. Eine entsprechende Feststoffmischung für die Emissionsschicht wird in Toluol gelöst. Der Feststoffgehalt liegt bei 18 g/l. Die Emissionsschicht wird in einer Stickstoffatmosphäre aufgeschleudert und 10 Minuten bei 180°C in einer Stickstoffatmosphäre ausgeheizt.

Im Anschluss werden die Proben ohne Kontakt zu Luft in eine Vakuumkammer eingebracht und weitere Schichten durch thermisches Verdampfen aufgebracht. Besteht eine solche Schicht aus mehreren Materialien, gilt für die Mischungsverhältnisse der Einzelkomponenten die weiter oben beschriebene Nomenklatur, wobei die Gewichtsanteile durch Volumenanteile zu ersetzen sind.

Zur Charakterisierung der OLEDs werden Strom-Spannungs-Leuchtdichtekennlinien gemessen. Die Leuchtdichte wird mit einer kalibrierten Photodiode bestimmt. Weiterhin wird das Elektrolumineszenzspektrum bei einer Leuchtdichte von 1000 cd/$m^2$ gemessen. Hieraus wird unter Annahme einer lambertschen Abstrahlcharakteristik die externe Quanteneffizienz (EQE, gemessen in Prozent) berechnet.

**Vergleichsbeispiel V2-1:**

Für die Emissionsschicht wird eine Feststoffmischung IC2(60%):WB1 (30%):20a(10%) verwendet. Hieraus wird wie oben beschrieben eine 60 nm dicke Emissionsschicht hergestellt. Anschließend wird eine 10 nm dicke Schicht des Materials ST1 und danach eine 40 nm dicke Schicht ST1 (50%):LiQ(50%) durch thermisches Verdampfen im Vakuum aufgebracht. Anschließend wird als Kathode eine 100 nm dicke Aluminiumschicht durch Verdampfen im Vakuum aufgebracht.

Die OLEDs zeigen grüne Emission, 14.2% EQE bei 1000 cd/$m^2$ und benötigen für diese Leuchtdichte 7.4 V Spannung.

**Erfindungsgemäßes Beispiel E2-1:**

Die OLED entspricht dem Beispiel V2-1, mit dem Unterschied, dass statt der Mischung IC2(60%):WB1 (30%):20a(10%) die Mischung IC2(60%):WB1 (25%):19d(15%) verwendet wird.

Die OLEDs zeigen grüne Emission, 14.6% EQE bei 1000 cd/$m^2$ und benötigen für diese Leuchtdichte 7.3 V Spannung.

**Erfindungsgemäßes Beispiel E2-2:**

Die OLED entspricht dem Beispiel E2-1, mit dem Unterschied, dass die Mischung IC2(60%):WB1(20%):19d(20%) verwendet wird.

Die OLEDs zeigen grüne Emission, 14.9% EQE bei 1000 cd/$m^2$ und benötigen für diese Leuchtdichte 8.1 V Spannung.

**Erfindungsgemäßes Beispiel E2-3:**

Die OLED entspricht dem Beispiel E2-1, mit dem Unterschied, dass die Mischung IC2(60%):WB1 (25%):9a(15%) verwendet wird.

Die OLEDs zeigen grüne Emission, 12.8% EQE bei 1000 cd/$m^2$ und benötigen für diese Leuchtdichte 7.5 V Spannung.

**Erfindungsgemäßes Beispiel E2-4:**

Die OLED entspricht dem Beispiel E2-1, mit dem Unterschied, dass die Mischung IC2(60%):WB1 (20%):9a(20%) verwendet wird.

Die OLEDs zeigen grüne Emission, 13.2% EQE bei 1000 cd/$m^2$ und benötigen für diese Leuchtdichte 7.7 V Spannung.

**Erfindungsgemäßes Beispiel E2-5:**

Die OLED entspricht dem Beispiel E2-1, mit dem Unterschied, dass die Mischung IC2(60%):WB1 (25%):9e(15%) verwendet wird.

Die OLEDs zeigen grüne Emission, 12.5% EQE bei 1000 cd/$m^2$ und benötigen für diese Leuchtdichte 7.3 V Spannung.

**Erfindungsgemäßes Beispiel E2-6:**

Die OLED entspricht dem Beispiel E2-1, mit dem Unterschied, dass die Mischung IC2(60%):WB1 (20%):9e(20%) verwendet wird.

Die OLEDs zeigen grüne Emission, 13.0% EQE bei 1000 cd/$m^2$ und benötigen für diese Leuchtdichte 7.4 V Spannung.

**Erfindungsgemäßes Beispiel E2-7:**

Die OLED entspricht dem Beispiel E2-1, mit dem Unterschied, dass die Mischung IC2(60%):WB1 (20%):6a(20%) verwendet wird.

Die OLEDs zeigen grünlich blaue Emission, 10.5% EQE bei 1000 cd/$m^2$ und benötigen für diese Leuchtdichte 7.2 V Spannung.

**Vergleich der Beispiele**

In den erfindungsgemäßen Beispielen zeigt sich, dass die Verwendung höherer Konzentrationen des Dotierstoffs vorteilhaft insbesondere für die Effizienz der erzeugten Bauteile ist. Diese höheren Konzentrationen können nur bei ausreichender Löslichkeit des Dotierstoffs erreicht werden, die durch Verwendung der erfindungsgemäßen Materialien gegeben ist. Insgesamt können mit den erfindungsgemäßen Materialien effiziente lösungsprozessierte OLEDs ohne die Verwendung von Übergangsmetallkomplexen erzielt werden.

**Tabelle 2:** Strukturformeln der Materialien für die OLEDs

| | |
|---|---|
| 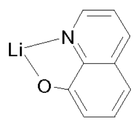 | 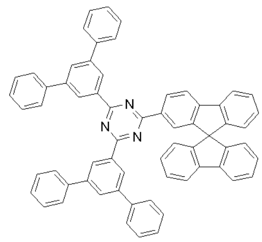 |
| LiQ | ST1 |
| 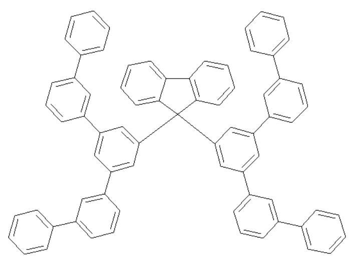 | 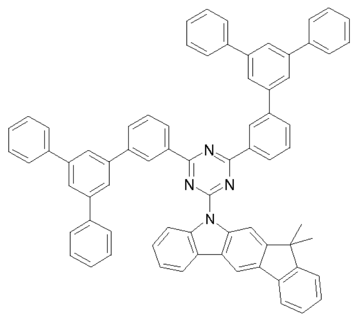 |
| WB1 | IC2 |

**Tabelle 3:** HOMO, LUMO, $T_1$, $S_1$ der relevanten Materialien

| Material | Methode | HOMO (eV) | LUMO (eV) | $S_1$ (eV) | $T_1$ (eV) |
|---|---|---|---|---|---|
| IC2 | org. | -5.78 | -2.84 | 3.04 | 2.69 |
| WB1 | org. | -6.16 | -2.24 | 3.38 | 2.95 |
| ST1 | org. | -6.03 | -2.82 | 3.32 | 2.68 |
| LiQ | M-org. | -5.17 | -2.39 | 2.85 | 2.13 |
| 6a | org. | -5.60 | -3.01 | 2.47 | 2.46 |
| 9a | org. | -5.58 | -3.32 | 2.18 | 2.17 |
| 9e | org. | -5.57 | -3.29 | 2.20 | 2.19 |
| 19d | org. | -5.89 | -3.66 | 2.11 | 2.10 |

**Patentansprüche**

1. Organische lumineszierende TADF-Verbindung der Formel (1), die keine Metalle enthält, die einen Abstand zwischen dem ersten angeregten Triplett-Zustand ($T_1$) und dem ersten angeregten Singulett-Zustand ($S_1$) von kleiner oder gleich 0.15 eV aufweist,

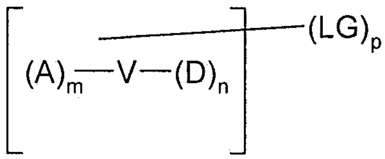

Formel (1)

wobei für die verwendeten Symbole und Indizes gilt:

m ist eine ganze Zahl ausgewählt aus 1, 2, 3, 4 oder 5;
n ist eine ganze Zahl ausgewählt aus 1, 2, 3, 4 oder 5;
p ist eine ganze Zahl, die größer oder gleich 1 ist; bevorzugt ist p eine ganze Zahl von 1 bis 12, ganz bevorzugt eine ganze Zahl von 1 bis 10, ganz besonders bevorzugt eine ganze Zahl von 1 bis 8, insbesondere bevorzugt eine ganze Zahl von 1 bis 6 und weiterhin bevorzugt eine ganze Zahl von 1 bis 4;
A ist eine Akzeptorgruppe, die eine Elektronenakzeptorgruppe darstellt, wobei die Elektronenakzeptorgruppe eine Cyanogruppe, Nitrilgruppe, Oxogruppe, $CF_3$ oder elektronenarme Heteroarylgruppe wie Pyrimidine, Pyrazine, Triazine enthält, die mit einem oder mehreren Resten $R^1$, die bei jedem Auftreten gleich oder verscheiden sein können, substituiert sein kann;
D ist eine Donorgruppe, die eine Elektronendonorgruppe darstellt, wobei die Elektronendonorgruppe eine Diarylaminogruppe, Diarylheteroarylaminogruppe, Carbazolgruppe, Indenocarbazolgruppe oder Indolocarbazolgruppe enthält, die mit einem oder mehreren Resten $R^1$, die bei jedem Auftreten gleich oder verscheiden sein können, substituiert sein kann;
LG ist eine löslichkeitsvermittelnde Gruppe, die an A, D und/oder V gebunden sein kann, bevorzugt ist sie an die Gruppen A und/oder D gebunden und ganz bevorzugt ist sie an die Gruppe D gebunden, wobei die Gruppe LG ausgewählt ist aus

einer geradkettigen Alkyl- oder Alkoxygruppe mit 3 bis 15 C-Atomen;
einer verzweigten oder cyclischen Alkyl- oder Alkoxygruppe mit 3 bis 40 C-Atomen, bevorzugt mit 3 bis 30 C-Atomen, ganz bevorzugt mit 3 bis 20 C-Atomen, ganz besonders bevorzugt mit 3 bis 15 C-Atomen und insbesondere bevorzugt mit 3 bis 10 C-Atomen;
einem aromatischen oder heteroaromatischen Ringsystem mit 3 oder mehr aromatischen oder heteroaromatischen Ringen, die mir einem oder mehreren, gleichen oder verschiedenen Resten $R^1$ substituiert sein können;
einem aromatischen oder heteroaromatischen Ringsystem mit 1 oder 2 aromatischen oder heteroaromatischen Ringen, die mit einem oder mehreren, gleichen oder verschiedenen Resten $R^1$, die ungleich H sind, in ortho Position substituiert sind, wobei die Ringe mit weiteren, gleichen oder verschiedenen Resten $R^1$ substituiert sein können;
einer Gruppe der Formel (LG-1)

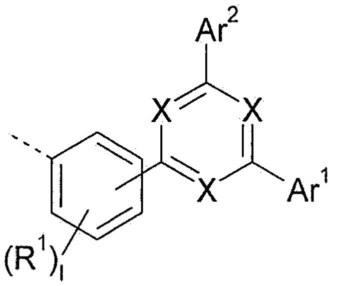

Formel (LG-1)

wobei für die verwendeten Symbole gilt:

$Ar^1$, $Ar^2$ sind jeweils unabhängig voneinander eine Aryl- oder Heteroarylgruppe, welche mit einem oder mehreren Resten $R^1$ substituiert sein kann,

X ist jeweils unabhängig voneinander N oder $CR^2$, wobei maximal eine der drei Gruppen X ein N sein darf; bevorzugt sind alle drei X gleich $CR^2$ und ganz bevorzugt ist X gleich CH,
$R^2$ ist jeweils unabhängig voneinander Wasserstoff, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Silylgruppe oder eine substituierte Ketogruppe mit 1 bis 40 C-Atomen, eine Alkoxy-carbonylgruppe mit 2 bis 40 C-Atomen, eine Aryloxycarbonylgruppe mit 7 bis 40 C-Atomen, eine vernetzbare Gruppe oder ein substituiertes oder unsubstituiertes aromatisches oder heteroaromatisches Ringsystem mit 5 bis 60 Ringatomen, oder eine Aryloxy- oder Heteroaryloxygruppe mit 5 bis 60 Ringatomen, oder eine Kombination dieser Systeme, wobei zwei oder mehrere der Gruppen $R^2$ ein mono- oder polycyclisches, aliphatisches oder aromatisches Ringsystem miteinander bilden können;
l ist 0, 1, 2, 3 oder 4;

wobei die gestrichelte Bindung die Bindung von LG an A, D oder V angibt.

V ist eine beliebige organische Brücke zwischen den Gruppen A und D oder eine Einfachbindung, wobei, wenn V eine Einfachbindung darstellt, entweder m oder n gleich 1 ist, und
wenn V eine organische Brücke darstellt, dann ist die Brücke V aus den Formeln (V-1) bis (V-7) ausgewählt,

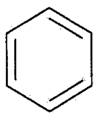
Formel (V-1)

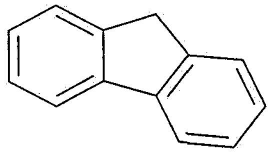
Formel (V-2)

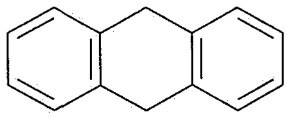
Formel (V-3)

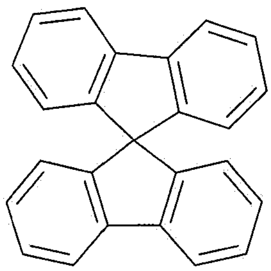
Formel (V-4)

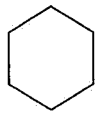
Formel (V-5)

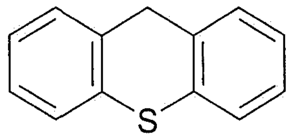
Formel (V-6)

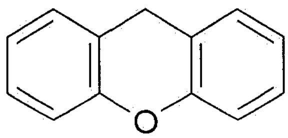
Formel (V-7)

wobei V mit einem oder mehreren Resten $R^1$, die bei jedem Auftreten gleich oder verscheiden sein können, substituiert sein kann;

$R^1$ ist gleich oder verscheiden bei jedem Auftreten Wasserstoff, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen ist oder eine Silylgruppe oder eine substituierte Ketogruppe mit 1 bis 40 C-Atomen, eine Alkoxy-carbonylgruppe mit 2 bis 40 C-Atomen, eine Aryloxycarbonyl-gruppe mit 7 bis 40 C-Atomen, eine vernetzbare Gruppe oder ein substituiertes oder unsubstituiertes aromatisches oder heteroaromatisches Ringsystem mit 5 bis 60 Ringatomen, oder eine Aryloxy- oder Heteroaryloxygruppe mit 5 bis 60 Ringatomen, oder eine Kombination dieser Systeme ist, wobei zwei oder mehrere der Gruppen $R^1$ miteinander ein mono- oder polycyclisches, aliphatisches oder aromatisches Ringsystem bilden können,

wobei bevorzugt ist, wenn zwei oder mehrere der Gruppen $R^1$ miteinander kein mono- oder polycyclisches, aliphatisches oder aromatisches Ringsystem bilden können.

2. Verbindung gemäß Anspruch 1, **dadurch gekennzeichnet, dass** die TADF-Verbindung eine organische Verbindung mit einem Molekulargewicht von kleiner oder gleich 5000 g/mol, ganz bevorzugt von kleiner oder gleich 4000 g/mol, besonders bevorzugt von kleiner oder gleich 3000 g/mol, ganz besonders bevorzugt von kleiner oder gleich 2000 g/mol, insbesondere bevorzugt von kleiner oder gleich 1500 g/mol und noch bevorzugter von kleiner oder gleich 1000 g/mol ist.

3. Verbindung gemäß Anspruch 1 oder 2, **dadurch gekennzeichnet, dass** die Gruppe LG ausgewählt ist aus den folgenden Gruppen, wobei die Gruppen weiter mit einem oder mehreren gleichen oder verschiedenen Resten $R^1$ substituiert sein können.

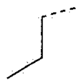

Formel (LG-5)

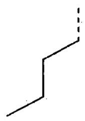

Formel (LG-6)

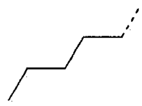

Formel (LG-7)

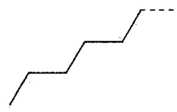

Formel (LG-8)

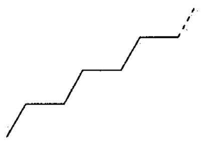

Formel (LG-9)

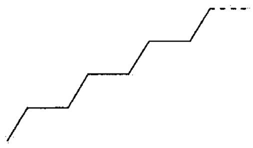

Formel (LG-10)

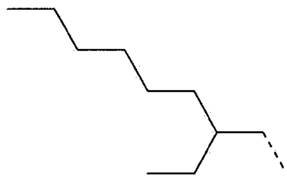

Formel (LG-11)

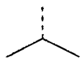

Formel (LG-12)

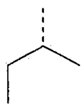

Formel (LG-13)

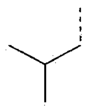

Formel (LG-14)

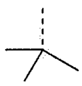

Formel (LG-15)

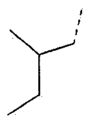

Formel (LG-16)

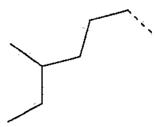

Formel (LG-17)

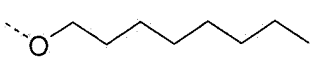

Formel (LG-18)

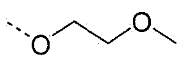

Formel (LG-19)

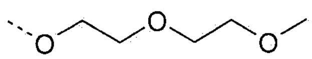

Formel (LG-20)

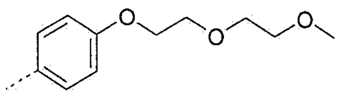
Formel (LG-21)

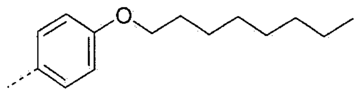
Formel (LG-22)

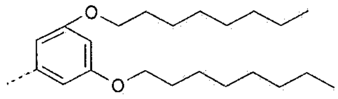
Formel (LG-23)

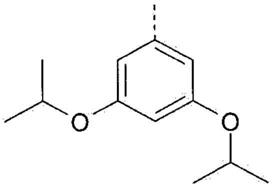
Formel (LG-24)

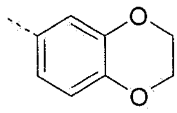
Formel (LG-25)

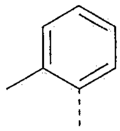
Formel (LG-26)

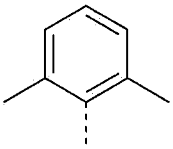
Formel (LG-27)

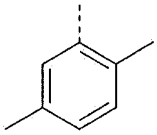
Formel (LG-28)

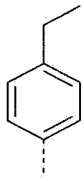
Formel (LG-29)

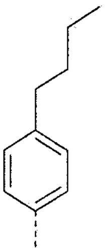
Formel (LG-30)

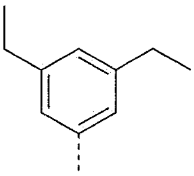
Formel (LG-31)

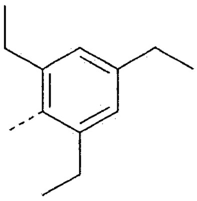
Formel (LG-32)

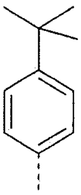
Formel (LG-33)

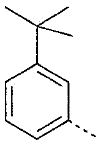
Formel (LG-34)

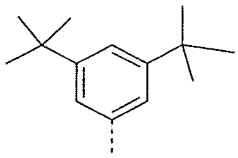
Formel (LG-35)

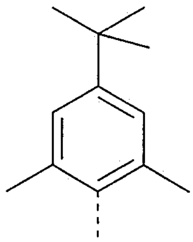

Formel (LG-36)

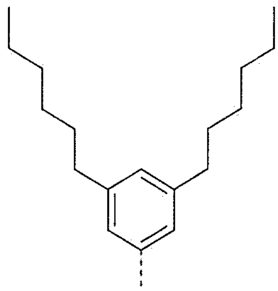

Formel (LG-37)

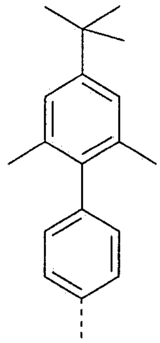

Formel (LG-38)

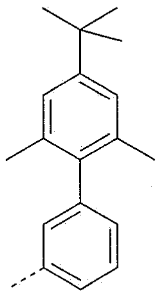

Formel (LG-39)

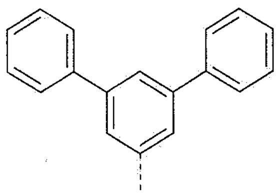

Formel (LG-40)

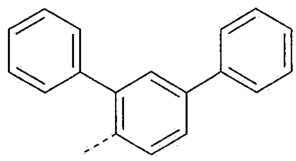

Formel (LG-41)

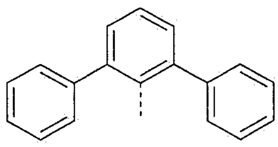

Formel (LG-42)

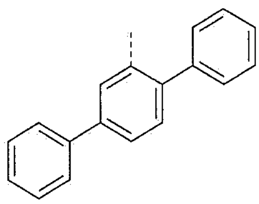

Formel (LG-43)

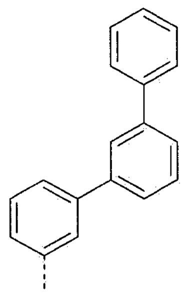

Formel (LG-44)

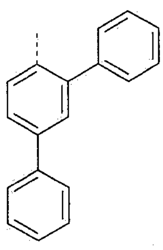

Formel (LG-45)

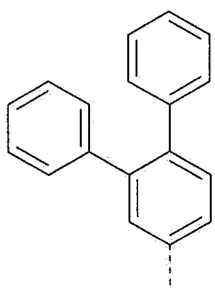

Formel (LG-46)

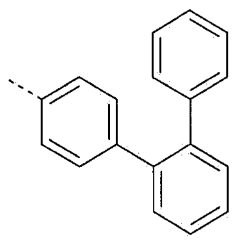

Formel (LG-47)

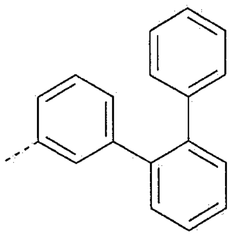

Formel (LG-48)

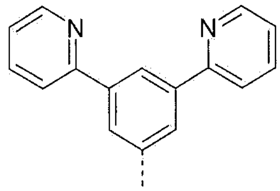

Formel (LG-49)

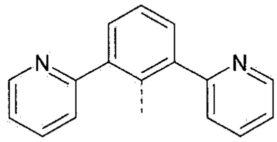

Formel (LG-50)

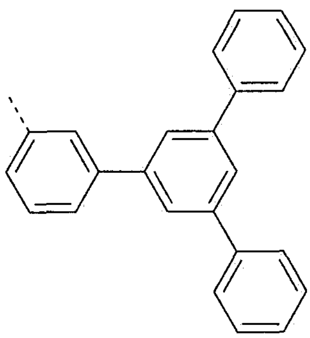

Formel (LG-51)

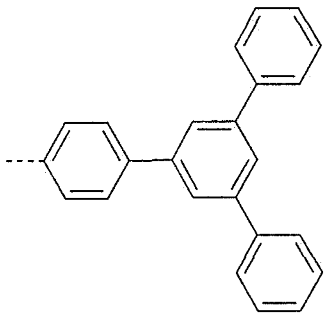

Formel (LG-52)

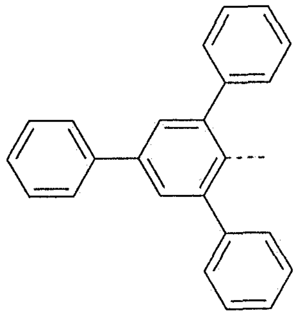

Formel (LG-53)

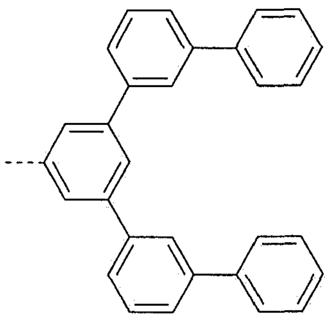

Formel (LG-54)

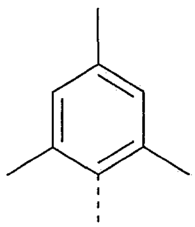

Formel (LG-27a)

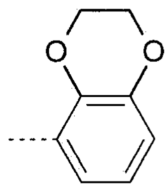

Formel (LG-55)

4. Verbindung gemäß einem oder mehreren der Ansprüche 1 bis 3, **dadurch gekennzeichnet, dass** die Verbindung wenigstens 3, bevorzugt wenigstens 4, ganz bevorzugt wenigstens 5 und insbesondere wenigstens 6 aromatische Ringe enthält.

5. Zusammensetzung enthaltend eine oder mehrere der Verbindungen gemäß einem oder mehreren der Ansprüche 1 bis 4 sowie ein oder mehrere funktionelle Materialien ausgewählt aus der Gruppe der elektronleitenden Materialien (ETM), elektroninjizierenden Materialien (EIM), elektronenblockierenden Materialien (EBM), lochleitenden Materialien (HTM), lochinjizierenden Materialien (HIM), lochblockierenden Materialien (HBM), fluoreszierenden Emittern, phosphoreszierenden Emittern, Matrixmaterialien und anorganischen Nanoteilchen, wobei Matrixmaterialien besonders bevorzugt sind

6. Zusammensetzung gemäß Anspruch 5, **dadurch gekennzeichnet, dass** die Zusammensetzung zwei Matrixmaterialien enthält.

7. Formulierung enthaltend wenigstens eine Verbindung gemäß einem oder mehreren der Ansprüche 1 bis 4 oder eine Zusammensetzung gemäß Anspruch 5 oder 6 sowie wenigstens ein Lösungsmittel.

8. Verwendung der Formulierung gemäß Anspruch 7 zur Herstellung einer oder mehrere Schichten einer organischen elektronischen Vorrichtung aus Lösung.

**9.** Verfahren zur Herstellung einer organischen elektronischen Vorrichtung, **dadurch gekennzeichnet, dass** wenigstens eine Schicht der elektronischen Vorrichtung mit Hilfe der Formulierung gemäß Anspruch 7 aus Lösung hergestellt wird.

**10.** Verwendung wenigstens einer Verbindung gemäß einem oder mehreren der Ansprüche 1 bis 4 oder wenigstens einer Zusammensetzung gemäß Anspruch 5 oder 6 in einer organischen elektronischen Vorrichtung, bevorzugt in einer organischen Elektrolumineszenzvorrichtung, ganz bevorzugt in einer organischen lichtemittierenden Diode (OLED) oder organischen lichtemittierenden elektrochemischen Zelle (OLEC, LEEC, LEC), ganz besonders bevorzugt in einer OLED und insbesondere bevorzugt in der Emissionsschicht (EML) einer OLED.

**11.** Organische elektronische Vorrichtung enthaltend wenigstens eine Verbindung gemäß einem oder mehreren der Ansprüche 1 bis 4 oder wenigstens eine Zusammensetzung gemäß Anspruch 5 oder 6.

**12.** Vorrichtung gemäß Anspruch 11, **dadurch gekennzeichnet, dass** sie ausgewählt ist aus organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren, organischen Photorezeptoren, bevorzugt eine organische Elektrolumineszenzvorrichtung.

**13.** Vorrichtung gemäß Anspruch 11 oder 12, **dadurch gekennzeichnet, dass** die Vorrichtung eine organische Elektrolumineszenzvorrichtung ist, die ausgewählt ist ausder Gruppe bestehend aus den organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

**14.** Vorrichtung gemäß einem oder mehreren der Ansprüche 11 bis 13, **dadurch gekennzeichnet, dass** es sich bei der Vorrichtung um eine organische Elektrolumineszenzvorrichtung handelt, die wenigstens eine Verbindung gemäß einem oder mehreren der Ansprüche 1 bis 4 oder wenigstens eine Zusammensetzung gemäß Anspruch 5 oder 6 in der Emissionsschicht enthält.

## Claims

**1.** Organic luminescent TADF compound of the formula (1) which does not contain any metals and which has a gap between the first excited triplet state ($T_1$) and the first excited singlet state ($S_1$) of not more than 0.15 eV,

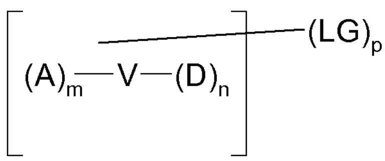

Formula (1)

where the symbols and indices used are as follows:

m is an integer selected from 1, 2, 3, 4 and 5;
n is an integer selected from 1, 2, 3, 4 and 5;
p is an integer greater than or equal to 1; preferably, p is an integer from 1 to 12, very preferably an integer from 1 to 10, very particularly preferably an integer from 1 to 8, especially preferably an integer from 1 to 6 and further preferably an integer from 1 to 4;
A is an acceptor group which is an electron acceptor group, where the electron acceptor group contains a cyano group, nitrile group, oxo group, $CF_3$ or electron-deficient heteroaryl group such as pyrimidines, pyrazines, triazines, which may be substituted by one or more $R^1$ radicals which may be the same or different at each instance;
D is a donor group which is an electron donor group, where the electron donor group contains a diarylamino group, diarylheteroarylamino group, carbazole group, indenocarbazole group or indolocarbazole group, which may be substituted by one or more $R^1$ radicals which may be the same or different at each instance;

LG is a solubilizing group which may be bonded to A, D and/or V, is preferably bonded to the A and/or D groups and is very preferably bonded to the D group, where the LG group is selected from
a straight-chain alkyl or alkoxy group having 3 to 15 carbon atoms;
a branched or cyclic alkyl or alkoxy group having 3 to 40 carbon atoms, preferably having 3 to 30 carbon atoms, very preferably having 3 to 20 carbon atoms, even more preferably having 3 to 15 carbon atoms and especially preferably having 3 to 10 carbon atoms;
an aromatic or heteroaromatic ring system having 3 or more aromatic or heteroaromatic rings which may be substituted by one or more identical or different $R^1$ radicals;
an aromatic or heteroaromatic ring system having 1 or 2 aromatic or heteroaromatic rings substituted in the ortho position by one or more identical or different $R^1$ radicals other than H, where the rings may be substituted by further identical or different $R^1$ radicals;
a group of the formula (LG-1)

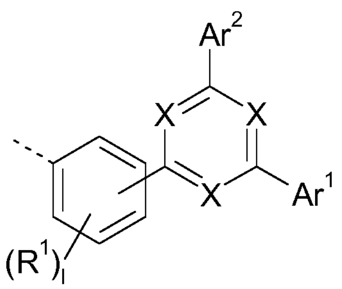

Formula (LG-1)

where the symbols used are as follows:

$Ar^1$, $Ar^2$ are each independently an aryl or heteroaryl group which may be substituted by one or more $R^1$ radicals,
X is in each case independently N or $CR^2$, where not more than one of the three X groups may be an N; all three X groups are preferably $CR^2$ and X is most preferably CH,
$R^2$ is in each case independently hydrogen, a straight-chain alkyl, alkoxy or thioalkoxy group having 1 to 40 carbon atoms or a branched or cyclic alkyl, alkoxy or thioalkoxy group having 3 to 40 carbon atoms or a silyl group or a substituted keto group having 1 to 40 carbon atoms, an alkoxycarbonyl group having 2 to 40 carbon atoms, an aryloxycarbonyl group having 7 to 40 carbon atoms, a crosslinkable group or a substituted or unsubstituted aromatic or heteroaromatic ring system having 5 to 60 ring atoms, or an aryloxy or heteroaryloxy group having 5 to 60 ring atoms, or a combination of these systems, where two or more of the $R^2$ groups may form a mono- or polycyclic, aliphatic or aromatic ring system with one another;
1 is 0, 1, 2, 3 or 4;

where the dotted bond indicates the bond from LG to A, D or V,

V is any organic bridge between the A and D groups or a single bond, where,
when V is a single bond, either m or n is 1, and
when V is an organic bridge, the bridge V is selected from the formulae (V-1) to (V-7)

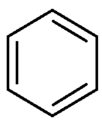

Formula (V-1)

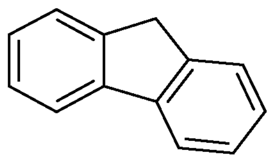

Formula (V-2)

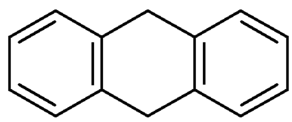

Formula (V-3)

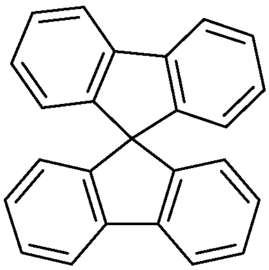

Formula (V-4)

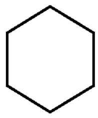

Formula (V-5)

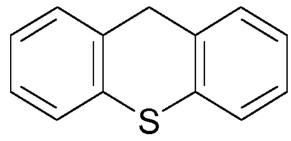

Formula (V-6)

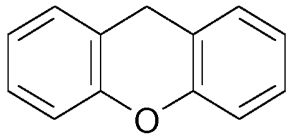

Formula (V-7)

where V may be substituted by one or more $R^1$ radicals which may be the same or different at each instance; $R^1$ is the same or different at each instance and is hydrogen, a straight-chain alkyl, alkoxy or thioalkoxy group having 1 to 40 carbon atoms or a branched or cyclic alkyl, alkoxy or thioalkoxy group having 3 to 40 carbon atoms or is a silyl group or a substituted keto group having 1 to 40 carbon atoms, an alkoxycarbonyl group having 2 to 40 carbon atoms, an aryloxycarbonyl group having 7 to 40 carbon atoms, a crosslinkable group or a substituted or unsubstituted aromatic or heteroaromatic ring system having 5 to 60 ring atoms, or an aryloxy or heteroaryloxy group having 5 to 60 ring atoms, or a combination of these systems, where two or more of the $R^1$ groups together may form a mono- or polycyclic, aliphatic or aromatic ring system, it being preferable when no two or more of the $R^1$ groups together can form a mono- or polycyclic, aliphatic or aromatic ring system.

**2.** Compound according to Claim 1, **characterized in that** the TADF compound is an organic compound having a molecular weight of not more than 5000 g/mol, very preferably of not more than 4000 g/mol, more preferably of not more than 3000 g/mol, even more preferably of not more than 2000 g/mol, especially preferably of not more than 1500 g/mol and even more preferably of not more than 1000 g/mol.

**3.** Compound according to Claim 1 or 2, **characterized in that** the LG group is selected from the following groups, where the groups may have further substitution by one or more identical or different $R^1$ radicals:

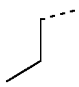

Formula (LG-5)

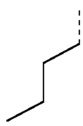

Formula (LG-6)

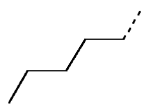

Formula (LG-7)

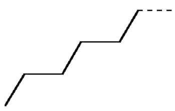

Formula (LG-8)

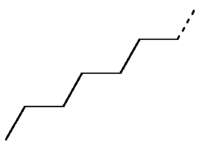

Formula (LG-9)

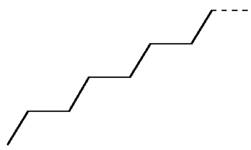

Formula (LG-10)

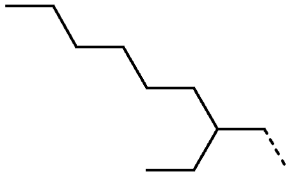

Formula (LG-11)

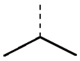

Formula (LG-12)

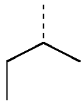

Formula (LG-13)

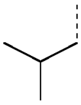

Formula (LG-14)

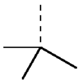

Formula (LG-15)

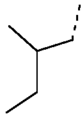

Formula (LG-16)

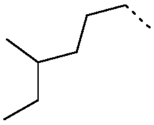

Formula (LG-17)

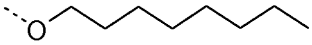

Formula (LG-18)

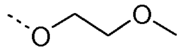

Formula (LG-19)

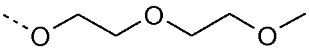

Formula (LG-20)

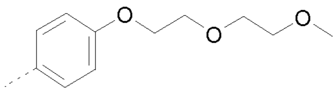

Formula (LG-21)

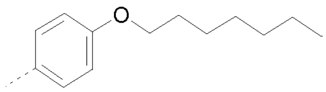

Formula (LG-22)

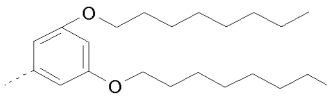

Formula (LG-23)

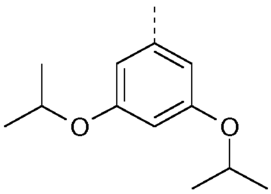

Formula (LG-24)

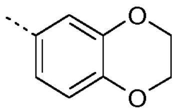

Formula (LG-25)

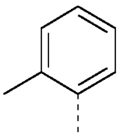

Formula (LG-26)

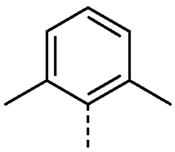

Formula (LG-27)

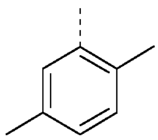

Formula (LG-28)

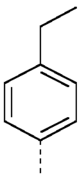

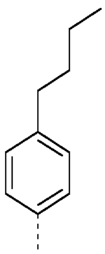

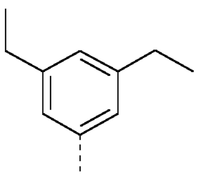

Formula (LG-29) Formula (LG-30) Formula (LG-31)

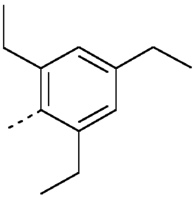 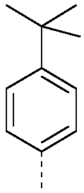 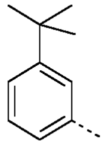

Formula (LG-32) Formula (LG-33) Formula (LG-34)

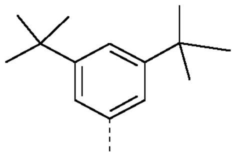 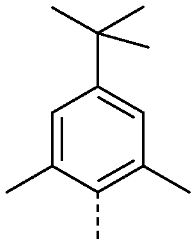 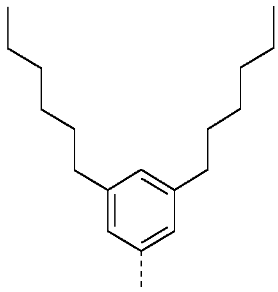

Formula (LG-35) Formula (LG-36) Formula (LG-37)

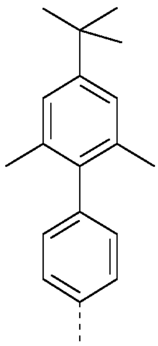 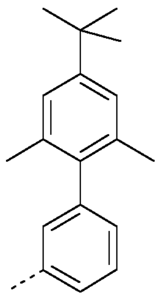 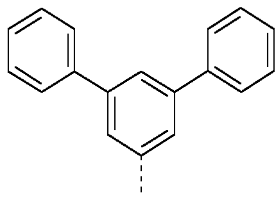

Formula (LG-38) Formula (LG-39) Formula (LG-40)

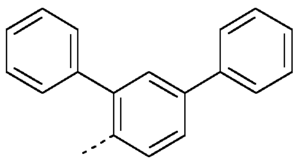 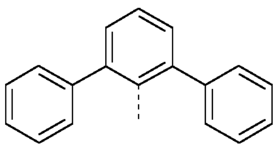 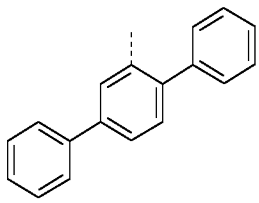

Formula (LG-41) Formula (LG-42) Formula (LG-43)

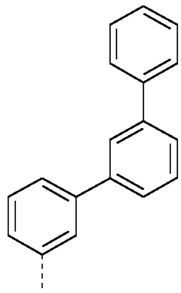

Formula (LG-44)

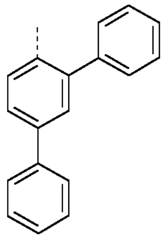

Formula (LG-45)

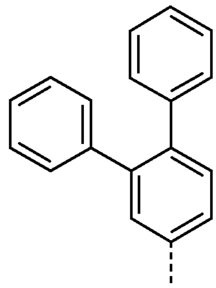

Formula (LG-46)

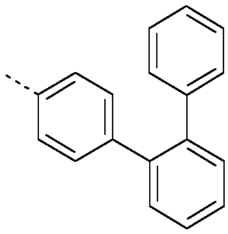

Formula (LG-47)

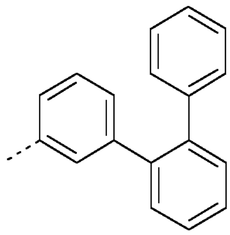

Formula (LG-48)

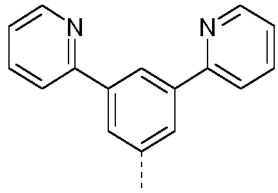

Formula (LG-49)

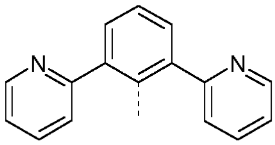

Formula (LG-50)

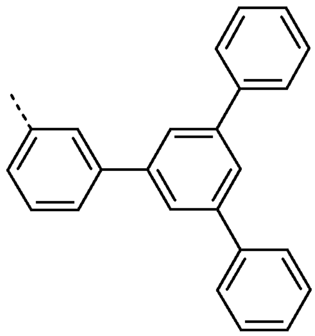

Formula (LG-51)

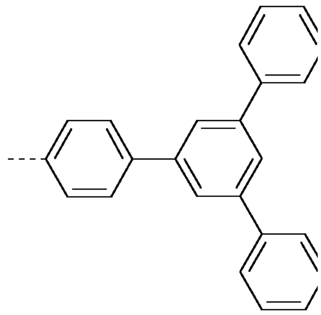

Formula (LG-52)

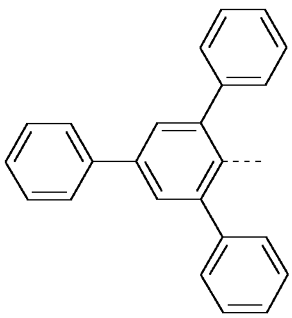

Formula (LG-53)

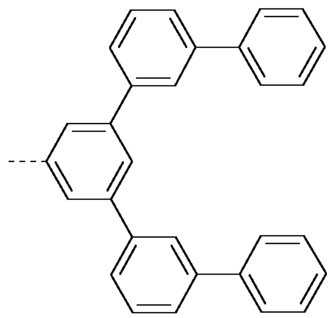

Formula (LG-54)

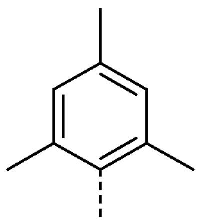

Formula (LG-27a)

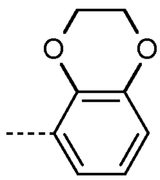

Formula (LG-55)

**4.** Compound according to one or more of Claims 1 to 3, **characterized in that** the compound contains at least 3, preferably at least 4, very preferably at least 5 and especially at least 6 aromatic rings.

**5.** Composition comprising one or more of the compounds according to one or more of Claims 1 to 4 and one or more functional materials selected from the group of the electron-conducting materials (ETM), electron-injecting materials (EIM), electron-blocking materials (EBM), hole-conducting materials (HTM), hole-injecting materials (HIM), hole-blocking materials (HBM), fluorescent emitters, phosphorescent emitters, matrix materials and inorganic nanoparticles, particular preference being given to matrix materials.

**6.** Composition according to Claim 5, **characterized in that** the composition comprises two matrix materials.

**7.** Formulation comprising at least one compound according to one or more of Claims 1 to 4 or a composition according to Claim 5 or 6 and at least one solvent.

**8.** Use of the formulation according to Claim 7 for production of one or more layers of an organic electronic device from solution.

**9.** Process for producing an organic electronic device, **characterized in that** at least one layer of the electronic device is produced from solution with the aid of the formulation according to Claim 7.

**10.** Use of at least one compound according to one or more of Claims 1 to 4 or of at least one composition according to Claim 5 or 6 in an organic electronic device, preferably in an organic electroluminescent device, very preferably in an organic light-emitting diode (OLED) or organic light-emitting electrochemical cell (OLEC, LEEC, LEC), even more preferably in an OLED and especially preferably in the emission layer (EML) of an OLED.

**11.** Organic electronic device comprising at least one compound according to one or more of Claims 1 to 4 or at least one composition according to Claim 5 or 6.

**12.** Device according to Claim 11, **characterized in that** it is selected from organic integrated circuits (OICs), organic field-effect transistors (OFETs), organic thin-film transistors (OTFTs), organic electroluminescent devices, organic solar cells (OSCs), organic optical detectors, organic photoreceptors, preferably an organic electroluminescent device.

**13.** Device according to Claim 11 or 12, **characterized in that** the device is an organic electroluminescent device selected from the group consisting of the organic light-emitting transistors (OLETs), organic field quench devices (OFQDs), organic light-emitting electrochemical cells (OLECs, LECs, LEECs), organic laser diodes (O-lasers) and organic light-emitting diodes (OLEDs), preferably OLECs and OLEDs, very preferably OLEDs.

**14.** Device according to one or more of Claims 11 to 13, **characterized in that** the device is an organic electroluminescent device comprising at least one compound according to one or more of Claims 1 to 4 or at least one composition according to Claim 5 or 6 in the emission layer.

**Revendications**

**1.** Composé TADF luminescent organique de la formule (1), qui ne contient pas de métaux, qui présente un écart entre le premier état triplet excité ($T_1$) et le premier état singulet excité ($S_1$) inférieur ou égal à 0, 15 eV

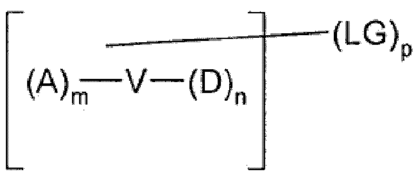

Formule (1)

dans laquelle les symboles et les indices utilisés ont les significations suivantes :

m est un nombre entier choisi parmi 1, 2, 3, 4 ou 5 ;
n est un nombre entier choisi parmi 1, 2, 3, 4 ou 5 ;
p est un nombre entier qui est supérieur ou égal à 1 ; p étant de préférence un nombre entier de 1 à 12, tout particulièrement un nombre entier de 1 à 10, de manière tout particulièrement préférée un nombre entier de 1 à 8, de manière particulièrement préférée un nombre entier de 1 à 6, et de manière davantage préférée un nombre entier de 1 à 4 ;
A est un groupe accepteur, qui est un groupe accepteur d'électrons, le groupe accepteur d'électrons contenant un groupe cyano, un groupe nitrile, un groupe oxo, $CF_3$ ou un groupe hétéroaryle pauvre en électrons, tel que des pyrimidines, des pyrazines, des triazines, qui peut être substitué avec un ou plusieurs radicaux $R^1$, qui peuvent être identiques ou différents à chaque occurrence ;
D est un groupe donneur, qui est un groupe donneur d'électrons, le groupe donneur d'électrons contenant un groupe diarylamino, un groupe diarylhétéroarylamino, un groupe carbazole, un groupe indénocarbazole ou un groupe indolocarbazole, qui peut être substitué avec un ou plusieurs radicaux $R^1$, qui peuvent être identiques ou différents à chaque occurrence ;
LG est un groupe promoteur de solubilité, qui peut être relié à A, D et/ou V, qui est de préférence relié aux groupes A et/ou D et qui est tout particulièrement relié au groupe D, le groupe LG étant choisi parmi :

un groupe alkyle ou alcoxy à chaîne linéaire de 3 à 15 atomes C ;
un groupe alkyle ou alcoxy ramifié ou cyclique de 3 à 40 atomes C, de préférence de 3 à 30 atomes C, tout particulièrement de 3 à 20 atomes C, de manière tout particulièrement préférée de 3 à 15 atomes C, et de manière particulièrement préférée de 3 à 10 atomes C ;
un système cyclique aromatique ou hétéroaromatique contenant 3 ou davantage de cycles aromatiques ou hétéroaromatiques, qui peuvent être substitués avec un ou plusieurs radicaux $R^1$ identiques ou différents ;
un système cyclique aromatique ou hétéroaromatique contenant 1 ou 2 cycles aromatiques ou hétéroaromatiques, qui sont substitués en position ortho avec un ou plusieurs radicaux $R^1$ identiques ou différents, qui sont différents de H, les cycles pouvant être substitués avec des radicaux $R^1$ supplémentaires, identiques ou différents ;
un groupe de la formule (LG-1) :

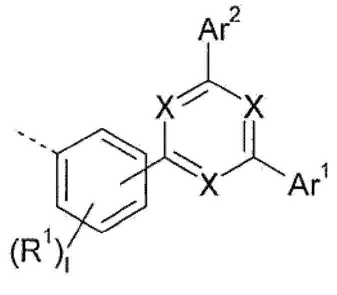

Formule (LG-1)

dans laquelle les symboles utilisés ont la signification suivante :

$Ar^1$, $Ar^2$ représentent chacun indépendamment l'un de l'autre un groupe aryle ou hétéroaryle, qui peut être substitué avec un ou plusieurs radicaux $R^1$,
les X représentent chacun indépendamment les uns des autres N ou $CR^2$, au plus un des trois groupes X pouvant être un N, les trois X représentant de préférence $CR^2$, et X représentant tout particulièrement CH,
les $R^2$ représentant chacun indépendamment les uns des autres l'hydrogène, un groupe alkyle, alcoxy ou thioalcoxy à chaîne linéaire de 1 à 40 atomes C, ou un groupe alkyle, alcoxy ou thioalcoxy ramifié ou cyclique de 3 à 40 atomes C, ou un groupe silyle, ou un groupe céto substitué de 1 à 40 atomes C, un groupe alcoxycarbonyle de 2 à 40 atomes C, un groupe aryloxycarbonyle de 7 à 40 atomes C, un groupe réticulable ou un système cyclique aromatique ou hétéroaromatique substitué ou non substitué de 5 à 60 atomes de cycle, ou un groupe aryloxy ou hétéroaryloxy de 5 à 60 atomes de cycle, ou

une combinaison de ces systèmes, deux ou davantage des groupes $R^2$ pouvant former les uns avec les autres un système cyclique mono- ou polycyclique, aliphatique ou aromatique ;
1 représente 0, 1, 2, 3 ou 4 ;

la liaison en pointillés indique la liaison de LG à A, D ou V,

V représente un pont organique quelconque entre les groupes A et D, ou une simple liaison,
lorsque V représente une simple liaison, m ou n représentant 1, et
lorsque V représente un pont organique, alors le pont V étant choisi parmi les formules (V-1) à (V-7) :

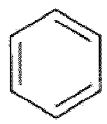

Formule (V-1)

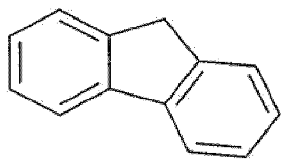

Formule (V-2)

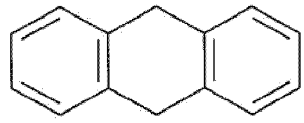

Formule (V-3)

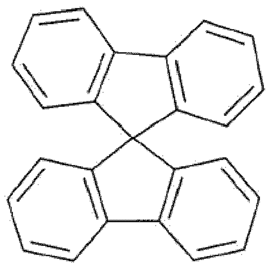

Formule (V-4)

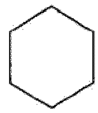

Formule (V-5)

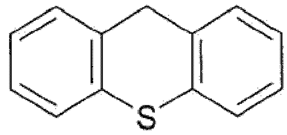

Formule (V-6)

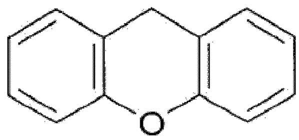

Formule (V-7)

dans lesquelles V peut être substitué avec un ou plusieurs radicaux $R^1$, qui peuvent être identiques ou différents à chaque occurrence ;
les $R^1$ représentent, de manière identique ou différente à chaque occurrence, l'hydrogène, un groupe alkyle, alcoxy ou thioalcoxy à chaîne linéaire de 1 à 40 atomes C, ou un groupe alkyle, alcoxy ou thioalcoxy ramifié ou cyclique de 3 à 40 atomes C, ou un groupe silyle, ou un groupe céto substitué de 1 à 40 atomes C, un groupe alcoxycarbonyle de 2 à 40 atomes C, un groupe aryloxycarbonyle de 7 à 40 atomes C, un groupe réticulable ou un système cyclique aromatique ou hétéroaromatique substitué ou non substitué, de 5 à 60 atomes de cycle, ou un groupe aryloxy ou hétéroaryloxy de 5 à 60 atomes de cycle, ou une combinaison de ces systèmes, deux ou davantage des groupes $R^1$ pouvant former les uns avec les autres un système cyclique mono- ou polycyclique, aliphatique ou aromatique, deux ou davantage des groupes $R^1$ ne formant de préférence pas les uns avec les autres de système cyclique mono- ou polycyclique, aliphatique ou aromatique.

**2.** Composé selon la revendication 1, **caractérisé en ce que** le composé TADF est un composé organique ayant un poids moléculaire inférieur ou égal à 5 000 g/mol, tout particulièrement inférieur ou égal à 4 000 g/mol, de manière particulièrement préférée inférieur ou égal à 3 000 g/mol, de manière tout particulièrement préférée inférieur ou

égal à 2 000 g/mol, de manière particulièrement préférée inférieur ou égal à 1 500 g/mol et de manière encore davantage préférée inférieur ou égal à 1 000 g/mol.

**3.** Composé selon la revendication 1 ou 2, **caractérisé en ce que** le groupe LG est choisi parmi les groupes suivants, les groupes pouvant en outre être substitués avec un ou plusieurs radicaux $R^1$ identiques ou différents :

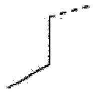

Formule (LG-5)

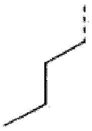

Formule (LG-6)

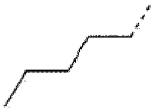

Formule (LG-7)

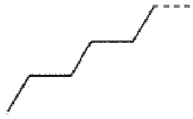

Formule (LG-8)

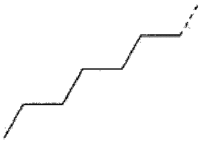

Formule (LG-9)

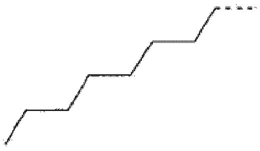

Formule (LG-10)

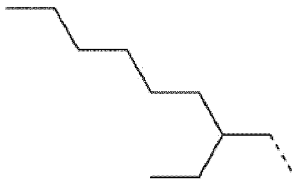

Formule (LG-11)

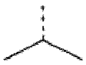

Formule (LG-12)

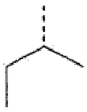

Formule (LG-13)

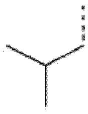

Formule (LG-14)

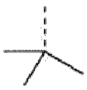

Formule (LG-15)

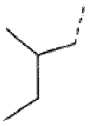

Formule (LG-16)

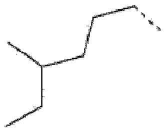

Formule (LG-17)

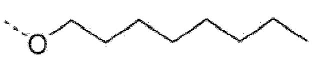

Formule (LG-18)

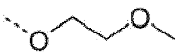

Formule (LG-19)

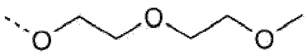

Formule (LG-20)

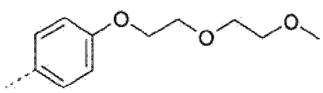

Formule (LG-21)

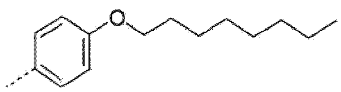

Formule (LG-22)

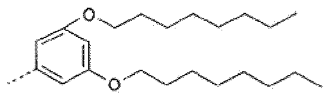

Formule (LG-23)

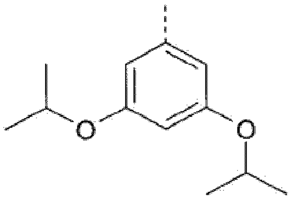

Formule (LG-24)

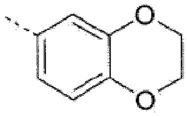

Formule (LG-25)

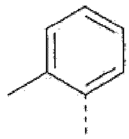

Formule (LG-26)

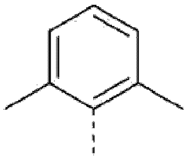

Formule (LG-27)

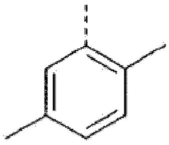

Formule (LG-28)

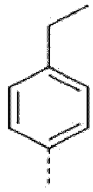

Formule (LG-29)

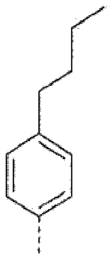

Formule (LG-30)

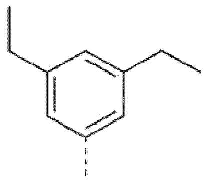

Formule (LG-31)

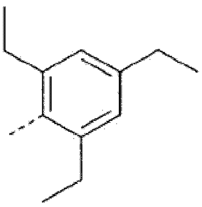

Formule (LG-32)

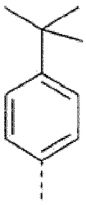

Formule (LG-33)

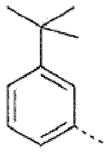

Formule (LG-34)

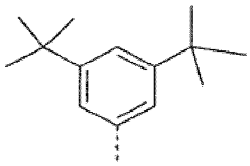

Formule (LG-35)

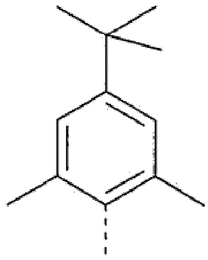

Formule (LG-36)

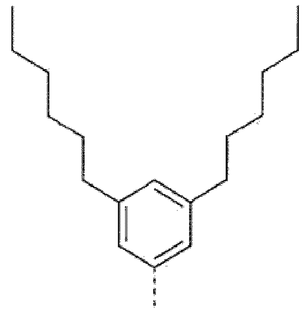

Formule (LG-37)

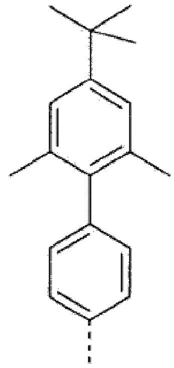

Formule (LG-38)

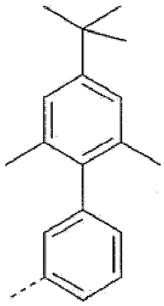

Formule (LG-39)

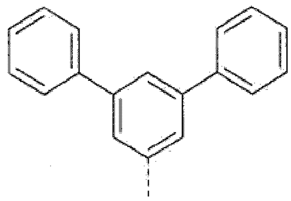

Formule (LG-40)

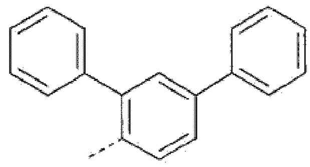

Formule (LG-41)

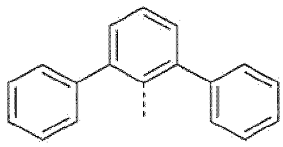

Formule (LG-42)

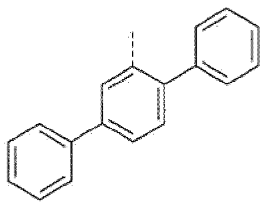

Formule (LG-43)

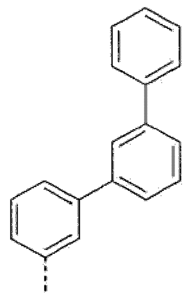

Formule (LG-44)

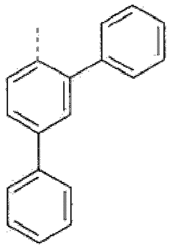

Formule (LG-45)

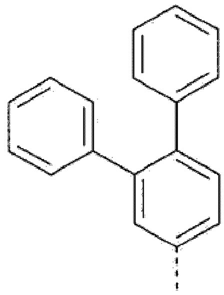

Formule (LG-46)

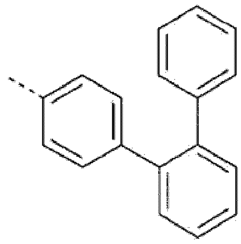

Formule (LG-47)

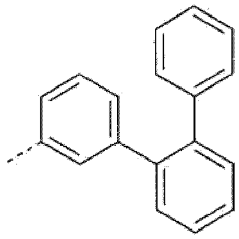

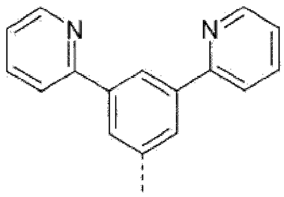

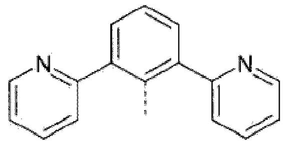

Formule (LG-48) Formule (LG-49) Formule (LG-50)

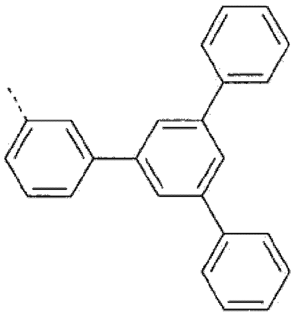

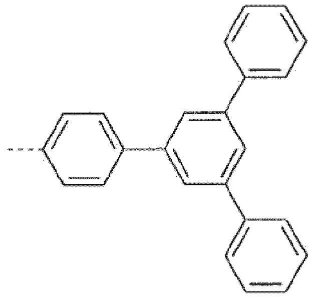

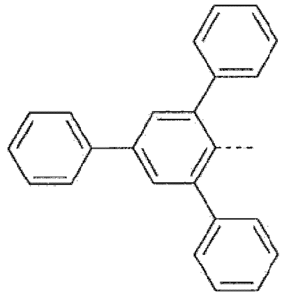

Formule (LG-51) Formule (LG-52) Formule (LG-53)

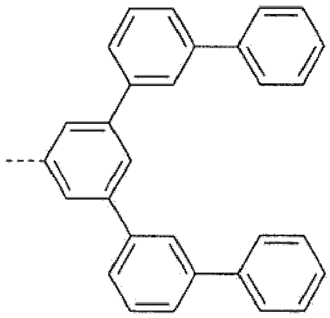

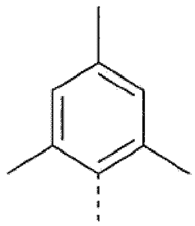

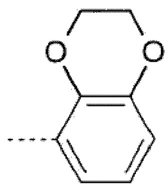

Formule (LG-54) Formule (LG-27a) Formule (LG-55)

**4.** Composé selon une ou plusieurs des revendications 1 à 3, **caractérisé en ce que** le composé contient au moins 3, de préférence au moins 4, tout particulièrement au moins 5 et notamment au moins 6 cycles aromatiques.

**5.** Composition contenant un ou plusieurs des composés selon une ou plusieurs des revendications 1 à 4, ainsi qu'un ou plusieurs matériaux fonctionnels choisis dans le groupe constitué par les matériaux conducteurs d'électrons (ETM), les matériaux injecteurs d'électeurs (EIM), les matériaux bloqueurs d'électrons (EBM), les matériaux conducteurs de trous (HTM), les matériaux injecteurs de trois (HIM), les matériaux bloqueurs de trous (HBM), les émetteurs fluorescents, les émetteurs phosphorescents, les matériaux de matrice et les nanoparticules inorganiques, les matériaux de matrice étant particulièrement préférés.

**6.** Composition selon la revendication 5, **caractérisée en ce que** la composition contient deux matériaux de matrice.

**7.** Formulation contenant au moins un composé selon une ou plusieurs des revendications 1 à 4 ou une composition selon la revendication 5 ou 6, ainsi qu'au moins un solvant.

**8.** Utilisation de la formulation selon la revendication 7 pour la fabrication d'une ou de plusieurs couches d'un dispositif

électronique organique à partir d'une solution.

**9.** Procédé de fabrication d'un dispositif électronique organique, **caractérisé en ce qu'**au moins une couche du dispositif électronique est fabriquée à l'aide de la formulation selon la revendication 7 à partir d'une solution.

**10.** Utilisation d'au moins un composé selon une ou plusieurs des revendications 1 à 4 ou d'au moins une composition selon la revendication 5 ou 6 dans un dispositif électronique organique, de préférence dans un dispositif électroluminescent organique, tout particulièrement dans une diode électroluminescente organique (OLED) ou une cellule électrochimique électroluminescente organique (OLEC, LEEC, LEC), de manière tout particulièrement préférée dans une OLED et de manière particulièrement préférée dans la couche d'émission (EML) d'une OLED.

**11.** Dispositif électronique organique contenant au moins un composé selon une ou plusieurs des revendications 1 à 4 ou au moins une composition selon la revendication 5 ou 6.

**12.** Dispositif selon la revendication 11, **caractérisé en ce qu'**il est choisi parmi les commutateurs intégrés organiques (OIC), les transistors à effet de champ organiques (OFET), les transistors à film mince organiques (OTFT), les dispositifs électroluminescents organiques, les cellules solaires organiques (OSC), les détecteurs optiques organiques, les photorécepteurs organiques, de préférence un dispositif électroluminescent organique.

**13.** Dispositif selon la revendication 11 ou 12, **caractérisé en ce que** le dispositif est un dispositif électroluminescent organique, qui est choisi dans le groupe constitué par les transistors électroluminescents organiques (OLET), les dispositifs d'extinction de champ organiques (OFQD), les cellules électrochimiques électroluminescentes organiques (OLEC, LEC, LEEC), les diodes lasers organiques (O-laser) et les diodes électroluminescentes organiques (OLED), de préférence les OLEC et les OLED, tout particulièrement les OLED.

**14.** Dispositif selon une ou plusieurs des revendications 11 à 13, **caractérisé en ce que** le dispositif est un dispositif électroluminescent organique, qui contient au moins un composé selon une ou plusieurs des revendications 1 à 4 ou au moins une composition selon la revendication 5 ou 6 dans la couche d'émission.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- EP 2599773 A1
- WO 2011116865 A1
- WO 2013064206 A1
- WO 2014056567 A1
- WO 2014094964 A1
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- US 20090134784 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 2005111172 A
- WO 2006117052 A
- WO 2010054729 A
- WO 2010054730 A
- WO 2010015306 A
- EP 652273 A
- WO 2009062578 A
- US 20090136779 A
- WO 2010050778 A
- WO 2011042107 A
- WO 2011088877 A
- WO 2011076325 A1
- US 10796832 B
- US 10656910 B
- US 60578236 B
- US 20070034833 A1
- US 20050109989 A1
- US 20130135558 A1
- WO 2011073149 A
- EP 1968131 A
- EP 2276085 A
- EP 2213662 A
- EP 1722602 A
- EP 2045848 A
- DE 102007031220
- US 8044390 B
- US 8057712 B
- WO 2009003455 A
- WO 2010094378 A
- WO 2011120709 A
- US 20100096600 A
- WO 2004037887 A
- WO 2004058911 A
- WO 2010097155 A


### In der Beschreibung aufgeführte Nicht-Patentliteratur


- **M. A. BALDO et al.** *Appl. Phys. Lett.,* 1999, vol. 75, 4-6
- **B. H. UOYAMA et al.** *Nature,* 2012, vol. 492, 234
- Hansen Solubility Parameters: A User's Handbook. Taylor and Francis Group, LLC, 2007
- **HANSON ; ABBOT.** Programm Hansen Solubility Parameters in Practice (HSPiP)
- *J. Am. Chem. Soc.,* 1993, vol. 115 (19), 8706-8715
- *Science,* 1996, vol. 271, 933
- *J. Am. Chem. Soc.,* 1997, vol. 30, 7019-7029
- *J. Am. Chem. Soc.,* 1993, vol. 115, 8706
- **X. PENG et al.** *J. Am. Chem. Soc.,* 1997, vol. 119, 7019-7029
- *J. Am. Chem. Soc.,* 1997, vol. 119, 7019-7029
- *Chem. Rev.,* 2007, vol. 107, 1233
- **M. S. ARNOLD et al.** *Appl. Phys. Lett.,* 2008, vol. 92, 053301
- **D. J. TOZER et al.** *J. Chem. Phys.,* 2008, vol. 128, 044118
- **H. UOYAMA et al.** *Nature,* 2012, vol. 492, 234
- *CHEMICAL ABSTRACTS,* 1416881-52-1
- *CHEMICAL ABSTRACTS,* 1206626-92-7
- *CHEMICAL ABSTRACTS,* 1418575-73-1